## Europäisches Patentamt
## European Patent Office
## Office européen des brevets

(19)

(11) Publication number : **0 032 134 B2**

(12) # NEW EUROPEAN PATENT SPECIFICATION

(45) Date of publication of the new patent specification :
**13.10.93 Bulletin 93/41**

(21) Application number : **81300050.2**

(22) Date of filing : **07.01.81**

(51) Int. Cl.⁵ : **C12N 15/21,** C12P 21/02,
C07H 21/04, C07C 233/00,
A61K 37/66, C12N 1/20

(54) DNA sequences, recombinant DNA molecules and processes for producing human interferon-alpha like polypeptides.

(30) Priority : 08.01.80 EP 80300079
03.04.80 EP 80301100
02.10.80 GB 8031737

(43) Date of publication of application :
15.07.81 Bulletin 81/28

(45) Publication of the grant of the patent :
15.08.84 Bulletin 84/33

(45) Mention of the opposition decision :
13.10.93 Bulletin 93/41

(84) Designated Contracting States :
AT BE CH DE FR GB IT LI LU NL SE

(56) References cited :
EP-A- 43 980
EP-A- 0 001 929
EP-A- 0 028 033
EP-A- 1 174 143
EP-B- 11 560
EP-B- 20 147
EP-B- 28 033
EP-B- 91 539
EP-B- 108 128
EP-B- 143 980
DT-A- 2 724 918
DT-A- 2 930 604
GB-A- 2 019 408
GB-A- 2 027 033
GB-A- 2 031 434
GB-A- 2 033 905
GB-A- 2 034 323
GB-A- 2 034 717
GB-A- 2 037 396
GB-B- 1 412 591
GB-B- 1 412 591
GB-B- 1 412 591
GB-B- 1 568 047
GB-B- 2 079 291
GB-B-15 215 91
US-A- 3 699 222
US-A- 4 184 917
US-A- 4 190 495
US-A- 4 190 495
US-A- 4 190 495
US-A- 4 237 224
US-A- 4 237 224
US-A- 4 237 224
US-A- 4 241 174
US-A- 4 241 174
US-A- 4 241 174
US-A- 4 262 090
US-A- 4 262 090
US-A- 4 262 090
US-A- 4 289 690
US-A- 4 393 201
US-A- 4 599 585
US-A- 4 695 542
US-A- 4 695 543

(73) Proprietor : BIOGEN, INC.
Fourteen Cambridge Center
Cambridge Massachusetts 02142 (US)

(72) Inventor : Weissmann, Charles
Eschenhaustrasse 34
CH-8053 Zurich (CH)

(74) Representative : Ritter, Stephen David et al
Mathys & Squire 10 Fleet Street
London EC4Y 1AY (GB)

EP 0 032 134 B2

(56) References cited :
RESEARCH DISCLOSURE, no. 18309, July 1979,
pages 361-362, "The production of interferon by
genetic engineering"
PROC. JAPAN. ACAD, vol. 55(9), Ser. B (1979), pages
464-469, T. TANIGUCHI et al. "Construction and
identification of a bacterial plasmid containing the
human fibroblast interferon gene sequence"
NATURE, vol. 284, March 27, 1980, pages 316-320, S.
NAGATA et al. "Synthesis in E.coli of a polypeptide
with human leukocyte interferon activity"
GENE, vol. 10 (1), May 1980, pages 1-10, N. MANTEI
et al. "The nucleotide sequence of a cloned human
leukocyte interferon cDNA"
NATURE, vol. 285, June 19, 1980, pages 547-549, T.
TANIGUCHI et al. "Human leukocyte and fibroblast
interferons are structurally related"
NATURE, vol. 287, October 2, 1980, pages 401-408, S.
NAGATA et al. "The structure of one of the eight or
more distinct chromosomal genes for human inter-
feron-alpha"
CHEMICAL ABSTRACTS, vol. 94, no. 9, March 2,
1981, page 550, abstract 63542q, Columbus, Ohio,
U.S.A., STEWART, W.E. et al., "Comparisons of sev-
eral biological and physico-chemical properties of
human leukocyte interferons produced by human
leukocytes and E.coli"
CHEMICAL ABSTRACTS, vol. 93, no. 25, December
22, 1980, page 624, abstract 236612x, Columbus,
Ohio, USA, STREULI, M. et al. "At least three human
type alpha-interferons: structure of alpha 2"
Nature vol.280 1979 pp. 815-819. Venezuela et al
Fiddes et al. Nature vol. 281 1979. pp 351-355
Kozak. Cell 15. 1978 pp. 1109-1125
Southern. Methods in Enzymology. vol. 68 1979. pp
152-176.
PNAS vol. 81. pp. 7698-7702. 1984 Johansen et al
Cell vol. 20. Perler et al.pp 555-566. 1980
Kitamura et al. Nature vol. 291. pp 547,552
Journal Molecular Biology vol. 151. pp 607-625 1981.
Seiler Tuyns et al.
Isaacs and Lindenmann . Proc. Royal Soc. Series B.
vol. 147 pp 258-267. 1957
Knight. Jr. PNAS vol. 73pp 520-523. 1976
Bridgen et al. Journal Biol. Chem. vol. 252. pp
6585-6587
Cavalieri et al.PNAS vol. 74.pp 3287-3291
Fujisawa et al. Journal Biol. Chem.vol. 253.
pp.8677-8679
Rubinsrein et al. Science. vol. 202. pp1289-1290.
1978
Wassenbach et al. Eur. J. Biochemistry. vol. 98 pp
1-8, 1979
Slate & Ruddle. Cell, vol. 16. pp 171-180. 1979
Rubinstein et al. PNAS USA vol. 76pp. 640-644, 1979
Zoon et al. PNAS USA vol. 76 pp 5601-5605, 1979
Stewart et al.. Virology, vol. 97 pp.473-476. 1979
Stewart. "The Interferon System", 1979. Springer
Verlag
Zoon et al.Science, vol. 207 pp. 527-528 1980 (Zoon
II)
Hunkapiller & Hood. Personal communication. 1980.
Research disclosure no. 18309 pp.361-362.
Proc. Japanase Acadamy. vol. 55(9), series B(1979)
pp.464-469. Taniguchi et al.
Nature vol. 284, 1980. pp. 316-320. Nagata et al
Gene vol. 10 1980. pp. 1-10. Mantei et al.
Nature vol. 285. 1980. pp.547-549. Taniguchi et al.
Nature vol. 287. 1980. pp. 401-408. Nagata et al.
Chemical abstracts vol. 94 no. 9 1981 p. 550. Ab-
stract 63542q, Columbus, Ohio. Stewart et al.
Gene 1980, vol. 11(3-4) 181-6.
Chemical Abstracts vol. 93 no. 25 1980 p. 624. Ab-
stract 236612 x, Columbus, Ohio. Streuli et al.
Science (Washington DC) 1980, vol. 209(4463),
1343-7. Streuli I.
Abstracts. "Conference on regulatory functions of
Interferons" N. York Academy of Sciences, 1979
Ambler et al. PNAS USA pp. 3732-3736, 1978
Beverly et al. Nature vol. 278 pp 119-120, 1979
Bolivar et al. Genw 2, pp. 95-113, 1977
Broome et al. PNAS USA, vol. 75 pp 2746-2749.1978
Burrell et al. Nature, vol. 279 pp.43-47. 1979
Casadaban et al. PNAS USA vol. 76 pp. 4530-4533.
1979
Chang et al. Nature vol. 275 pp. 617-24f
Farrell et al. PNAS USA vol. 75 pp.5893-5897. 1978
Fraser et al. PNAS USA vol. 75 pp. 5936-5940, 1978

(56) References cited :
Fritsch et al. C.R. Academy of Science, vol.287. pp.
1453-1456 1978.
Goeddel et al.. Nature vol. 281 pp.544-548. 1979
Goeddel et al.. PNAS USA vol. 76 pp.106-110, 1979
Grunstein et al. PNAS USA vol. 72 pp. 3961-3965.
1975
Havell et al. Journal Biological Chemistry, vol. 252
pp. 4425-4427 1977.
Herberman et al. Nature vol.277 pp. 221-223. 1979
Itakura et al.Science vol. 198 pp.1056-1063. 1977.
Kerr et al. PNAS USA, vol. 75. pp. 256-260. 1978
Knight. PNAS USA vol.73 pp. 520-523.1976
Lewis et al. European Journal Biochemistry vol. 86.
pp 497-509 1978
Martial et al. Science vol. 205.pp. 602-606. 1979.
Maxam et al. PNAS USA vol. 74 pp.560-564. 1977
Mercereau-Puijalon et al. Nature vol. 275 pp.
505-510, 1978
Rosenfeld. Readers Digest pp. 130-133. 1979.
Seeburg et al.Nature vol. 276. pp. 795-798. 1978.
Shine et al. Nature vol. 270 pp. 494-499. 1977.
Sutcliffe. PNAS USA vol. 75 pp. 3737-3741, 1978
Villa-Komaroff et al. PNAS USA vol. 75 pp.
3727-3731. 1978
Wagner. Virology vol.13. pp. 323-337 1961.
Report "International Workshop on Interferon in the
treatment of cancer". 1975
Berg et al.Conference on regulatory function 0f
Interferons. Abstracts no. 19 1979.
Britten & Davidson. Quarterly Review of Biology no.
46 pp. 111-133.
Goeddel et al. Nature vol. 287 pp 411-416 1980.
Goeddel et al. Nature vol. 290 pp. 20-26 1981
Kennell Prog. Nuclear Acid Research Mol. Biol. vol
11, pp. 259-301 1971.
Lawn et al. Cell vol. 15. pp. 1157-1174.
Rougeon et al. Nucl. Acids Research vol. 2. pp.
2365-2378. 1975.
Taniguchi et al. PNAS USA vol.77 pp. 4005-4006.
1980.
Weissmann. Interferon 81, vol. 3 pp. 101-134.
Gressner et al Nature vol. 251. 1974. pp 543-545.
Pauker et al. Journal of General Virology vol. 35.
1977 pp. 341-351.
Lin et al. Journal gener. Virology vol. 39. pp. 125-130.
Nature. vo. 286 p. 110. July 1980.
The Interferon System. A current review to 1978.
Tex. Rep. Biol. Med. vol. 35 1977 pp 1-29, 117-149,
173-193, 205-209.
Gillespie et al. Tex. Rep. Biol. Med. vol. 41 pp.
537-542. 1981-1982
Zoon et al. Abstract no. 32. New York academy of
science. Conference on regulatory functions of in-
terferons, 1979.
Szostak et al. Nature vol. 265. pp. 61-63.
Wallace et al. Nucleic acid research , vol. 6(11),
1979.pp 3452-3557.
Noyes et al. PNAS USA vol. 76(4). 1979 pp.
1770-1774.
Szostak et al. Methods in Enzymology vol. 68. 1979
pp.419-429.
Valenzuela et al. Nature vol. 313 1985. pp. 698-700
Shepard et al Nature vol. 294 1981. pp. 563-565.
Henco et al. Journal Molecular Biology vol. 185 pp.
227-260.
Lathe et al Genetic Engineering vol 4 pp 1-56.
Smith et al. Genetic Engineering vol. 3 pp. 1-32.
Zoller et al. Methods in Enzymology vol. 100 1983 pp.
4689-5000.
Wasylyk et al. PNAS USA vol. 77(12). 1980 pp.
7024-7028.
Patient. Nature vol. 308 pp. 15-16.
Hopkins et al. Journal Molecular Biology vol. 107,
1976 pp.549-569.
Hinnen et al. PNAS USA vol. 75(4) 1978. pp
1929-1933.
Wigler et al. Cell vol. 16 1979 pp.777-785.
Seliger et al. European Journal Cell Biology vol. 25
1981 pp 8-9.
Imai et al. Journal Immunology vol. 128 1982, pp.
2824-2825.
Lewy et al PNAS USA vol. 78(10) 1981 pp.6186-6190.
Rubinstein et al. Phil. Trans. R. Soc. Lond. B. vol.
299, pp 39-50.
Pestka et al. Archives of Biochemistry and
Biophysics vol. 221(1) 1983, pp 1-37.
Lathe. Journal Molecular Biology vol. 183. pp.1-12

## Description

TECHNICAL FIELD OF INVENTION

This invention relates to DNA sequences, recombinant DNA molecules and processes for producing interferon and interferon-like polypeptides. More particularly, the invention relates to DNA sequences expressed in appropriate host organisms. The recombinant DNA molecules disclosed herein are characterized by DNA sequences that code for polypeptides having an immunological or biological activity of human leukocyte interferon. As will be appreciated from the disclosure to follow, the DNA sequences, recombinant DNA molecules and processes of this invention may be used in the production of polypeptides useful in antiviral and antitumor or anticancer agents and methods.

BACKGROUND ART

In this application the interferon nomenclature announced in *Nature,* 286, p. 2421 (July 10, 1980) will be used. This nomenclature replaces that used in our earlier applications from which this application claims priority. *E.g.,* IF is now designated IFN and leukocyte interferon is now designated IFN-α.

Two classes of interferons ("IFN") are known to exist. Interferons of Class I are small, acid stable (glyco)-proteins that render cells resistant to viral infection (A. Isaacs and J. Lindenmann, "Virus Interference I. The Interferon", *Proc. Royal Soc. Ser. B.,* 147, pp 258-67 (1957) and W. E. Stewart, II, *The Interferon System,* Springer-Verlag (1979) (hereinafter *"The Interferon System")).* Although to some extent cell specific *(The Interferon System,* pp. 135-45), IFNs are not virus specific. Instead IFNs protect cells against a wide spectrum of viruses.

Human interferons ("HuIFN") have been classified into three groups α, β and γ. HuIFN-α or leukocyte interferon is produced in human leukocyte cells and together with minor amounts of HuIFN-β (fibroblast interferon) in lymphoblastoid cells. HuIFN-α has been purified to homogeneity and characterized *(e.g.* M. Rubenstein *et al.,* "Human Leukocyte Interferon: Production, Purification To Homogeneity And Initial Characterization" *Proc. Natl. Acad. Sci. USA,* 76, pp. 640-44 (1979)). It is heterogeneous regard to size presumably because of the carbohydrate moiety. Two components have been described, one of 21000 to 22000 and the other of 15000-18000 molecular weight. The component of lower molecular weight has been reported to represent a non-glycosylated form. The smaller form of HuIFN-α has also been reported to retain most or all of its HuIFN-α activity (W. E. Stewart, II *et. al.,* "Effect of Glycosylation Inhibitors On The Production And Properties Of Human Leukocyte Interferon", *Virology,* 97, pp. 473-76 (1979)). A portion of the amino acid sequence of HuIFN-α have lymphoblastoid cells and its amino acid composition have been reported (K. C. Zoon *et al.,* "Amino Terminal Sequence Of The Major Component of Human Lymphoblastoid Interferon", *Science,* 207, pp. 527-28 (1980) and M. Humkapiller and L. Hood, personal communication (1980)).

HuIFN-α has also been reported to exist in several different forms, e.g. British patent application 2,037,296A. These forms appear to differ from each other structurally and physiologically. No accepted nomenclature has been adopted for these forms of HuIFN-α. Therefore, in this application each form will be referred to by a number after the general HuIFN-α designation, *i.e.,* HuIFN-α1 or HuIFN-α3.

HuIFN-α may, like many human proteins, also be polymorphic. Therefore, cells of particular individuals may produce HuIFN-α species within the more general HuIFN-α group or forms within that group which are physiologically similar but structurally slightly different than the group or form of which it is a part. Therefore, while the protein structure of an HuIFN-α may be generally well-defined, particulalar individuals may produce a HuIFN-α that is a slight variation thereof, this allelic variation probably being less severe than the difference between the various forms of HuIFN-α.

HuIFN- is usually not detectable in normal or healthy cells *(The interferon System,* pp. 55-57). Instead, the protein is produced as a result of the cell's exposure to an IFN inducer. IFN inducers are usually viruses but may also be non-viral in character, such as natural or synthetic double-stranded RNA, intracellular microbes, microbial products and various chemical agents. Numerous attempts have been made to take advantage of these non-viral inducers to render human cells resistant to viral infection (S. Baron and F. Dianzani (eds.), *Texas Reports On Biology And Medicine,* 35 ("Texas Reports"), pp. 528-40 (1977)). These attempts have not been very successful. Instead, use of exogenous HuIFN itself is now preferred.

Interferon therapy against viruses and tumours or cancers has been conducted at varying dosage regimes and under several modes of administration (The Interferon System, pp. 305-321). For example, interferon has been effectively administered orally, by innoculation - intraveneous, intramuscular, intranasal, intradermal and subcutaneous -, and in the form of eye drops, ointments and sprays. It is usually administered one to three times daily in dosages of $10^4$ to $10^7$ units. The extent of the therapy depends on the patient and the condition being threated. For example, virus infections are usually treated by daily or twice daily doses over several days to two weeks and tumors and cancers are usually treated by daily or multiple daily doses over several months or years. The most effective therapy for a given patient must of course be determined by the attending physician who will consider such well known factors as the course of the disease, previous therapy, and the patient's response to interferon in selecting a mode of administration and dosage regime.

As an antiviral agent, HuIFN has been used to treat the following: respiratory infections *(Texas Reports,* pp. 486-96); herpes simplex keratitis *(Texas Reports,* pp. 497-500); acute hemorrhagic conjunctivitis *(Texas Reports,* pp. 501-10); varicella zoster *(Texas Reports,* pp. 511-15); cytomegalovirus infection *(Texas Reports,* pp. 523-27); and hepatitis B *(Texas Reports,* pp. 516-22). See also *The Interferon System,* pp. 307-19. However, large scale use of IFN as an antiviral agent requires larger amounts of IFN than heretofore have been available.

HuIFN has other effects in addition to its antiviral action. For example, it antagonizes the effect of colony stimulating factor, inhibits the growth of hemopoietic colony-forming cells and interferes with the normal differentiation of granulocyte and marcrophage precursors *(Texas Reports,* pp. 343-49). It also inhibits erythroid differentiation in DMSO-treated Friend leukemia cells *(Texas Reports,* pp. 420-28). HuIFN may also play a role in regulation of the immune response. Depending upon the dose and time of application in relation to antigen, HuIFN-α can be both immunopotentiating and immunosuppressive *in vivo* and *in vitro (Texas Reports,* pp. 357-69). In addition, specifically sensitized lymphocytes have been observed to produce HuIFN-α after contact with antigen. Such antigen-induced HuIFN-α could therefore be a regulator of the immune- response, affecting both circulating antigen levels and the expression of cellular immunity *(Texas Reports,* pp. 370-74). HuIFN is also known to enhance the activity of killer lymphocytes and antibody-dependent cell-mediated cytotoxicity (R. R. Herberman *et al,.* "Augmentation By Interferon Of Human Natural And Antibody Dependent Cell-Mediated Cytotoxicity", *Nature,* 277, pp. 221-23 (1979): P. Beverley and D. Knight, "Killing Comes Naturally", *Nature* 278, pp. 119-20 (1979); *Texas Reports,* pp. 375-80). Both of these species are probably involved in the immunological attack on tumor cells.

Therefore, in addition to its use as a human antiviral agent, HuIFN has potential application in antitumor and anticancer therapy *(The interferon System,* pp. 319-21). It is now known that IFNs affect the growth of many classes of tumors in many animals *(The Inteferon System,* pp. 292-304). They, like other antitumor agents, seem most effective when directed against small tumors. The antitumor effects of animal IFN are dependent on dosage and time but have been demonstrated at concentrations below toxic levels. Accordingly, numerous investigations and clinical trials have been and continue to be conducted into the antitumor and anticancer properties of IFNs. These include treatment of several malignant diseases such as osteosarcoma, acute myeloid leukemia, multiple myeloma and Hodgkin's disease *(Texas Reports,* pp. 429-35). In addition, HuIFN has recently been shown to cause local tumor regression when injected into subcutaneous tumoral nodules in melanoma- and breast carcinoma affected patients (T. Nemoto *et al.,* "Human Interferons And intralesional Therapy Of Melanoma And Breast Carcinoma", *Amer, Assoc. For Cancer Research,* Abs. Nr. 994, p. 246 (1979)). Although the results of these clinical tests are encouraging, the antitumor and anticancer applications of IFN have been severely hampered by lack of an adequate supply of purified IFN.

Today, HuIFN-α is produced either through human cells grown in tissue culture or through human leukocytes collected from blood donors. $2.6 \times 10^9$ IU of crude HuIFN-α have been reported from 800 l of cultured Namalva cells (P. J. Bridgen *et al., supra).* At very large blood centres, *e.g.,* the Finish Red Cross Center in Helsinki, Finland, the production capacity is about $10^{11}$ IU of crude HuIFN-α annually. Since dosage is typically $3 \times 10^6$ IU per patient per day, these sources are not adequate to provide the needed commercial quantities of HuIFN-α. Therefore, production of HuIFN-α by other procedures is desirable. Because the specific activity of IFN-α is high, in the order of $4.0 \times 10^8$ to $10^9$ IU/mg, the amount of HuIFN-α required for commercial applications is low. For example, 100 grams of pure HuIFN-α would provide between 3 and 30 million doses.

Recent advantages in molecular biology have made it possible to introduce the DNA coding for specific nonbacterial eukaryotic proteins into bacterial cells. In general, with DNA other than that prepared via chemical synthesis, the construction of such recombinant DNA molecules comprises the steps of producing the single-stranded DNA copy (cDNA) of a purified messenger RNA (mRNA) template for the desired protein; converting the cDNA to double-stranded DNA; linking the DNA to an appropriate site in an appropriate cloning vehicle to form a recombinant DNA molecule and transforming an appropriate host with that recombinant DNA molecule. Such transformation may permit the host to produce the desired protein.

Several non-bacterial proteins and genes have been obtained in *E. coli* using recombinant DNA technology. These include a protein displaying rat proinsulin antigenic determinants (L. Villa-Komaroff *et al.,* "A Bacterial Clone Synthesizing Proinsulin", *Proc. Natl. Acad. Sci. USA,* 75, pp. 3727-31 (1978)), rat growth hormone (P. H. Seeburg *et al.,* "Synthesis Of Growth Hormone By Bacteria", *Nature* 276, pp. 795-98 (1978)), mouse dihydrofolate reductase (A. C. Y. Chang *et al.,* "Phenotypic Expression In *E. coli* Of A DNA Sequence Coding For Mouse Dihydrofolgate Reductase", *Nature,* 275, pp. 617-24 (1978)), human somatostatin (K. Itakura *et al.,* "Expression in *Escherichia coli* Of A Chemically Synthesized Gene For the Hormone Somatostatin", *Science,* 198, pp. 1056-63 (1977)); European patent applications 0,001,929, 0,001,930, and 0,001,931 and cognate applications in other countries), the A and B polypeptide chains of human insulin (D. V. Goeddel *et al.,* "Expression in *Escherichia coli* Of Chemically Synthesized Genes For Human Insulin", *Proc. Natl. Acad. Sci. USA,* 76, pp. 106-10 (1979) and the European and related patent specifications, *supra),* antigens of human hepatitis B virus (C. J. Burrell *et al.,* "Expression In *Escherichia coli:* Of Hepatitis B Virus DNA Sequences Cloned In Plasmid pBR322", *Nature,* 279, pp. 43-7 (1979) and M. Pasek *et al.,* "Hepatitis B Virus Genes And Their Expression In *E. coli. Nature,* 282, pp. 575-79 (1979)), human growth hormone (D. V. Goeddel *et al.,* "Direct Expression In *Escherichia coli* Of A DNA Sequence Coding For Human Growth Hormone", *Nature,* 281, pp. 544-51 (1979)), SV40 t antigen (T. M. Roberts *et al.,* "Synthesis Of Simian Virus 40 t Antigen In *Escherichia coli', Proc. Natl, Acad. Sci. USA,* 76, pp.5596-600 (1979)). and human fibroblast interferon (HuIFN-β) (T. Taniguchi *et al.,* "Construction And Identification Of A Bacterial Plasmid Containing The Human Fibroblast Interferon Gene Sequence", *Proc. Japan Acad.,* 55, Ser. B, pp. 464-69 (1979) together with personnel communication 1980).

None of these recombinant DNA processes, however is directed, as is this invention, toward the synthesis of HuIFN-α. This is the problem to which the present invention is addressed. Its solution is not facilitated as were the above described recombinant DNA schemes by the availability of the sequence information required to prepare a synthetic gene (e.g., somatostatin) or of a cell type or virus rich in a particular DNA sequence (e.g., hepatitis viral antigen) of mRNA species (e.g., rat insulin) which allows preparation and identification of bacterial clones containing the desired hybrid DNA, or of a system allowing the selection of *E. coli* host that express the desired protein (e.g., mouse dihydrofolate reductase). Neither is it aided by the report of a plasmid which is said to contain a DNA sequence that hybridizes to a mRNA from a poly(A) RNA, that mRNA producing HuIFN-β activity in oocytes (e.g., fibroblast interferon). Nor is the solution of the present invention addressed as is the apparent suggestion of Research Disclosure No. 18309, pp. 361-62 (1979) to preparing pure or substantially pure HuIFN-αmRNA before cloning of the HuIFN-α gene.

Finally, it should be recognized that the selection of a DNA sequence or the construction of a recombinant DNA molecule which hybridizes to a mRNA from polyA RNA, that mRNA producing HuIFN activity in oocytes, is not sufficient to demonstrate that the DNA sequence or the hybrid insert of the recombinant DNA molecule corresponds to HuIFN. Instead, only the production of a polypeptide that displays an immunological or biological activity of HuIFN can actually demonstrate that the selected DNA sequence or constructed recombinant DNA molecule corresponds to HuIFN. More importantly, it is only after HuIFN activity is shown that the DNA sequence, recombinant DNA molecule or sequences related to them may be employed to select other sequences corresponding to HuIFN in accordance with this invention.

It will therefore be appreciated from the foregoing that the problem of producing HuIFN-α with the use of recombinant DNA technology is much different than any of the above described processes. Here, a particular DNA sequence of unknown structure - that coding for the expression of HuIFN-α in an appropriate host - must be found in and separated from a highly complex mixture of DNA sequences in order for it to be used in the production of HuIFN-α. Moreover. this location and separation problem is exacerbated by the predicted exceedingly low concentration of the desired DNA sequence in the complex mixture and the lack of an effective means for rapidly analyzing the many DNA sequences of the mixture to select and separate the desired sequence.

DISCLOSURE OF THE INVENTION

The present invention solves the problems referred to by locating and separating DNA sequences that code for the expression of HuIFN-α in an appropriate host and thereby providing DNA sequences, recombinant DNA molecules and methods by means of which a host is transformed to produce a polypeptide displaying an immunological or biological activity of human leukocyte interferon.

By virtue of this invention, it is possible to obtain polypeptide(s) displaying an immunological or biological activity of HuIFN-α for use in antiviral, antitumor or anticancer agents and methods. This invention allows the production of these polypeptides in amounts and by methods hitherto not available.

As will be appreciated from the disclosure to follow, the DNA sequences and recombinant DNA molecules of the invention are capable of directing the production, in an appropriate host, of a polypeptide displaying an immunological or biological activity of HIFN-α. Replication of these DNA sequences and recombinant DNA molecules in an appropriate host also permits the production in large quantities of genes coding for these polypeptides. The molecular structure and properties of these polypeptides and genes may be readily determined. The polypeptides and genes are useful, either as production in the host or after appropriate derivatization or modification, in compositions and methods for detecting and improving the production of these products themselves and for use in antiviral and antitumor or anticancer agents and methods.

This process is therefore distinguishable from the prior processes, above mentioned, in that this process, contrary to the noted prior processes, involves the preparation and selection of DNA sequences and recombinant DNA molecules which contain appropriate DNA sequences which code for at least one polypeptide displaying an immunological or biological activity of HuIFN-$\alpha$.

It will be appreciated from the foregoing that a basic aspect of this invention is the provision of a DNA sequence which is characterized in that it codes for a polypeptide displaying an immunological or biological activity of an $\alpha$-type interferon for use in cloning or expression in bacteria, yeasts or animal cells or in selecting DNA sequences that code for an $\alpha$-type interferon, the DNA sequence being selected from:

(1) the DNA inserts of Z-pBR322(Pst)/HcIF-4c, Z-pBR322(Pst)/HcIF-2h, Z-pBR322(Pst)/HcIF- SN35, Z-pBR322(Pst)/HcIF-SN42 and Z-PKT287(Pst)/HcIF-2H-AH6, said DNA inserts being exemplified, but not limited to, the DNA inserts of the recombinant DNA molecules carried by the microorganisms identified by accession numbers DSM 1699-1703, respectively,

(2) the DNA inserts of Z-pBR322(Pst)/HcIF-II-206 and Z-pBR322(Pst)/HcIF-SN35-AHL6, said DNA inserts being exemplified, but not limited to, the DNA inserts of the recombinant DNA molecules carried by the microorganisms identified by accession numbers ATCC 31633-31634, respectively.

(3) the hybridizing portion of each of:

HchrIF-A, the subcloned HindIII fragment of chr 3 in E. coli HB101 (DSM 1914);

HchrIF-B, the subcloned EcoRI fragment of chr 12 in E.coli HB101 (DSM 1915);

HchrIF-C, the subcloned HindIII fragment of chr 12 in E.coli HB101 (DSM 1916);

HchrIF-D, the subcloned EcoRI fragment of chr 13 in E.coli HB101 (DSM 1917);

HchrIF-E, the subcloned EcoRI fragment of chr 23 in E.coli HB101 (DSM 1918);

HchrIF-F, the subcloned HindIII fragment of chr 23 in E.coli HB101 (DSM 1919);

HchrIF-G, the subcloned EcoRI fragment of chr 26 in E.coli HB101 (DSM 1920);

HchrIF-H, the subcloned HindIII fragment of chr 26 in E.coli HB101 (DSM 1921);

HchrIF-I, the subcloned HindIII/BamHI fragment of chr 35 in E.coli HB101 (DSM 1922)

HchrIF-J, the subcloned BamHI fragment of chr 35 in E.coli HB101 (DSM 1923);

HchrIF-K, the subcloned Tac-Tac fragment of chr 23 in E.coli HB101 (ATCC 31760);

HchrIF-L, the subcloned BglII-BglII fragment of chr 101 in E.coli HB101 (ATCC 31761)

HchrIF-M, the subcloned HindIII-HindIII fragment of chr 10r in E.coli HB101 (ATCC 31762)

HchrIF-N, the subcloned BglII-BglII fragment of chr 26 in E.coli HB101 (ATCC 31763)

HchrIF-O, the subcloned HindIII-HindIII fragment of chr 30 in E.coli HB101 (ATCC 31764)

HchrIF-P, the subcloned BglII-Tac fragment of chr 13 in E.coli HB101 (ATCC 31765); and

HchrIF-Q, the subcloned BglII-Tac fragment of chr 161 in E.coli HB101 (ATCC 31766).

(4) DNA sequences which hybridize to any of the foregoing DNA inserts and which code of a polypeptide of the IFN-$\alpha$ type, and

(5) DNA sequences which are degenerate as a result of the genetic code to the DNA sequences and inserts defined in paragraphs (1) to (5) and which code for a polypeptide of the IFN-$\alpha$ type.

These sequences permit the production of interferon and interferon-like polypeptides in hosts selected from bacteria, yeasts or animal cells according to the processes of this invention.

BRIEF DESCRIPTION OF THE DRAWINGS

Figure 1 is a schematic outline of one embodiment of a process of this invention for preparing a mixture of recombinant DNA molecules. some of which are characterized by inserted DNA sequences that code for polypeptides of this invention.

Figure 2 is a schematic outline of the initial clone screening process of this invention.

Figure 3 is a schematic outline of one embodiment of a clone screening process using DNA sequences prepared in accordance with the invention.

Figure 4 is a restriction map of one of the clones of the invention. The positions of the restriction sites are based on fragment sizing by agarose gel electrophoresis. Figures 8-10 display the position of these restriction sites as determined by nucleotide sequence data.

Figure 5 is a schematic outline of the process of determining the orientation of a DNA insert in one recombinant DNA molecule of this invention.

Figure 6 displays the partial nucleotide sequence of some cloning vehicles useful in accordance with this invention.

Figure 7 displays the results of a Sephadex G-100 fractionation of supernatant prepared from a bacterial culture of this invention.

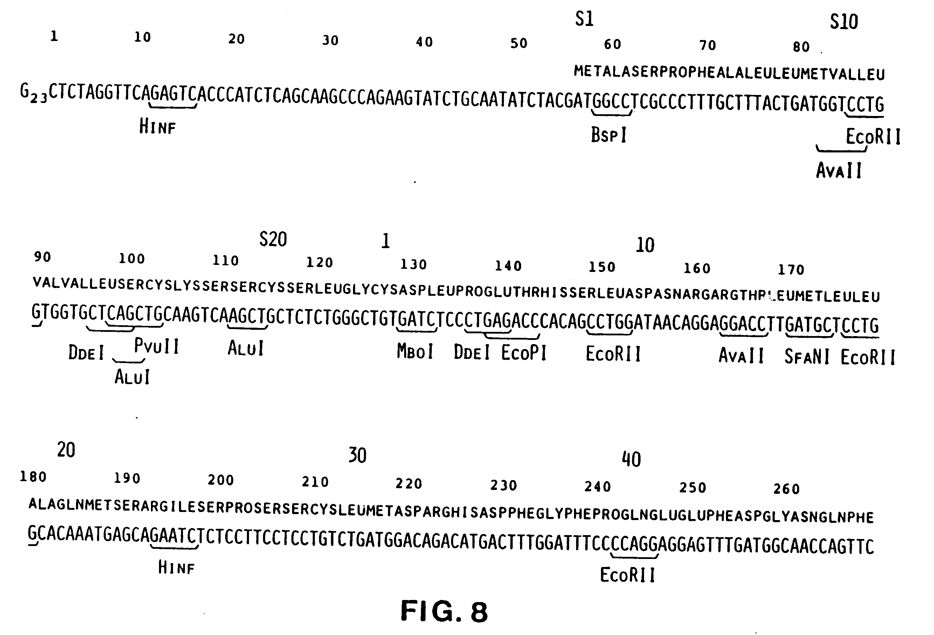

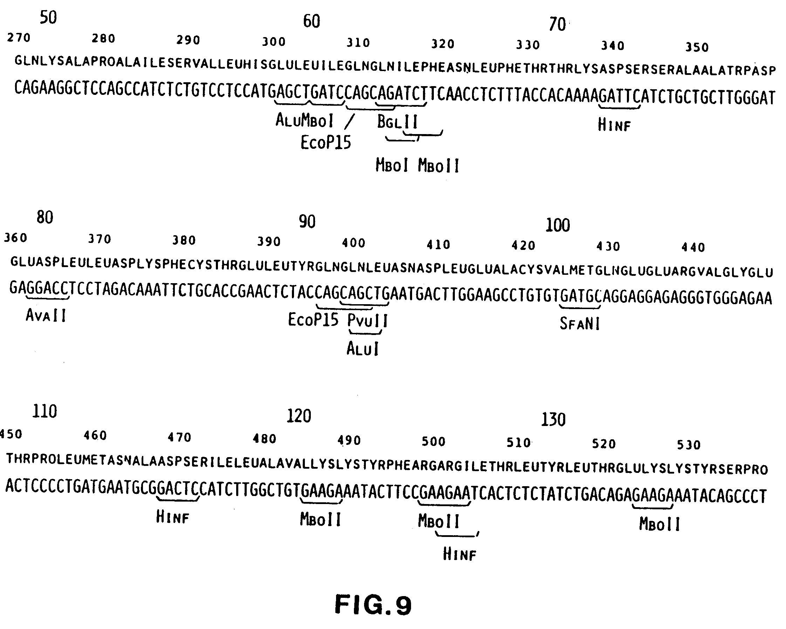

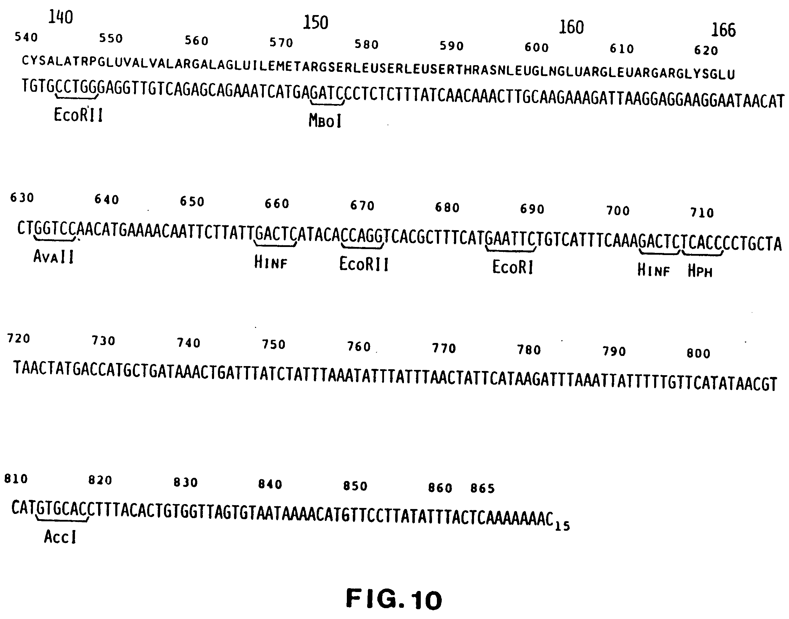

Figures 8-10 display the nucleotide sequence of a DNA insert to a recombinant DNA molecule of this invention. The sequence is numbered from the nucleotide following the polyG 5' tail to the nucleotide before the polyA residues and polyC 3' tails. Nucleotides 57-125 represent a signal sequences and nucleotides 125-626 represent the "mature" interferon and the stop codon. The amino acid sequence of the signal sequence is depicted above its nucleotide sequence in lower case letters and the amino acid sequence of the "mature" interferon is depicted above its nucleotide sequence in upper case letters. Various restriction endonuclease recognition sites in this gene are also depicted in Figures 8-10, these sites being determined by analysis of nucleotide sequence data.

Figure 11 is a schematic comparison of the restriction maps of two DNA inserts of recombinant DNA molecules of this invention.

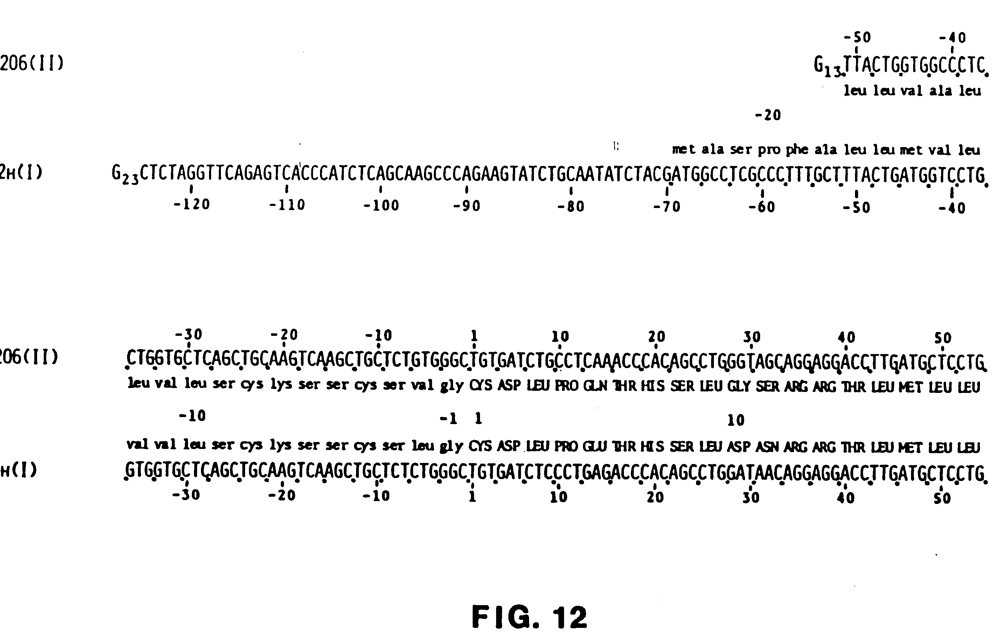

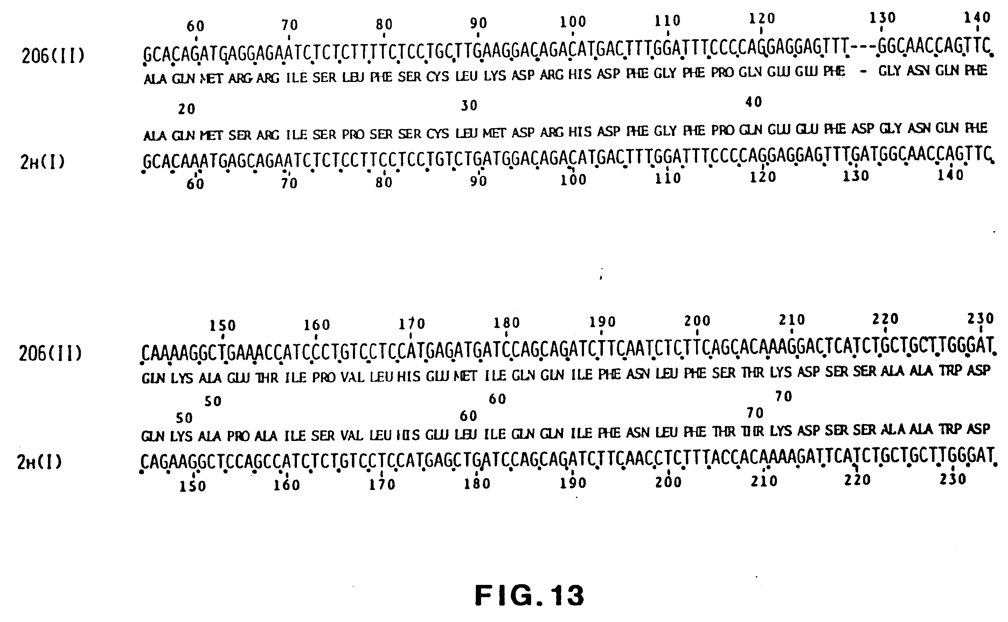

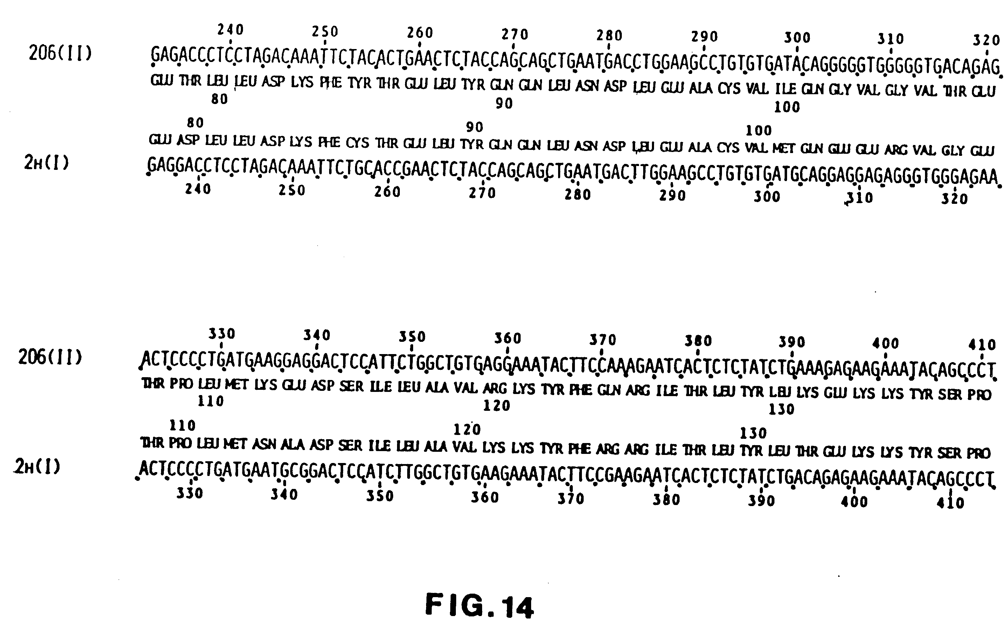

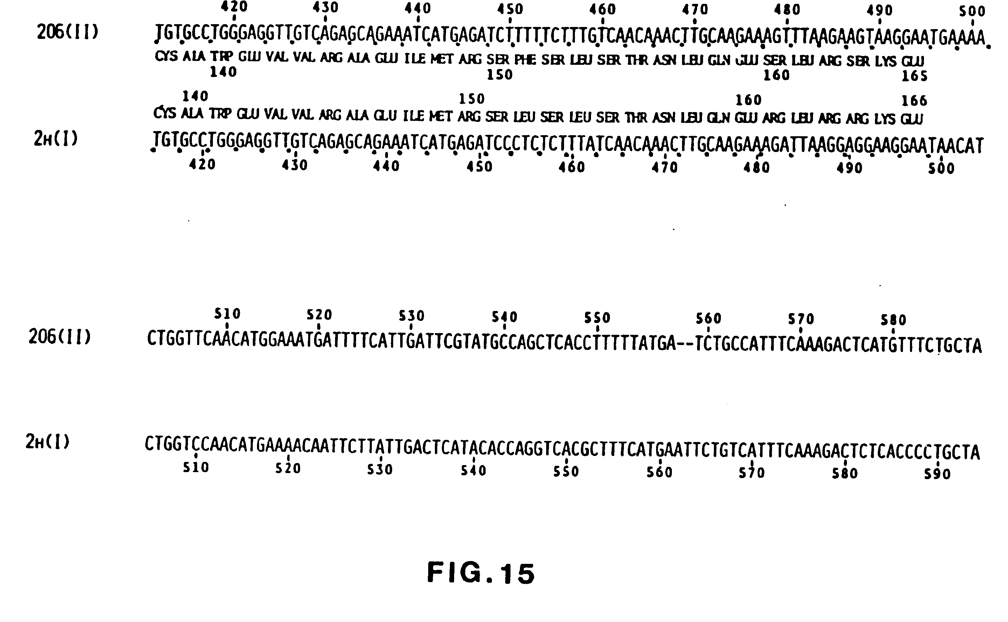

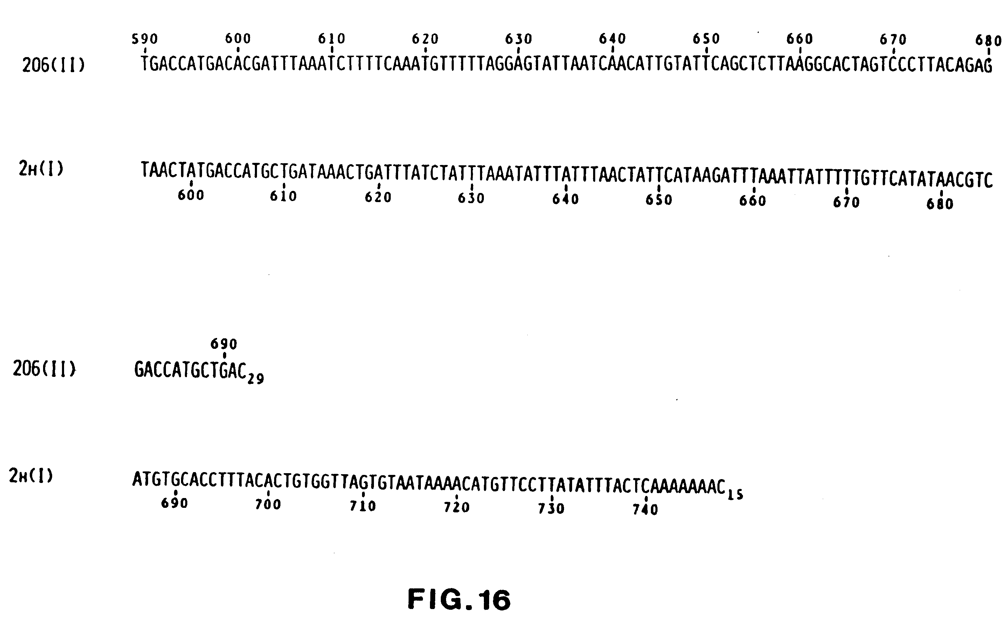

Figures 12-16 display the nucleotide sequences of two DNA inserts of recombinant DNA molecules of this invention. The sequences are numbered from the nucleotide following the polyG 5' tail to the nucleotide before the polyA residues and polyC 3' tails. The amino acid sequence of the signal sequence for each of these inserts is depicted above its respective nucleotide. sequence in lower case letters and the amino acid sequence of the "mature" interferon is depicted above its nucleotide sequence in upper case letters. Streuli et al., Science, 209(19 September 1980), pages 1343 to 1347 discloses the displayed DNA and amino acid sequences for clone 206(II).

Figure 17 displays a partial restriction map of Z-pBR322(Pst)/Hc/F-II 206 and the sequencing strategy employed to determine the nucleotide sequence of the Hif-II-206 fragment displayed in Figures 12-16.

Figure 18 displays the partial restriction maps of a series of hybrid phages which hybridize to the Hif-2h fragment.

Figure 19 disolays a partial restriction map of the hybrid insert of Z-paR322Pst/Hchrlf-35HBα and the sequencing strategy employed to determine its nucleotide sequence.

Figures 20-23 display the nucleotide sequence of the HchrIF-35HBα framant and the amino acid sequence derived from it.

Figure 24 displays partial linkage maps for HuIFN-α related genes. The arrows show regions which form R-loops with induced leukocyte poly(A) RNA. The hatched box (chr-16) indicates the sequence which was inferred from blotting experiments. but was not revealed by R-loop mapping.

Figure 25 is a schematic outline of the construction of plasmid C8-IFN-α1.

Figure 26 is a schematic outline of the construction of plasmid LAC-AUG(α2).

Figure 27 displays the reconstruction of the AUG initiation codon and the CYS initial codon in the construction of LAC-AUG(α2).

Figure 28 is a schematic outline of the construction of a plasmid C8-IFN-α2 and the hybrid molecules I, II, III and IV.

Figures 29-32 display the nucleotide sequence and amino acid sequence encoded thereby for IFN-α4b and its signal sequence.

## BEST MODE OF CARRYING OUT THE INVENTION

In order that the invention herein described may be more fully understood the following detailed description is set forth.

In the description the following terms are employed:

*Nucleotide*-A monomeric unit of DNA or RNA consisting of a sugar moiety (pentose), a phosphate, and a nitrogenous heterocyclic base. The base is linked to the sugar moiety via the glycosidic carbon (1' carbon of the pentose). That combination of a base and a sugar is called a nucleoside. Each nucleotide is characterized by its base. The four DNA bases are adenine ("A"), guanine ("G"), cytosine ("C") and thymine ("T"). The four RNA bases are A, G, C and uracil ("U").

*DNA Sequence*-A linear array of nucleotides connected one to the other by phosphodiester bonds between the 3' and 5' carbons of adjacent pentoses.

*Codon*-A DNA sequence of three nucleotides (a triplet) which encodes through mRNA an amino acid, a translation start signal or a translated termination signal. For example, the nucleotide triplets TTA, TTG, CTT, CTC, CTA and CTG encode for the amino acid leucine ("Leu"), TAG, TAA and TGA are translation stop signals and ATG is a translation start signal.

*Reading Frame*-The grouping of codons during translation of mRNA into amino acid sequences. During translation the proper reading frame must be maintained. For example, the sequence GCTGGTTGTAAG may be translated into three reading frames or phases, each of which affords a different amino acid sequence:

*GCT GGT TGT AAG*-Ala-Gly-Cys-Lys

*G CTG GTT GTA* AG-Leu-Val-Val

GC *TGG TTG TAA* G-Trp-Leu-(STOP)

*Polypeptide*-A linear array of amino acids connected one to the other by peptide bonds between the α-amino and carboxy groups of adjacent amino acids.

*Genome*-The entire DNA of a cell or a virus. It includes *inter alia* the structural genes coding for the polypeptides of the substance, as well as operator, promoter and ribosome binding and interaction sequences, including sequences such as the Shine-Dalgarno sequences.

*Structural Gene*-A DNA sequence which encodes through its template or messenger RNA ("mRNA") a sequence of amino acids characteristic of a specific polypeptide.

*Transcription*-The process of producing mRNA from a structural gene.

*Translation*-The process of producing a polypeptide from mRNA.

*Expression*-The process undergone by a structural gene to produce a polypeptide. It is a combination of transcription and translation.

*Plasmid*-A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet$^R$) transforms a cell previously sensitive to tetracycline into one which is resistant to it. A cell transformed by a plasmid is called a "transformant".

*Phage or Bacteriophage*-Bacterial virus many of which consist of DNA sequences encapsidated in a protein envelope or coat ("capsid").

*Cloning Vehicle*-A plasmid, phage DNA or other DNA sequences which are able to replicate in a host cell, which are characterized by one or a small number of endonuclease recognition sites at which such DNA sequences may be cut in a determinable fashion without attendant loss of an essential biological function of the DNA, *e.g.,* replication, production of coat proteins or loss of promoter or binding sites, and which contain a marker suitable for use in the identification of transformed cells, *e.g.,* tetracycline resistance or amplicillin resistance. A cloning vehicle is often called a vector.

*Cloning*-The process of obtaining a population of organisms or DNA sequences derived from one such organism or sequence by asexual reproduction.

*Recombinant DNA Molecule* or *Hybrid DNA*-A molecule consisting of segments of DNA from different genomes which have been joined end-to-end outside of living cells and have the capacity to infect some host cell and be maintained therein.

*Expression Control Sequence*-A sequence of nucleotides that controls and regulates expression of structural genes when operatively linked to those genes. They include the *lac* system, the *trp* system, major operator and promoter regions of phage λ, the control region of fd coat protein and other sequences known to control the expression of genes of prokaryotic or eukaryotic cells and their viruses.

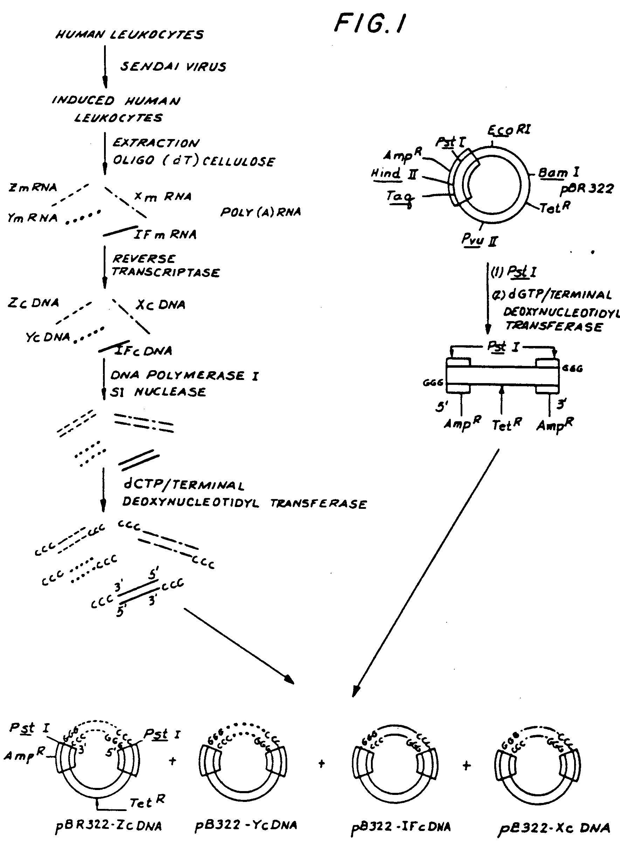

Referring now to Figure 1, we have shown therein a schematic outline of one embodiment of a process for preparing a mixture of recombinant DNA molecules, some of which are characterized by inserted DNA sequences that code for polypeptides having an immunological or biological activity of human leukocyte interferon.

## PREPARATION OF POLY(A) RNA CONTAINING HUMAN INTERFERON mRNA (IFN-αRNA)

Human leukocytes were induced for 5 hours at 37°C with Sendai virus and extracted to yield a poly(A) RNA mixture containing human leukocyte interferon mRNA ("HuIFN-αmRNA"). Induction was by the Cantell procedure *(The Interferon System,* pp. 130-31 and the references cited therein). The poly(A) RNA mixture is illustrated without regard to its actual proportions in Figure 1. Induced leukocytes were harvested and $10^{11}$ cells were resuspended in 1 l of a solution containing 8 g NaCl, 0.2 g KCl, 1.15 g $Na_2HPO_4 \cdot 2H_2O$ and 0.2 g $KH_2PO_4$ dissolved in 1 l of water ("PBS") and added slowly with vigorous stirring to 17 l 20 mM Tris-HCl (pH 7,5),1 mM EDTA ("TE buffer"),2% sodium dodecyl sulfate ("SDS") in a 50 l separatory funnel. Self-digested Pronase (Calbiochem) was added to 200 μg/ml and the solution stirred for 1 h at room temperature. $10^6$ counts/minute ("cpm") of $^{125}$I-globin mRNA were added as a marker for recovery of the poly(A) RNA and to control for mRNA degradation during subsequent steps. 2M Tris-HCl (pH 9) in an amount equal to 1/20 of the total volume ("1/20 vol") was added and the mixture extracted with vigorous stirring with 15 l of redistilled phenol for 10 min. Three l chloroform were added and the mixture stirred for 5 min. After allowing 30 min for phase separation, the aqueous phase was removed and extracted again with phenol and chloroform. The resultant aqueous phase, totalling 19.1 l, was combined with 60 g SDS. Nucleic acids were precipitated from the aqueous phase with 1/10 vol 3M sodium acetate (pH 5.5) and 2 vol ethanol.

After storage overnight at -20°C, the fibrous nucleic acid precipitate was removed by filtration through a plastic tea sieve. This material was then stirred with 200 ml TNE (50 mM Tris-HCl (pH 7.5), 100 mM NaCl, 5

mM EDTA) containing 0.5% SDS. It subsequently dissolved on addition of a further 350 ml of that solution. The non-fibrous precipitate was collected by centrifugation in 1 l Sorvall bottles in a Sorvall RC-3 centrifuge for 15 min at 5,000 rpm and dissolved in 350 ml TNE containing 0.5% SDS. The two TNE solutions were combined, extracted 3 times with 1 vol phenol, 3 times with 1/2 vol ether and 3 times with 1 vol ether. RNA recovery from the aqueous phase totalled 775 mg, as measured by absorbance at 260 nm.

Isolation of the poly(A) RNA mixture was achieved by repeated batch adsorption to oligo(dT) cellulose (type 7 P-L Biochemicals, Inc.). 2.7 grams oligo(dT) cellulose were added to 500 ml, *i.e.*, about half of the RNA-containing solution described above. After stirring for 1 h at room temperature to effect adsorption of the poly(A) RNA to the oligo(dT) cellulose, the cellulose and the mixture of mRNAs bound to it were collected by centrifugation and washed once with 50 ml TNE and a second time with 15 ml TNE. The bound poly(A) RNA was then eluted by five successive washes with 2 ml $H_2O$. The yield was 860 µg poly(A) RNA as measured by optical density (Preparation A). The supernatant RNA solution from the first adsorption was subjected to two further adsorption cycles, exactly as described above. The second and third adsorptions yielded 600 µg and 170 µg RNA respectively and were combined

(Preparation B).

RNA was assayed for HuIFN-αmRNA by injection into *Xenopus laevis* oocytes(The *Interferon System,* pp. 93-95): RNA was dissolved in 15 mM Tris-HCl (pH 7.5). 88 mM NaCl ("TNK buffer") to give a concentration of about 1 mg/ml. Fifty nl of this solution were injected into each of 50 oocytes. The oocytes were incubated overnight at room temperature in Barth medium (Gurdon, *J. Embryol and Exper. Morph.,* 20, pp. 401-414 (1968) and Barth, *J Embryol and Exper. Morph.,* 7, pp. 210-222 (1959)). The incubated oocytes were then rinsed and homogenized with a Pasteur pippette in a 1.5 ml Eppendorf centrifuge tube in 0.5 ml 52 mM Tris glycine buffer (pH 8.9). The mixture was centrifuged for 2 min in an Eppendorf centrifuge and the supernatant was drawn off and frozen at -20°C for assay. IFN-α activity was determined by the plaque reduction assay described by H. Strander and K. Cantell, "Production Of Interferon By Human Leukocytes In Vitro", *Ann. Med. exp. Fenn.,* 44, pp. 265-73 (1966). One unit IFN-α reduces virus plaques by 50%. The potency of an IFN-a preparation is expressed relative to the human reference HuIFN-α 69/19 (International Symposium on Standardization of Interferon and Interferon Inducers, 1969). Alternatively, the assay was based on the reduction of cytopathic effect, essentially as described by W. E. Stewart, II and S. E. Sulkin, "Interferon Production In Hamsters Experimentally Infected With Rabies Virus", *Proc. Soc. Exp. Biol. Med.,* 123, pp. 650-3 (1966), except that human CCL-23 cells were used and that challenge was with Mengo virus. The oocyte extracts had 300 IU of IFN-α activity per µg of RNA injected. In later assays inclubation of injection oocytes was for 48 hrs and only the incubation medium was assayed because most of the interferon is excreted by the oocytes (A. Coleman and J. Morser, "Export Of Proteins From Oocytes Of *Xenopus laevis",* Cell, 17, pp. 517-26 (1979)). For further purification of the poly(A) RNA sufficient 0.5 M ethylene diamine tetraacetic acid ("EDTA") was added to the poly(A) RNA Preparation A to bring the concentration to 5 mM EDTA. The resultant solution was extracted twice with an equal vol of TNE-saturated phenol and 5 times with an equal vol of ether. It was then passed through a 0.1-ml Chelex-100 Bio-Rad column, heated for 90 sec at 100°C and layered onto a 13-ml 5-23% sucrose gradient containing 50 mM Tris-HCl (pH 7.5),1 mM EDTA, 0.2 M NaCl. 10,000 cpm of 5'-terminally $^{32}$P-labeled DNA fragments produced by simultaneous digestion of pBR322 with restriction enzymes *Hind*III and *Pst*I (New

England
Biolabs), were added as size markers. Centrifugation was in an SW40 rotor at 10°C and 35.000 rpm for 16 h. Fractions (0.6 ml) were collected with an ISCO gradient collector at 1 ml/min. The fractions were assayed for HuIFN-αmRNA as described above and their position relative to the $^{32}$P-DNA markers was noted for future reference. In subsequent centrifugations, HuIFN-αmRNA-containing fractions were identified relative to the markers. The fractions with HuIFN-αmRNA activity contained 80 µg of poly(A) RNA. They were mixed with 2 mol TNE containing 0.5% SDS and 0.02% polyvinyl sulfate (in later preparations polyvinyl sulfate was omitted) and applied to a 50-µl oligo(dT) cellulose column. After washing the column as described above, 40 µg of the RNA mixture were eluted with 4 washes of 0.6 ml sterile distilled water. After ethanol precipitation, the RNA was dissolved to 1 mg/ml in 0.5 mM EDTA.

An assay for HuIFN-αmRNA activity was carried out as described above on a portion of the poly(A) RNA precipitate. It had a specific activity of 3600 IU interferon/µg of RNA injected. Therefore, the sucrose gradient had enriched the poly(A) RNA about 10-fold in regard to HuIFN-αmRNA. In a subsequent, similar preparation about a 40-fold enrichment was obtained. Preparation B was purified similarly and, since it had a similar specific activity as Preparation A, the two were pooled.

At this point it should be recognized that even the poly(A) RNA product obtained from the sucrose gradient contains a very large number of different mRNA's. Except for the mRNA specific for IFN-α, the other mRNAs are undesirable contaminants (Figure 1). Unfortunately, these contaminant RNAs behave similarly to HuIFN-αmRNA throughout the remainder of the cloning process of this invention. Therefore, their presence in the poly(A) RNA will result in the ultimate preparation of a large number of unwanted bacterial clones which contain genes that code for polypeptides other than IFN-α. This contamination presents complex screening problems in the isolation of the desired IFN-α hybrid clones. In the case of IFN-α, the screening problem is further exacerbated by the lack of a sufficiently purified sample of HuIFN-αmRNA or DNA or portion thereof to act as a screening probe for the identification of the desired clones. Therefore, the screening process for the IFN-α clones is very time-consuming and difficult. Further, because only a very small percentage of IFN-α clones themselves are expected to express IFN-α in a biologically active or immunologically active form, the isolation of an active clone is a "needle in a haystack" screening process.

Advantageously, we may use recombinant DNA technology to provide a purified sample of HuIFN-αmRNA or cDNA or a portion thereof. This purified mRNA or cDNA can be used to screen rapidly very large numbers of bacterial clones and thereby markedly increase the probability of isolating a clone which expresses IFN-α in an active form.

## SYNTHESIS OF cDNA MIXTURE CONTAINING HuIFN-αcDNA

The poly(A) RNA enriched for IFN-αmRNA (Prepartion A + B) was used as a template to prepare single-stranded complementary DNA (cDNA) (Figure 1) *(Cf,* A. Efstratiadis *et al.,* "Full Length And Discrete Partial Reverse Transcripts Of Globin And Chorion mRNAs", *Cell,* 4, pp. 367-78 (1975) and references cited therein). The 800-μl reaction mixture contained 40 mM Tris-HCl (pH 7.5), 30 mM NaCl, 5 mM MgCl$_2$, 0.5 mM DTT (Cal-Biochem), 20 μg/ml oligo(dT) 12-18 (P & L Biochemicals), 5 mMdGTP (Schwarz), dCTP (Laevosan) and dTTP (Sigma), 5 mM ($^{32}$P-dATP (NEN, specific activity 100,000 cpm/nmole), 60 μg/ml poly(A) RNA and 280 units avian myeloblastosis virus (AMV) reverse transcriptase (a gift from Life Sciences, Inc., St. Petersburg, Florida). After incubation for 1 h at 37°C, 0.5 M EDTA and 20% SDS (recrystallized) were added to 10 mM EDTA and 0.1% SDS. The mixture was extracted with 1 vol phenol (distilled). The phenol phase was washed with 200 μl 200 mM Tris-HCl (pH 7.5), 1 mM EDTA and 0.1% SDS, and the aqueous phases combined. These were extracted with an equal vol ether (Fluka, pro anal.) and chromatographed on a 5 ml Sephadex G-100 column in TNE. Fractions of 0.1 ml were collected at 0.3 ml/min. Fractions displaying radioactivity (as measured by Cerenkov radiation) were combined and 3 M sodium acetate added to 0.3 M. The nucleic acids were precipitated with 2.5 vol of ethanol. After storage overnight at -20°C, the samples were centrifuged and the supernatant discarded. The precipitate was dissolved in 180 μl distilled water and transferred to a siliconized Eppendorf tube. 20 μl 5 M NaOH were added and the mixture kept at room temperature for 40 min. 20 μl of 5 M sodium acetate, 100 μl distilled water and 500 μl ethanol were added. After cooling overnight at -20°C, the resulting precipitate was collected by centrifugation at a force equivalent to 10,000 times the force of gravity (10000 × g) for 20 min at 0°C. The yield of single-stranded cDNA was 10 μg.

Again, it is to be understood that the single-stranded cDNA product prepared above is in reality a complex mixture of a large number of different cDNAs transcribed from the corresponding mRNAs present in the poly(A) RNA mixture (Figure 1). Only a very few of these cDNAs are IFN-α related, *i.e.,* HuIFN-αcDNAs. Another factor also acts to complicate the cDNA mixture - each mRNA species of the poly(A) RNA mixture is usually not transcribed completely. Instead, for each mRNA species the transcription process may stop before the end of the mRNA is reached. Therefore, a large variety of cDNA species may be produced from each mRNA species (not shown in Figure 1). Each species will behave more or less similarly in the subsequent cloning process so that bacterial clones will be produced which contain recombinant DNA molecules having only a fragment of the gene for a particular protein. The presence of these fragment-containing clones even further complicates the final clone screening process.

The sizes of the various single-stranded cDNAs were determined by electrophoresis of a small aliquot on a alkaline 2% agarose gel using 30 mM NaOH, 2 mM EDTA as electrolyte (M. W. McDonell et al., "Analysis Of Restriction Fragments of T7 DNA And Determination Of Molecular Weights By Electrophoresis In Neutral And Alkaline Gels", *J. Mol. Biol.,* 110, pp. 119-46 (1977)). The $^{32}$P-cDNA had a length of 600-1000 nucleotides, relative to single-stranded globin cDNA and $^{32}$P-labeled DNA fragments used as size markers.

## PREPARATION OF DOUBLE-STRANDED cDNA

The single-stranded cDNA may be rendered double-stranded by treatment with DNA polymerase I (T. Maniatis *et al.,* "Amplification And Characterization Of A β-Globin Gene Synthesized *In Vitro",* Cell, 8, pp. 163-82 (1979)). The precipitated single-stranded cDNA from above was dissolved in 200 μl H$_2$O, heated at 100°C for 2 min and incubation in 500 µl 0.1 M heat denatured potassium phosphate buffer (pH 6.9), 10 mM MgCl$_2$, 10 mM DTT (Calibiochem), I mM each of dATP (Merck), dGTP (Schwarz) and dCTP (Laevosan),1 mM $^3$H-dTTP (NEN, specific activity 100,000 cpm/nmole) and 1 50 units-ml of *E. coli* DNA polymerase I (Boehringer-Mannheim). After 6.5 h at 15°C,0,5 M EDTA and 20% SDS were added to 10 mM EDTA and 0.1% SDS. The mixture was then extracted with 500 µl phenol and the phenol phase was reextracted with 250 µl 20 mM Tris-HCl (pH 7.5) 5 mM EDTA ("TE buffer"). The two aqueous phases were combined and chromatographed on a 5-ml Sephadex G-100 column under the same conditions described previously. Sodium acetate (3 M) was added to 0.3 M and 2.5 vol ethanol were mixed into precipitate the DNA. A total of 13 µg DNA was recovered.

The DNA was treated with nuclease S$_1$, prepared by the method of R. C. Wiegand *et al.,* "Specificity Of The S$_1$ Nuclease *Aspergillus oryzae", J. Biol, Chem.,* 250, pp. 8848-55 (1975). The precipitated DNA was dissolved. in 250 µl S$_1$ buffer (0.2 m NaCl, 50 mM sodium acetate (pH 4.5), 10 mM zinc sulfate) and warmed at 37°C for 30 min. 1.5 µl S$_1$ enzyme (11 units/µ1) were added and the mixture incubated at 37°C for 30 min. SDS and EDTA were added to 0.1% SDS and 5 mM EDTA, and the mixture was extracted with 250 µl phenol. The phenol phase was washed with 100 µl TE buffer. The aqueous phases were combined and chromatographed on a Sephadex G-100 (Pharmacia) column in TNE, 0.1 ml fractions were collected at 0.3 ml/min and the Cerenkov radiation of each fraction was determined. 8 µg of double-stranded cDNA were recovered after precipitation with ethanol and sodium acetate as above.

Again, it must be recognized that the double-stranded cDNA produced above is a mixture of a large number of cDNAs and fragments thereof, only a very few of which are HuIFN-αcDNA or its fragments (Figure 1).

## CLONING OF DOUBLE-STRANDED DNA

A wide variety of host/cloning vehicle combinations may be employed in cloning the double-stranded cDNA prepared as above described. For example, useful cloning vehicles may consist of segments of chromosomal, non-chromosomal and synthetic DNA sequences, such as various known bacterial plasmids, *e.g.,* plasmids from *E. coli* including col E1, pCR1, PBR322 and their derivatives, wider host range plasmids, *e.g.,* RP4, phage DNA, *e.g.,* the numerous derivatives of phage λ, *e.g.,*NM 989, and vectors derived from combinations of plasmids and phage DNAs such as plasmids which have been modified to employ phage DNA or other expression control sequences or yeast plasmids such as the 2 µ plasmid or derivatives thereof. Useful hosts may include bacterial hosts such as strains of *E. coli, e.g., E. coli* HB 101, *E. coli* X1776, *E. coli* X2282, *E. coli* MRCI and strains of *Pseudomonas, Bacillus subtilis, Bacillus stearothermophilus* and other bacilli, yeasts and other fungi, animal or plant hosts such as animal (including human) of plant cells in culture or other hosts. Of course, not all host/vector combinations may be equally efficient. The particular selection of host/cloning vehicle combination may be made by those of skill in the art after due consideration of the principles set forth without departing from the scope of this invention.

Furthermore, within each specific cloning vehicle, various sites may be selected for insertion of the double-stranded DNA. These sites are usually designated by the restriction endonuclease which cuts them. For example, in pBR322 the *Pst*I site is located in the gene for β-lactamase, between the nucleotide triplets that code for amino acids 181 and 182 of that protein. This site was employed by Villa-Komaroff *et al., supra,* in their synthesis of protein displaying rat proinsulin antigenic determinants. One of the two *Hind*II endonuclease recognition sites is between the triplets coding for amino acids 101 and 102 and one of the several *Taq* sites at the triplet coding for amino acid 45 of β-lactamase in pBR322. In similar fashion, the *Eco*RI site and the *Pvu*II site in this plasmid lie outside of any coding region, the *Eco*RI site being located between the genes coding for resistance to tetracycline and ampicillin, respectively. This site was employed by Itakura *et al.* and Goeddel *et al.* in their recombinant synthetic schemes, *supra.* These sites are well recognized by those of skill in the art. It is, of course, to be understood that a cloning vehicle useful in this invention need not have a restriction endonuclease site for insertion of the chosen DNA fragment. Instead, the vehicle could be joined to the fragment by alternative means.

The vector or cloning vehicle and in particular the site chosen therein for attachment of a selected DNA fragment to form a recombinant DNA molecule is determined by a variety of factors, e.g., number of sites susceptible to a particular restriction enzyme, size of the protein to be expressed, susceptibility of the desired protein to proteolytic degradation by hose cell enzymes, contamination of the protein to be expressed by host cell proteins difficult to remove during purification, expression characteristics, such as the location of start and stop codons relative to the vector sequences, and other factors recognized by those of skill in the art. The choice of a vector and an insertion site for a particular gene is determined by a balance of these factors, not all selections being equally effective for a given case.

Although several methods are known in the art for inserting foreign DNA into a cloning vehicle or vector to form a recombinant DNA molecule, the method preferred for a first construction in accordance with this invention is described in Vila-Komaroff *et al., supra,* and displayed in Figure 1. This method is characterized by digesting the plasmid (in particular pBR322) with that restriction enzyme specific to the site chosen for the insertion (in particular PstI) and adding dGMP tails to the termini by terminal transferase. dGMP tails are added to the 5' termini of the cut plasmid to regenerate the *PstI* site and permit linkage to a cDNA fragment carrying the complementary tails. In similar fashion, the double-stranded cDNA is elongated by the addition of dCMP tails to the 3' termini to allow joining to the tailed plasmid. The tailed plasmid and cDNA are then annealed to insert the cDNA in the appropriate site of the plasmid and to circularize the hybrid DNA, the complementary character of the tails permitting their cohesion (Figure 1). The resulting combinant DNA molecule now carries a gene at the chosen restriction site (Figure 1).

Of course, other known methods of inserting DNA sequences into cloning vehicles to form recombinant DNA molecules are equally useful in this invention. These include, for example, direct ligation, synthetic linkers, exonuclease and polymerase-linked repair reactions followed by ligation, or extension of the DNA strand with DNA polymerase and an appropriate single stranded template followed by ligation.

It should, of course, be understood that the nucleotide sequences or cDNA fragment inserted at the selected site of the cloning vehicle may include nucleotides which are not part of the actual structural gene for the desired polypeptide or may include only a fragment of the complete structural gene for the desired protein. It is only required that whatever DNA sequence is inserted, a transormed host will produce a polypeptide having a biological or immunological activity of HuIFN-$\alpha$ or that the DNA sequence itself is of use as a hybridization probe to select clones which contain DNA sequences useful in the production of polypeptides having an immunological or biological activity of HuIFN-$\alpha$

The cloning vehicle or vector containing the foreign gene is employed to transform a host so as to permit that host to express the protein or portion thereof for which the hybrid DNA codes. The selection of an appropriate host is also controlled by a number of factors recognized by the art. These include, for example, compatibility with the chosen vector, toxicity of proteins encoded by the hydrid plasmid, ease of recovery of the desired protein, expression characteristic, biosafety and costs. A balance of these factors must be struck with the understanding that not all hosts may be equally effective for expression of a particular recombinant DNA molecule.

In the present synthesis, the preferred initial cloning vehicle is the bacterial plasmid pBR322 and the preferred initial restriction endonuclease site therein is the *PstI* site (Figure 1). The plasmid is a small (molecular weight approx. 2.6 megadaltons) plasmid carrying resistance genes to the antibiotics ampicillin (Amp) and tetracycline (Tet). The plasmid has been fully characterized (F. Bolivar *et al.,* "Construction And Characterization Of New Cloning Vehicles II. A Multi-Purpose Cloning System", Gene, pp. 95-113 (1977); J. G. Sutcliffe, "pBR322 Restriction Map Derived From The DNA Sequence: Accurate DNA Size Markers Up To 4361 Nucleotide Pairs Long", *Nucleic Acids Research,* 5, pp. 2721-28 (1978)). Insertion of the DNA product in this site provides a large number of bacterial clones each of which contains one of the DNA genes or fragments thereof present in the DNA product previously prepared. Again, only a very few of these clones will contain the gene of IRN-$\alpha$ or fragments thereof (Figure 1). The preferred host for initial cloning in accordance with the invention is *E. coli* HB101. Other experiments were conducted with *E. coli* X1776, a host described in British patent 1,516,458 and placed on deposit with the American Type Culture Collection, Rockville, Maryland, USA, where it has been assigned ATCC No. 31244.

## 1. Preparation of PstI-Cleaved, dGMP-elongated pBR322

Plasmid pBR322 (20 $\mu$g) was digested with 21 units *Pst*1 endonuclease (MRE Portion Downs or New England Biolabs) in 150 $\mu$l 10 mM Tris-HCl (pH 7.5), 6 mM MgCl$_2$, 50 mM NaCl, 6 mM 2-mercaptoethanol, 200 mg/$\mu$l bovine serum albumin ("BSA") (Calbiochem). After 2 h at 37°C, the mixture was extracted with 1 vol phenol-chloroform (1:1) and 1 vol ether and precipitated with ethanol.

Addition of homopolymeric dGMP tails (Figure 1) by terminal deoxynucleotidyl transferase (TdT) (purified according to F. J. Bollum, "Deoxynucleotide Polymerizing Enzymes From Calf Thymus Gland", in *Methods in Enzymology,* (L. Grossman and K. Moldave, eds.), Academic Press, New York, 128, pp. 591-611 (1968)) was done in a 328 $\mu$l reaction volume containing 100 mM sodium cacodylate (pH 7.2), 10 mM NaH$_2$PO$_4$, 5 mM MgCl$_2$ 1 mM dGTP, 50$\mu$g/$\mu$l BSA, and 3-6 units of TdT (purified as above) per $\mu$g of DNA. Incubation was at 37°C for 20 min. EDTA was added to 10 mM and the mixture extracted as above and dialyzed for 2 days against TNE buffer.

## 2. Preparation of dCMP-elongated DNA

Double-stranded DNA was elongated with dCMP residues by standard procedures *(E.g.,* Villa-Komaroff

*et al., supra).* 150 ng of the double-stranded cDNA described above were incubated in 8 μl 100 mM sodium cacodylate (pH 7.2), 2.5 mM $CoCl_2$, 50 μg/μl BSA, 0.1 mM dCTP containing 3-6 units of purified TdT per μg od DNA for 8 min at 27°C and then frozen at -20°C. As before, the dCMP-elongated DNA is a mixture of different species, only a very few of which are IFN-related (Figure 1).

### 3. Preparation of $Ca^{++}$-Treated E. coli X1776

A single colony of *E. coli* X1776 was inoculated into 100 ml tryptone medium (C. Weissmann and W. Boll, "Reduction Of Possible Hazards In The Preparation Of Recombinant Plasmid DNA", *Nature,* 261, pp. 428-29 (1976), supplemented with 100 μg/ml diaminopimelic acid (Koch-Light Laboratories), 10 μg/ml nalidixic acid (Calbiochem) and 10 μg/ml tetracycline (Achromycin®, American Cyanamid). The culture was grown at 37°C to an apparent optical density of 0.6 to 650 nm ($OD_{650}$) (as measured in a Beckman DB spectrophotometer) and chilled in ice for 30 min. The culture was then sedimented at 4000 rpm in a Sorvall H4 swinging bucket rotor, the cells washed with 50 ml 10 mM NaCl, repelleted by centrifugation, and resuspended in 20 ml 100 mM $CaCl_2$. The suspension was cooled in ice for 30 min, pelleted by centrifugation and resuspended in 4 ml of 100 mM $CaCl_2$ and kept on ice overnight for use. *E. coli* HB101 was prepared for transformation by the method of M. Mandel and A. Higa, "Calcium-Dependent Bacteriophage DNA Infection", *J. Mol. Biol.,* 53, pp. 159-62 (1970). Aliquots (0.5 ml) were kept frozen at -70°C and retained their activity for at least 3 months.

### 4. Annealing of dGMP-elongated pBR322 and dCMP-elongated DNA

The annealing of the tailed, *Pst*I-cleaved pBR322 and tailed cDNA was as described in J. Van den Berg *et al.,* "Comparison Of Cloned Rabbit And Mouse β-globin Genes Showing Strong Evolutionary Divergence of Two Homologous Pairs Of Introns", *Nature,* 276, pp. 37-44 (1978). 8 ng of dCMP-elongated DNA product were mixed with 22 ng of dGMP-elongated *Pst*I-cleaved pBR322 in 50 μl TNE buffer. Incubation was for 4 successive 1 h stages at 65°C, 46°C, 37°C and 20°C. 20 μl 100 mM Tris-HCl (pH 7.5), 100 mM $CaCl_2$, 100 mM $MgCl_2$ and 50 μl TNE buffer were added and the mixture cooled in ice for 20 min.

The product is, of course, a large mixture of different recombinant DNA molecules and some cloning vehicles without inserted DNA sequences. However, each recombinant DNA molecule contains a cDNA segment at the *Pst*I site. Each such cDNA segment may comprise a gene or a fragment thereof. Only a very few of the cDNA segments code of IFN or a portion thereof (Figure 1). The vast majority code for one of the other proteins or portions thereof whose mRNA's were part of the poly(A) RNA used in the process of this invention (Figure 1).

### 5. Transfection Of E. coli X1776 With The Annealed Hybrid Plasmids

The transfection of *E. coli* X1776 with the mixture of recombinant DNA molecules was as described in J. Van den Berg *et al., supra.* P3 containment facilities were used for the transfection process and all subsequent steps in which the resulting transformed bacteria were handled. The annealed pBR322 recombinant DNA molecules were added to 100 μl of $Ca^{++}$-treated *E. coli* X1776, prepared previously, and the mixture cooled in ice for 20 min, heated at 20°C for 10 min, and 0.6 ml tryptone medium added. The mixture was plated onto 2 tryptone medium agar plates supplemented as above. Transfection efficiency was $3.3 \times 10^4$ colonies per μg of annealed pBR322 transfecting DNA; native pBR322 gave $3 \times 10^6$ colonies per μg.

Since plasmid pBR322 includes the gene for tetracycline resistance, *E. coli* hosts which have been transformed with a plasmid having that gene intact will grow in cultures containing that antibiotic to the exclusion of those bacteria not so transformed. Therefore, growth in tetracycline-containing culture permits selection of hosts transformed with a recombinant DNA molecule or recyclized vector.

After 48 h at 37°C, individual colonies were picked and suspended in 100 μl tryptone medium (supplemented as above) in the wells of microtiter plates (Dynatech). After incubation at 37°C overnight, 100 μl 40% glycerol were mixed into each well. The plates were stored at -20°C and a library of 100,000 individual clones of transformed *E. coli* X1776 was prepared.

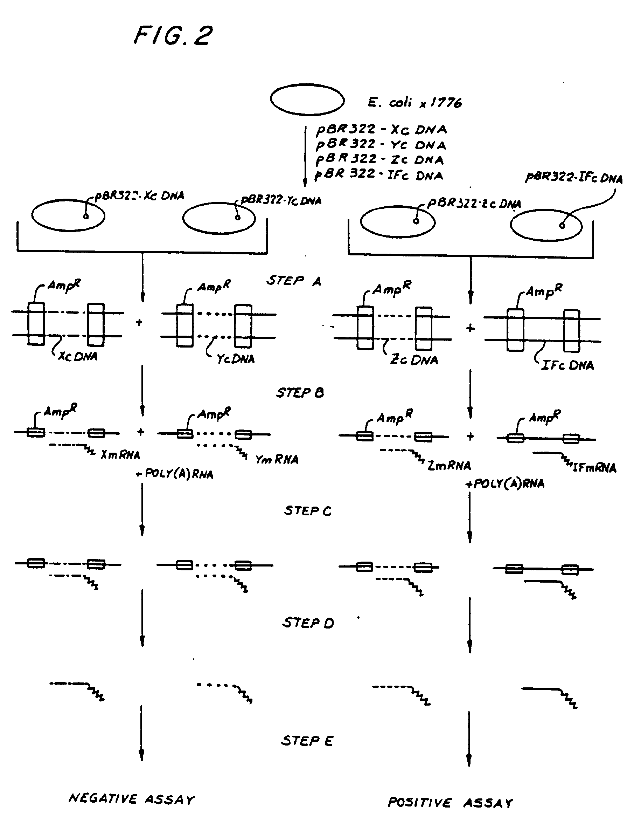

These 100,000 clones contain a variety of recombinant DNA molecules representing complete or partial copies of the mixture of mRNAs in the poly(A) RNA preparation from IFN-producing leukocytes (Figure 2). The majority of these will contain only a single recombinant DNA molecule. Only a very few of these recombinant DNA molecules are related to IFN. Accordingly, the clones must be screened to separate the IFN-related clones from the others.

## SCREENING FOR A CLONE CONTAINING HuIFN-αcDNA

There are several approaches to screen for backcterial clones containing human leukocyte interferon cDNA ("HuIFN-αcDNA"). These include, for example, RNA selection hybridization (Alwine *et al., infra)*, differential hybridization (T. P. St. John and R. W. Davies, "Isolation Of Galactose-Inducible DNA Sequences From Saccharomyces Cerevisiae By Differential Plaque Filter Hybridization", *Cell,* 16, pp. 443-452 (1979); Hoeijmakers *et al., infra),* hybridization with a synthetic probe (B. Noyes *et al.,* "Detection And Partial Sequence Analysis Of Gastrin mRNA By Using An Oligodeoxynucleotide Probe", *Proc. Natl. Acad. Sci. USA,* 76, pp.l 1770-1774 (1979)) or screening for clones that produce the desired protein by immunological (L. Villa-Komaroff *et al., supra)* or biological (A. C. Y. Chang *et al., supra)* assays. We have chosen RNA selection hybridization as being the most convenient and promising method for primary screening of clones containing IFN-αcDNA.

### A. RNA Selection Hybridization Assay

### 1. Overview Of The Initial Assay

Referring now to Figure 2, recombinant DNA was isolated from a culture of a mixture of 512 clones from the above library of clones (two mixtures of 2 clones shown in Figure 2) (Step A). The reason for selecting this batch size will be explained below. The recombinant DNA molecules were cleaved, denatured and hybridized to leukocyte poly(A) RNA containing IFN-αmRNA prepared as before (Step B). All recombinant DNA molecule-poly(A) RNA hybrids were separated from the non-hybridized poly(A) RNA (Step C). The poly(A) RNA was recovered from the hybrids and purified (Step D). The recovered RNA was assayed for IFN-αmRNA activity as above (Step E). If, and only if, the mixture of recombinant DNA molecules contains a recombinant DNA molecule having an inserted nucleotide sequence capable of hybridizing to the IFNmRNA in the poly(A) RNA under stringent hybridization conditions, will the mRNA released from that hybrid cause the formation of IFN-α in oocytes, because mRNA released from any other recombinant DNA molecule-poly(A) RNA hybrid will not be IFN-α-related. If a group of 512 clones gave a positive response, the clones were regrouped in 8 lots of 64, and each lot assayed as before. This process was continued until a single clone responding to this assay was identified.

There is no assurance that the recombinant DNA molecules and bacterial clone transformed therewith, which are thus identified, contain the complete IFN-αcDNA sequence of IFN-α or even that the DNA sequence actually codes for IFN-α. However, the recombinant DNA molecules will certainly contain extensive nucleotide sequences complementary to the IFN-αmRNA coding sequence. Therefore, the recombinant DNA molecule may at least be used as a source of a probe to screen rapidly other recombinant DNA molecules and clones transformed with them to identify further sets of clones which may contain an authentic and complete IFN-α nucleotide coding sequence.

### 2. Theoretical Considerations

The conditions for the hybridization (Step B) are critical. The absolute concentrations and the ratio of recombinant DNA molecule and poly(A) RNA must be chosen so as to take into consideration reaction rate and stoichiometry. The proper choice is difficult to make, because the proportion of IFN-αmRNA in the poly(A) RNA is not known. In order to assure controlled and adequate kinetics, the hybridization was carried out under conditions where the concentration of DNA sequences from the recombinant DNA molecules was in excess as compared to the estimated IFN-αmRNA concentration. In a mixture of 512 possible different recombinant DNA molecules, an IFN-α-related DNA sequence ("IFN-αR DNA") will either not occur (giving a negative assay), or it will constitute at least about 1/512 of the recombinant DNA molecule mixture and therefore the concentration of the IFN-αR DNA, if any, can thus be adjusted in the hybridization step to ensure adequate hybridization rates. In addition, the amount of the IFN-αR DNA in the reaction mixture must be sufficient to bind enough IFN-αmRNA from the poly(A) RNA to allow detection of IFN-α after injection into oocytes of the mRNA recovered from the recombinant DNA molecule-poly(A) RNA hybrid.

In order to detect IFN-α by the assays available, its concentration should be 100 IU/ml or higher. Because 0.5 ml aliquots are required for replicate determinations, 50 IU should be generated in the oocytes. The poly(A) RNA from induced leukocytes, used previously, generates about 500 IU IFNr-α upon injection of 1 µg into oocytes. Therefore, at least 0.1 µg poly(A) RNA has to be injected to generate the needed 50 IU. Model experiments with rabbit globin mRNA and rabbit β-globin cDNA clones showed that the overall recovery of $^{125}$I-globin mRNA in the oocyte relative to $^{125}$I-globin mRNA added to the hybridization mix was about 10%, and the recovery of mRNA activity about 5%. Therefore, at least 0.1/0.05 = 2 µg of leukocyte poly(A) RNA should be used for the hybridization assay. To ensure an adequate safety margin, 12 µg of poly(A) RNA were used per assay.

To calculate how much DNA from the recombinant DNA molecules is required to bind the IFN-αmRNA in 12 µg of poly(A) RNA, the IFN-αmRNA content of poly(A) RNA was estimated. One µg of poly(A) RNA generates 500 IU of IF. The specific activity of IFN-α lies between $2 \times 10^8$ and $10^9$ IU/mg protein. 500 IU of IFN-α therefore correspond to between $500/2 \times 10^8 = 2.5 \times 10^{-6}$ mg (2.5 ng) and $500/10^9 = 5 \times 10^{-7}$ mg (0.5 ng) of interferon.

The relationship between the amount of IFN-αmRNA injected into an oocyte and the amount of IFN-α produced is unknown. In the case of β-globin mRNA, about 30 molecules of protein per molecule mRNA are produced per hour; this valve is about 6 for β-globin (J.B. Gurdon *et al.*, "Message Stability In Injected Frog Oocytes; Long Life Of Mammalian And β-Globin Messages", *J. Mol. Biol., 80*, pp. 539-51 (1973)). Assuming an average value of 20 for IFN-α, a molecular weight of 18000 for IFN-α and a molecular weight of 330,000 for IFN-αmRNA, then 26 mg ($18000/330000 \times 20 \times 24$) of IFN-α should be produced in 24 h per mg of IFN-αmRNA injected. If the specific activity of IFN-α is $2 \times 10^8$/mg ($2 \times 10^2$ IU/ng), then 1 ng IFN-αmRNA will yield $26 \times 2 \times 10^2 = 5.2 \times 10^3$ IU of IFN-α. If the specific activity is $10^9$/mg ($10^3$IU/ng), the amount of IFN-α produced would be $2.6 \times 10^4$IU. Because 1 µg of leukocyte poly(A) RNA yields 500 IU of IFN-α, under the above assumed conditions, the concentration of IFN-αmRNA in 1 µg poly(A) RNA would fall between 0.1 ng to 0.02 ng and the proportion of IFN-αmRNA in leukocyte poly(A) RNA would lie between 1:10,000 and 1:50,000. Therefore, 12 µg of poly(A) RNA contains about 1.2 ng to 0.2 ng IFN-αmRNA.

Should the translation ratio of the IFN-αmRNA in the oocytes be lower by an order of magnitude than the average for globin mRNA, the IFN-αmRNA content of the poly(A) RNA would be 10 times higher than calculated above, or between about 5 1:1000 to 1:5000. And, 12 µg of poly(A) RNA would then contain about 12 ng to 2 ng of IFN-αmRNA. On the other hand, should the translation ratio of the IFN-αmRNA in the oocytes be higher by an order of magnitude than the average for globin mRNA, the IFN-αmRNA content of the poly(A) RNA would be 10 times lower than calculated above, or between about 1:100,000 and 1:500,000. And, 12 µg of poly(A) RNA would then contain 0.1 ng to 0.02 ng IFN-αmRNA.

Plasmid pBR322 has 4361 b.p. The complete cDNA of IFN-αmRNA would add about 800-1000 b.p. to pBR322 on formation of pBR322-IFN-αcDNA to a total of about 5200-5400 b.p. Its molecular weight would thus be about 12 times ($2 \times 5200/800$) that of the IFN-αmRNA alone. Therefore, in order to bind the IFN-αmRNA calculated above to be present in 12 µg poly(A) RNA required for the assay, an amount of recombinant DNA molecules equal to 12 times the amount of the IFN-αmRNA will be required (stoichiometric amount).

Because the IFN-αmRNA content of the poly(A) RNA used to prepare the recombinant DNA molecules had been increased 10 to 40-fold over that of the crude poly(A) RNA, the group of 512 clones should have 10 to 40 times more clones containing the desired IFN-αmRNA than calculated from the above.

If IFN-αmRNA is 1 part of 1000 of the crude poly(A) RNA, then 12 µg of poly(A) RNA contain 12 ng IFN-αmRNA and the stoichiometric amount of IFN-αcDNA plasmid is 144 ng. Since a group of 512 clones will contain at least 5 with IFN-αcDNA inserts, the amount of total hybrid plasmid DNA required is 14.8 µg ($144 \times 512/5 \times 10^{-3}$). If IFN-αmRNA is 1 part in 10,000, then 12 µg of poly(A) RNA contain 1.2 ng IFN-αmRNA and the amount of IFN-αcDNA plasmid required is 14.4 ng. A group of 512 clones will contain either O or 1 IFN-αcDNA insert, so that the amount of total hydrid plasmid DNA required is 7.4 µg ($14.4 \times 512 \times 10^{-3}$). If IFN-αmRNA is 1 part in 100,000, then the amount of total hybrid plasmid DNA required is 0.74 µg ($1.44 \times 512 \times 10^{-3}$). In order to ensure that the hybridization reaction will proceed under DNA excess conditions (i.e., excess recombinant DNA as compared to poly(A) RNA), 20 µg of the mixture (about 1.4 to 30-fold excess) was chosen for the assay.

Hybridization must be conducted under conditions which ensure (a) that the hybridized portion of the poly(A) RNA is recovered intact and in a biologically active form, (b) that non-specific DNA-mRNA association is prevented, and (c) that the hybridization reaction goes to at least 75% completion. These conditions are most likely to be met by hybridization in 80% formamide, 0.4 M NaCl (J. Casey and N. Davidson, "Rates Of Formation And Thermal Stability Of RNA:DNA And DNA:DNA Duplexes At High Concentrations Of Formamide", *Nucleic Acids Res.*, 4, pp. 1539-52 (1977)). In this solution, hybridization can be conducted at about 40°C (rather than the 60°-70°C required when formamide is omitted). Lower temperatures are preferred to minimize damage to the poly(A) RNA. We chose a hybridization temperature of 56°C. This is about 3° below the $T_{1/2\,i}$ (J. Casey and N. Davidson, *supra)* and about 10-13° below $T_{1/2\,d}$ (Hamaguchi & Geidushek. *J. Amer. Chem. Soc.*, 84, p. 1329). Therefore, this temperature should not allow hybridization of sequences with less than about 87% homology, since a 1% mismatch lowers the $T_{1/2\,d}$ by 1' (T. F. Bonner *et al.*, "Reduction In The Rate Of DNA Reassociation By Sequence Divergence", *J. Mol. Biol.*, 81, pp. 123-35 (1973)).

In the present hybridization, self-hybridization of DNA is not a major problem because the mixture of DNA's being used consists of the same vector (pBR322) and a variety of cDNA inserts. Therefore, most of the DNA sequences will be heteroduplexes in which the inserts are available for hybridization to poly(A) RNA. It is very unlikely that complementary cDNA inserts which form part of different duplexes will interact because of topological constraints. In any event, DNA:DNA reassociation is minimized under the reaction conditions used (J. Casey and N. Davidson, *supra).*

To determine the hybridization time required to ensure at least 75% reaction, a second order rate equation was employed:

$$t = \frac{1n\ \dfrac{Co - Ro + Ro\ (1 - \frac{R}{Ro})}{(1 - \frac{R}{Ro})\ Co}}{k_R\ (Co - Ro)} = 3.9\ h$$

where:

R = molar nucleotide concentration of hybridized RNA

Co = molar nucleotide concentration of initial DNA to hybridized

Ro = molar nucleotide concentration of initial RNA to be hybridized

$k_R$ = rate constant for RNA-DNA hybridization

t = time (sec)

and:

$\dfrac{R}{Ro}$ =0.75 (75% reaction completion)

$k_R$ = 472 ($k_R$ = 1/12 $k_d$ (J. Casey and N. Davidson, *supra)*

where:

$k_d$ = second order rate constant for DNA under the chosen conditions of hybridization and: $k_d$= $1.7 \times 10^5$ $\times\ L_{\frac{1}{2}} \times N^{-1}$ (J. R. Hutton and J. G. Wetmur, "Renaturation Of Bacteriophage $\partial$ X174 DNA-RNA Hybrid: RNA Length Effect And Nucleation Rate Constant", *J. Mol. Biol.,* 77, pp. 495-500 (1973))

L = 900 (chain length in b.p.; about 900 are present in the full IFN-$\alpha$cDNA insert)

N = 900 (complexity in b.p. of the hybrid chain; here the complexity is 900 because the 900 nucleotides of the IFN-$\alpha$mRNA join with the complementary 900 nucleotides of the IFN-$\alpha$cDNA insert)

Co = $2.5 \times 10^{-7}$ (Based on a 40 $\mu$l solution containing the previously determined 20 $\mu$g of recombinant DNA molecules to be used in the assay, again assuming that the IFN-$\alpha$cDNA insert will be 1/12 of a recombinant DNA molecule and will occur in at least 1 of the 512 clones, and assigning 662 as the average molecular weight of one DNA base pair)

Ro = $8.7 \times 10^{-8}$ (Based on a 40 $\mu$l solution containing the previously determined 12 $\mu$g of poly(A) RNA to be used in the assay, again assuming that the poly(A) RNA contains 1:10,000 parts IFN-$\alpha$mRNA (given the large excess of DNA a different proportion will have little effect on the rate of hybridization) and assigning 343 as the average molecular weight of one ribonucleotide of RNA).

3. Execution Of The Initial Assay

*Step A*-Preparation and Cleavage of the Recombinant DNA Molecule Mixture

The desired number of bacterial clones was inoculated onto tryptone medium agar plates supplemented as above, by transferring to it an aliquot from each microtiter well with use of a mechanical device. After incubation at 37°C, each clone had given rise to a colony of several mm diameter. All colonies were washed off the plate(s) and pooled to give an inoculum used to inoculate 1 l of tryptone medium supplemented as above in a 2 l Erlenmyer flask. The culture was shaken at 37°C to an apparent $OD_{650}$ of about 0.8 (estimated visually). One volume of supplemented tryptone medium and chloramphenicol to 170 $\mu$g/ml were added to the culture which was further shaken at 37°C for 16 h. 20 ml chloroform were added and the culture shaken again for 10 min at 37°C to kill the bacteria (C. Weissmann and W. Boll, *supra).* The culture was decanted from the chloroform and the cells were harvested by centrifugation (Sorvall GS3 rotor) for 15 min at 6000 rpm and 4°C. About 1-2 g cells were obtained for each 1-liter preparation. The cells were suspended in 30 ml 20 mM Tris-HCl (pH 7.5). centrifuged for 20 min at 5000 rpm and 4°C (Sorvall SW rotor) and resuspended in 30 ml 50 mM Tris-HCl (pH 7.5). 0.25 vol of lysozyme solution (10 mg/ml in 50 mM Tris-HCl (pH 7.5)) were added and after cooling for 10 min at 0°C 0.33 vol (based on the vol of the original 50 mM Tris-HCl-culture suspension) 0.5 M EDTA (pH 8.0) were gently mixed in without shaking. After another 10 min at 0°C, 1/16 vol (again based on the original volume) of 2% Triton X-100 were added. After 60 min, the sample was centrifuged for 60 min at 10,000 rpm and 0°C in a Sorvall SW rotor. The supernatant was transferred to a beaker containing a magnetic stirrer, and 3 M NaOH was added with stirring until a pH of 12.5 was reached, as measured at 20°C, using a glass electrode and an Orion Research model 601 pH meter, standardized with Beckman pH 10 Carbonate Buffer Standard (No. 3505). After stirring 10 min at 20°C, the pH was adjusted to 8.5. After 3 min further stirring 1/9 vol 5 M NaCl and 1 vol phenol (distilled and equilibrated with 9.5 M NaCl) were added and vigorous stirring continued for 5 min. The phases were separated by centrifugaton (GSA Sorvall rotor) at 10,000 rpm and 0°C for 10 min. The supernatant containing Form I DNA (circular double-stranded DNA) was carefully removed from the interphase (which contains single-stranded DNA) and extracted 3 times with chloroform. (Phenol must be largely removed at this step). The Form I DNA fraction will contain those recombinant DNA molecules (pBR322-cDNA insert) originally used in transforming those host cells which form part of the 512 clones chosen for assay.

Pancreatic RNAase A (5 mg/ml, preheated 10 min at 85°C) was added to the Form I DNA to a concentration of 20μg/ml and the mixture incubated 60 min at 37°C. 1/5 vol 5 M NaCl were added and the mixture adjusted with 30% polyethylene glycol 6000 (Union Carbide, autoclaved 20 min at 120°C) up to a final concentration of 7.5% PEG. After 2-16 h at -10°C, the precipitate was collected in a Sorval SW Rotor for 20 min at 8,000 rpm and 0°C, dissolved in 0.075 M NaCl, 0.0075 M Na-citrate to an absorbance of 20 at 260 nm, and adjusted to 0.5% SDS. The solution was incubated for 30 min at 37°C with 0.5 mg/ml Pronase (self-digested at 20 mg/ml, 2 h at 37°C) and extracted 3 times with 1 vol distilled phenol and 2 times with 1 vol chloroform. The sample (up to 2 ml of a 1 mg/ml DNA solution) was centrifuged through a 5 to 23% sucrose gradient in 50 mM Tris-HCl (pH 7.5),1 mM EDTA for 15 h at 21,000 rpm and 15°C using an SW 27 Beckman Rotor. Fractions were collected and the $OD_{260}$ monitored. DNA-containing fractions were pooled and the DNA precipitated with sodium acetate and ethanol. 20 to 100μg of the Form I DNA mixture were recovered by centrifugation.

Twenty μg of purified Form I DNA were digested in 150 μl 10 mM Tris-HCl (pH 7.5), 6 mM $MgCl_2$, 50 mM NaCl, 6 mM 2-mercaptoethanol, 200 μg/ml BSA or gelatin and 20 units HindIII (New England Biolabs). The HindIII restriction enzyme cleaves the Form I DNA at a site within the pBR322 moiety (It is unlikely that the cDNA moiety is also cleaved, but if it is, the assay should not be substantially affected). After 2 h at 37°C, an aliquot (1%) was analyzed by electrophoresis through a 1% agarose gel in 50 mM Tris-acetate (pH 7.8), 2 mM EDTA for 1 h at 50 mA to ascertain whether digestion was complete. If digestion was not complete, more HindIII was added and incubation continued for 2 h. When the Form I DNA was converted totally to linear molecules. Pronase (Calbiochem), EDTA and SDS were added to 0.5 mg/ml, 10 mM and 0.5% respectively. After 30 min at 37°C, the solution was extracted with 30 μl phenol-chloroform (1:1). The organic phase was washed with 50 μl 20 mM Tris-HCl (pH 7.5), 1 mM EDTA, and the combined aqueous phases extracted 3 times with ether, filtered through a 0.1 ml Chelex column, collected in an EDTA-boiled Pyrex® tube and precipitated with 1/10 vol 3 M sodium acetate and 2.5 vol ethanol. After standing overnight at -20°C, the DNA was collected by centrifugation.

*Step B*-Hybridization of the DNA with Poly(A) RNA

Two hybridization mixtures were prepared. Mixture I contained 4 μl of 10-fold concentrated hybridization buffer (4 M NaCl, 0.1 PIPES (pH 6.4, 1,4 piperazinediethane sulfonic acid, Sigma), 50 mM EDTA, 0.5 μl (about 5 ng $^{125}$I-globin mRNA (5000 cpm) and 6 μl induced leukocyte poly(A) RNA (2 μg/μl), an amount sufficient to generate 6000 IU of IFN when injected into oocytes. Mixture II contained 10 μg of the HindIII digested Form I DNA from above and 0.1 μg of PstI-digested Z-pBR322(H3)/RcβG-4.13 (a pBR322 derivative that contains the β-globin sequence in the HindIII site) (Mantei et al., "Rabbit β-globin mRNA Production In Mouse L Cells Transformed With Cloned Rabbit β-globin Chromosomal DNA", *Nature*, 281, pp. 40-46 (1979)). The $^{125}$I-globin mRNA in mixture I and the β-globin DNA in mixture II serve as internal positive controls for the hybridization assay. Both mixtures were dried in a stream of nitrogen gas. 40 μl of 80% formamide were added to the residue of mixture II and the solution was denatured for 10 min at 100°C and chilled quickly in ice. The denatured solution was used to dissolve the residue of mixture I and the resulting solution incubated at 56°C for 4 h.

*Step C*-Separation Of Hybridized Poly(A) RNA-DNA From Non-Hybridized Poly(A) RNA

After dilution to 1 ml with cold 0.9 M NaCl, 0.09 M Na-citrate and formamide (100%) to 4% (by volume) the solution was filtered at 0.5 ml/min through a Millipore filter (0.45 μm pore size), the filter having been first tested for its capacity to retain RNA-DNA hybrids, because not all filters obtained from the manufacturer were equally efficient.

*Step D*-Purification Of Hybridized Poly(A) RNA

The above filter, with poly(A) RNA hybrids attached, was immersed in 1 ml 0.15 M NaCl, 0.015 M Na-citrate, 0.5% SDS for 10 min at 37°C, rinsed with 50 mM Tris-HCl (pH 7.5), 10 mM $MgCl_2$, 2 mM $CaCl_2$ and placed in 0.6 ml of fresh buffer. After the addition of 5 μl iodoacetate-treated DNAase (5 mg/ml) (S. B. Zimmermann and G. Sandeen, *Anal. Biochem.*, 14, p. 269 (1966); P. A. Price et al., "Alkylation Of A Histidine Residue At The Active Site Of Bovine Pancreatic Deoxyribonuclease", *J. Biol. Chem.*, 244, pp. 924-32 (1969)), the filter was incubated at 37°C for 10 min.

The filter was removed and the solution extracted with 1 vol phenol and 1 vol ether and passed through a 0.1-ml Chelex column. 5 μg of carrier RNA (purified yeast RNA) were added to the solution and the RNA precipitated with sodium acetate and ethanol. The precipitate was collected by centrifugation at 10,000 xg, dissolved in 100 μl 1 mM EDTA, heated for 90 sec at 100°C, and TNE and SDS added to 2 × TNE and 0.5% SDS. The RNA was adsorbed to a 100 μl oligo(dT) cellulose column, eluted with four washes of 0.3 ml distilled water and precipitated with sodium acetate and ethanol. After 16 h at -20°C the precipitated RNA was separated by centrifugation and dissolved in 2 μl TNK buffer.

*Step E*-Determination Of IFN-αmRNA Activity

The poly(A) RNA solution from above was injected into 40 oocytes (about 50 nl per oocyte). The oocytes were incubated at 23°C for 24-48 hours, homogenized and centrifuged (or the incubation medium recovered and assayed as described previously for IFN-α.

### 4. Subsequent Assay - Hybridization To Filter-Bound DNA

Most subsequent assays of a recombinant DNA molecule from a single clone were carried out with DBM or DPT paper-bound DNA, because the assay conditions were no longer critical and the assay is more convenient. DPT paper gave lower backgrounds and was used preferentially. DBM paper was prepared as described (J. C. Alwine *et al.*, "Method For Detection Of Specific RNAs In Agarose Gels By Transfer To Diazobenzyl Oxymethyl-Paper And Hybridization With DNA Probes", *Proc. Natl. Acad. Sci. USA,* 14, pp. 5350-54 (1977)). APT paper was prepared by a procedure of B Seed (pers. commun.): Sheets of Whatman 540 paper (20 g) were agitated for 16 h at 20°C with a mixture of 70 ml 0.5 M NaOH, 2 mg/ml $NaBH_4$ and 30 ml 1,4-butanediol diglycidyl ether. The paper was then transferred to a solution of 10 ml 2-aminothiophenol in 40 ml acetone and agitated for 10h. The paper was exhaustively washed with acetone, 0.1 N HCl, $H_2O$, 0.1 N HCl, $H_2O$ and dried. APT paper was diazotized to DPT paper as described for the conversion of ABM to DBM paper (Alwine *et al., supra).*

DNA (up to 15 μg) was bound to 50 $mm^2$ diazotized ABM (DBM) or diazotized APT (DPT) paper as described by J. H. J. Hoeijmakers *et al.* "The Isolation Of Plasmids Containing DNA Complementary To Messenger RNA For Variant Surface Glycoproteins Of Trypanosoma Brucei", *Gene,* in press, 1980) and set forth below.

Hybrid plasmid DNA was digested with endonuclease *Pstl*, treated with 500 μg Pronase per ml, 0.5% SDS, and 10 mM EDTA for 30 min at 37°C, extracted with phenol and ether, passed through a 0.1 ml Chelex column, and precipitated with ethanol. The heat-denatured DNA (up to 5 μg, with a small amount $^{32}$P-DNA added as tracer) was incubated overnight at 0°C with 1 $cm^2$ DBM or DPT paper in 200 μl 25 mM potassium phosphate buffer (pH 6.5). Filters were washed three times for 5 min at room temperature with 50 mM potassium phosphate buffer (pH 6.5), 1 % glycine and three times with 99% recrystallized formamide. A further incubation with 99% formamide for 2 min at 68°C was followed by three washes in 50 mM potassium phosphate buffer (pH 6.5) at 20°C and two washes in 0.4 M NaOH at 37°C for 10 min. About 40-60% of the radioactivity was retained on the filters. The filters were incubated for 3 h at 38°C in pre-hybridization medium A, supplemented with 1% glycine, using 330 μl per filter. Medium A contains 50% formamide, 5 × SSC, 0.04% polyvinyl pyrrolidone, 0.04% Ficoll (Pharmacia), 0.1% SDS, 25 μg poly(A) (P & L) and 100 μg yeast RNA (BDH, extracted six times with phenol and precipitated with ethanol). The filters were washed twice in medium A and then hybridized for 16 h at 38°C with poly(A) RNA as indicated (usually 5-8 μg) in medium A under paraffin oil. The RNA was added as follows: one wet DNA filter was blotted and put in a sterile Petri dish, 20-40 μl of the RNA solution were pipetted on this filter and a second DNA filter (either a duplicate or a control) was put on top and the sandwich was covered with a sterile paraffin oil. After the hybridization the filters were successively washed in medium A (2 times), in a solution containing 1 × SSC, 0.2% SDS, 1 mM EDTA (3 times, 10 min at 20°C each), medium A (2 h at 38°C and in 50% formamide, 5 × SSC, 0.1% SDS (3 times, 10 min at 20°C). Hybridized RNA was eluted by heating for 1 min at 100°C in 200 μl 10 mM Tris-HCl (pH 7.4),1 mM EDTA and 0.1 % SDS. The elution step was repeated twice, the eluates were combined and the RNA was precipitated with ethanol after addition of 2 μg yeast RNA (purified as above). The washed pellet was vacuum dried, dissolved in 3 μl $H_2O$ and injected into oocytes. IFN-α activity was assayed as above.

### 5. Results Of The RNA Selection Hybridization Assay

The assays from 8 groups of 512 clones *(i.e.,* groups T, Y, j, K, ·, O, ε and π were negative. The assays from 4 groups of 512 clones *(i.e.,* groups I, δ, N and λ) were positive, albeit not consistently. The positive assays are reported in the following format: IU/ml of IFN-α produced by the RNA released from poly(A) RNA-DNA hybrid (assay from control hybridization using Z-pBR322(H3)/Rcβ G-4.13, *supra);* the assays in which the experimental results were higher than the background control are underscored.

| Group | IU/ml |
|---|---|
| I | <60 (<60); *110* (<20); <110 (<110); <110 (<110); <35 (<35) |
| δ | *20* (<20) |
| N | *35* (<20); <110(<110); *200* (<110) |
| λ | <60 (<60); *60* (<20); <110 (<110); <110 (<110) |

Group λ was subdivided into 8 subgroups of 64 clones and hybridized and assayed as before. The subgroups gave the following results, presented in the same format as above:

| Subgroup | IU/ml |
|---|---|
| λ-I | <35 (<35); <35 (<35) |
| λ-II | *130* (<30); <45 (<45) |
| λ-III | *225* (<35); *35* (<30); *35* (<30); *600* (<30); <20 (<20) |
| λ-IV | *85* (<35); <25 (<25) |
| λ-V | <35 (<35) |
| λ-VI | <35 (<35) |
| λ-VII | <35 (<35) |
| λ-VIII | <35 (<35) |

Subgroup λ-III was subdivided into 8 sets of 8 clones, and hybridized and assayed:

| Set | IU/ml |
|---|---|
| λ-III-1 | <20 (<20); <20 (60); *35* (<30) |
| λ-III-2 | <35 (<35); <30 (<30); *150* (<20); *600* (<35); *110* (60) |
| λ-III-3 | <25 (<25); <30 (<30) |
| λ-III-4 | *30* (<30); <20 (<20); <20 (60) |
| λ-III-5 | 30 (?); (<35); <20 (<20); <35 (60) |
| λ-III-6 | <30 (<30); <20 (<20) |
| λ-III-7 | <30 (<20) |
| λ-III-8 | <30 (<20) |

Because the first positive result was achieved with the set λ-III-4, the individual colonies of this set (designated A to H) were hybridized and assayed;

λ-III-4-B <35* (<35); <20 (60);

λ-III-4-C 35(60);*60** (<35);*111** (<11);11** (<11);*20* (<20);

Therefore, clone λ-III-4-C contains a recombinant DNA molecule capable of hybridizing IFN-αmRNA.

The recombinant DNA molecule in this clone is designated: Z-pBR322(Pst)/HclF-4C ("Hif-4C"), and the bacterial strain containing it: *E. coli* X1776 (Z-pBR322(Pst)/HclF-4C) *("E. coli* Hif-4C"). This nomenclature indicates that the recombinant DNA molecule originated in Zurich (Z) and is plasmid pBR322 containing at the *Pst*I site a HIFN-αcDNA ("HclF"); the particular recombinant DNA molecule being derived from clone λ-III-4-C ("4C").

## RECLONING AND CHARACTERIZATION OF Z-pBR322(Pst)/HclF-4C

Since primary clones of transformed cells occasionally contain more than one species of recombinant DNA molecule (Efstratiadis *et al.,* "The Primary Structure Of Rabbit β-globin mRNA As Determined From Cloned DNA", *Cell,* 10, pp. 571-85 (1977)), Hif-4C was isolated from *E. coli* X1776 (Hif-4C) clones and purified as described above. Samples of Hif-4C and pBR322 were digested with *Pst*I and analyzed by electrophoresis on a

* The DBM paper method was used in this assay.

1% agarose gel. Hif-4C gave two bands, one with the mobility of *Pst*-cleaved pBR322, the other with a mobility corresponding to about 320 b.p.

*E. coli* HB101 was transformed with the isolated Hif-4C as described above. Six clones of tetra-cycliner-esistant, transformed bacteria were picked, small cultures prepared and Form I DNA purified and analyzed by *Pst*I cleavage and agarose gel electrophoresis as before. All samples showed cleavage patterns identical to Hif-4C. One of those recloned recombinant DNA molecules was designated Z-pBR322(Pst)/HcIF-4c ("Hif-4c") and used for further experimentation. The lower case "c" designates a recloned DNA molecule.

To determine the capacity of Hif-4c and its cDNA insert to hybridize to IFN-$\alpha$mRNA, Hif-4c (115 $\mu$g) was digested to completion with 125 units of *Pst*I, extracted with phenol and chloroform, and precipitated with ethanol as described above. An aliquot (10 $\mu$g) was 5' terminally labeled (to serve as a tracer in subsequent steps) by dissolving it in 100 $\mu$l 50 mM Tris-HCl (pH 7.5), passing it through a 0.1 ml Chelex 100 column and treating it with 0.6 units bacterial alkaline phosphatase for 1 h at 65°C. Tenfold concentrated TNE (40 $\mu$l) was added and the solution extracted 3 times with 1 vol phenol and 3 times with 1 vol chloroform. The DNA was precipitated with 2 vol ethanol at -20°C overnight and collected by centrifugation. For further purification, the sample in 0.5 ml TNA was adsorbed to 0.25 ml DEAE cellulose (Whatman DE 52, prewashed with 2 ml 150 mM NaCl, 50 mM Tris-HCl (pH 7.5). 2 mM EDTA) ("NET-buffer"). washed with 2 ml of NET buffer, eluted with 0.4 ml 1.5 M NaCl, 20 mM Tris-HCl (pH 7.5), 2 mM EDTA and precipitated with ethanol as above. The DNA was incubated with $\gamma$-$^{32}$P-ATP (specific activity about 5000 Ci/mmole) and polynucleotide kinase, (A. M. Maxam and W. Gilbert, "A New Method For Sequencing DNA", *Proc. Natl. Acad. Sci. USA,* 74, pp. 560-564 (1977)) and purified by chromatography on a 3 ml Sephadex-G50 column in TNE. The eluted fractions were pooled and the $^{32}$P-DNA precipitated with ethanol as above; yield, about $10^7$ dpm.

The unlabeled *Pst*I-cleaved Hif-4c DNA (90 $\mu$g) was mixed with $6 \times 10^5$ dpm of $^{32}$P-labeled *Pst*I cleaved Hif-4c DNA from above and electrophoresed through a $10 \times 20 \times 0.7$ cm, 2% horizontal agarose gel in 50 mM Tris-acetate buffer (pH 7.8) using a 2.5 cm slot. An x-ray film was exposed to the gel and the position of the 320 bp fragment determined. The gel strip containing the radioactive band ($1.3 \times 10^5$ dpm) was cut out, crushed by pressing through a plastic 2 ml syringe and extracted overnight at 4°C by agitation with ten times the gel vol of NET buffer. The DNA was adsorbed to a 0.1-ml hydroxyapatite column (prewashed with 1 ml NET buffer). The column was washed with 1 ml 0.1 M K-phosphate buffer (pH 7.5) and the DNA eluted with 0.2 ml 1 M K-phosphate buffer (pH 7.5). The eluate was diluted 10-fold with sterile distilled $H_2O$ and the DNA adsorbed to and eluted from DEAE and precipitated with ethanol as described above. This DNA is called "Hif-4c fragment".

The Hif-4c fragment (120 ng) was bound to DPT paper ($0.5 \times 0.5$ cm) as described above. As a control, 120 ng $\beta$-globin cDNA fragment excised with *Hind*III from the hybrid plasmid Z-pBR322(H3)Rc$\beta$G-4.13 (F. Mey-er *et al.,* "Transposition Of AT-linked, Cloned DNA From One Vector To Another", *Experimentia,* 35, p. 972 (1979); N. Mantei *et al., supra)* and processed similarly. Hybridization of duplicate filters to poly(A) RNA (in 20 $\mu$l), washing of the filters and recovery of the RNA from the filters were as described above. After injection into oocytes the following IFN-$\alpha$ activities were detected:

| DNA fragment | amount of leukocyte poly (A) RNA* ($\mu$g) | time of hybridization | IFN-$\alpha$ activity (IU/ml)** (duplicate assay) |
|---|---|---|---|
| Hif-4c | 2.5 | 16 h | 250;100 |
| $\beta$-globin cDNA | 2.5 | 16 h | 4;1 |
| Hif-4c | 7.5 | 16 h | 3000;1000 |
| $\beta$-globin cDNA | 7.5 | 16 h | 4;30 |
| Hif-4c | 7.5 | 5 h | 1000;1000 |
| $\beta$-globin cDNA | 7.5 | 5 h | 10;1 |

\* 1 $\mu$g of this RNA gave 4600 IU/ml.
\*\* Oocyte supernatant after 48 h incubation , assayed by cytopathic effect reduction (W. E. Stewart, II and S. E. Sulkin, *supra*).

Thus, Hif-4c contains an insert capable of hybridizing to IFN-$\alpha$mRNA.

IDENTIFICATION OF CLONES OF E. COLI CONTAINING RECOMBINANT DNA MOLECULES CROSS-HYBRIDIZING TO THE INSERT IN Hif-4c

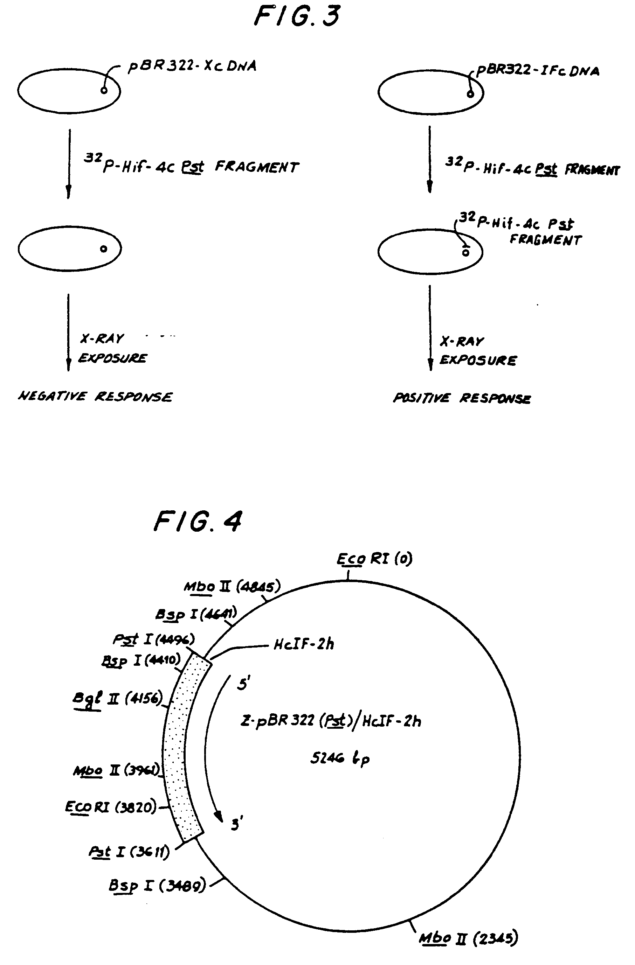

Since the cDNA insert in recombinant DNA molecule Hif-4c was only about 320 b.p., or a third of the estimated size of IFN-αmRNA, the purified Hif-4c fragment described above was used as a probe to screen for bacterial clones containing recombinant DNA molecules having related hybrid DNA inserts (Figure 3).

The 64 bacterial clones constituting subgroup λ-III described above were stamped onto a Millipore membrane (8 cm diameter), placed on an agar plate (supplemented with diaminopimelic acid, nalidixic acid and tetracycline, as above) and incubated for 24 h at 37°C. The filter was placed onto a 0.75 ml drop of 0.5 M NaOH and after 2-3 min transferred onto a paper towel to remove excess liquid; the step was repeated. The filter was neutralized, using 1 M Tris-HCl (pH 7.5), and washed with 1.5 M NaCl - 0.5 M Tris-HCl (pH 7,4) in a similar fashion as above and air dried. The filter was dipped in 0.3 M NaCl, air dried and heated at 80°C for 2 h in a vacuum.

Hif-4c Pst Fragment (30 ng) was $^{32}$P-labeled by nick translation (A. J. Jeffreys and R. A. Flavell, "The Rabbit β-Globin Gene Contains A Large Insert In the Coding Sequence", *Cell*, 12, pp. 1097-1108 (1977)) using β-$^{32}$P dATP and α-$^{32}$P dCTP (specific activity, 40 Ci/mmole each). The filter bearing the λ-III colonies was prehybridized in 4 × SET (SET is 0.15 M NaCl, 30 mM Tris-HCl (pH 8.0), 1 mM EDTA), 0.1% (w/v) Ficoll, 0.1% polyvinylpyrolidine, 0.1% (w/v) BSA, 0.5% SDS, and 200 μg/ml denatured, fragmented salmon sperm DNA for 7 h at 68°C and hybridized with $2 \times 10^5$ cpm of $^{32}$P-labeled Hif-4c fragment in 4 × SET, 0.02% (w/v) Ficoll, 0.02% polyvinylpyrrolidine, 0.02% w/v BSA, 0.5% SDS and 200 μg/ml denatured salmon sperm DNA at 68°C for 16 h. The filter was rinsed with SET-0.5% SDS at room temperature, washed with 2 × SET - 0.5% SDS for 5 h at 68°C, replacing the solution once, and with 3 mM Trizma base at room temperature for 4 h, replacing the solution once. After drying the filter, an x-ray film was exposed to the filter for 80 h using a screen. Three colonies gave a strong positive response, namely λ-III-7D, λ-III-2H and λ-III-4C, and 2 colonies a weak one, namely λ-III-1E, λ-III-3D.

Small cultures were prepared from the Hif-4c related clones. Form I DNA was purified, cleaved with Pst I and analyzed by agarose gel electrophoresis as described above. All Form I DNAs gave rise to a large fragment (plasmid pBR322 moiety) and a small one (hybrid insert). The recombinant DNA molecule for λ-III-2H released the largest insert, namely about 900 b.p. This recombinant DNA molecule was designated Z-pBR322(Pst)/HcIF-2H ("Hif-2H") and its insert "Hif-2H fragment".

Hif-2H was tested for its capacity to bind IFN-αmRNA by binding it to DPT paper (4 μg/100 mm²) and hybridizing it to poly(A) RNA (0.3 μg/μl), all as described above, for 16 h and determining IFN-αmRNA activity:

| DNA sample | IFN-$\alpha$ activity (IU/ml)* |
|---|---|
| Hif-2H | 250 ± 50 (average of 4 determinations) |
| Z-pBR322(H3)/Rc$\beta$G-4.13 | 30 (average of 2 determinations) |
| pBR322 | 20 |

\* Assayed by cytopathic effect reduction.

Hif-2H was recloned as described for Hif-4C and designated Hif-2h.

In a further experiment an additional set of *E. coli* clones containing recombinant DNA molecules was prepared and colonies hybridizing to the labeled Hif-4c fragment were identified. In order to ensure a high yield of plasmids with long cDNA inserts, part of the double-stranded $^{32}$P-labeled leukocyte cDNA prepared enzymatically from leukocyte poly(A) RNA (the same cDNA preparation as described above) was fractionated by size by centrifuging through a sucrose density gradient, using the same procedure described for the centrifugation of the poly(A) RNA. The fractions containing the cDNA with a sedimentation velocity corresponding to a 600 b.p. DNA fragment or greater were pooled and the cDNA recovered after ethanol precipitation. The cDNA was elongated with dCMP residues, hybridized to dGMP-elongated Pst I-cleaved pBR322 and the hybrid DNA used to transform *E. coli* as before, except that *E. coli* HB101 was used. The bacteria were distributed onto 8 cm diameter Millipore filters. placed on Tryptone medium agar plates (containing 10 μg/ml tetracycline) and grown until small colonies appeared. A replica filter was prepared by pressing a fresh, moist Millipore filter onto the colony-bearing filter, peeling it off, placing it face upward on an agar plate containing 4.4% glycerol and incubating it until small colonies appeared. This colony-bearing filter was covered with a further Millipore filter, frozen at -55°C and stored (D. Hanahan and M. Meselson, "A Protocol For High Density Plasmid Screening", Sept. 1978, personal communication). Eighteen filters, bearing a total of about 5000 colonies were prepared. One replica of each filter was used for hybridization to the $^{32}$P-labeled. *PSt* I-excised Hif-4c DNA fragment, exactly as described above. About 185 positive colonies were identified on a autoradiogram, recloned on Millipore filters and identified once more by hybridization. 95 clones giving the strongest hybridization response were designated Z-pBR322(Pst)/HclF-SN1 to SN95 and used for further investigation.

It is, of course, evident, given the ability of Hif-2h to produce a polypeptide displaying an immunological or biological activity of HuIFN *(infra)*, that Hif-2h and other DNA sequences related to it, e.g. Hif-4c, may be employed in this method of clone screening equally well on other clones containing DNA sequences arising from recombinant DNA technology, synthesis, natural sources or a combination thereof or clones containing DNA sequences related to any of the above DNA sequences by mutation, including single or multiple, base substitutions, insertions, inversions, or deletions to select other DNA sequences and clones which also code for HuIFN. Therefore, such DNA sequences and their identification also fall within this invention (e.g., *infra).* It is also to be understood that DNA sequences, which are not screened by the above DNA sequences, yet which as a result of their arrangement of nucleotrides code on expression for the polypeptides coded for by the expression of the above DNA sequences also fall within this invention.

## FURTHER CHARACTERIZATION OF Hif-2h DNA INSERT

As described above recombinant DNA molecule Hif-2h contains an insert of about 900 b.p., and hybridizes to human leukocyte interferon nRNA. The following additional characteristics were determined.

### 1. *Hybrid Arrested Translation*

If mRNA is hybridized to a cloned, complementary cDNA, the translation of the mRNA is inhibited. however heat denaturation of the hybrid releases translatable mRNA (B. M. Paterson *et al.,* "Structural Gene Identification And Mapping By DNA-mRNA Hybrid-Arrested Cell-Free Translation", *proc. Natl. Acad. Sci. USA,* 74, pp. 4370-74 (1977)). 2.2 µg *Pst* I-cleaved Hif-2h, and as a control 2 µg *Hind*III-cleaved Z-pBR322(H3)/Rcβ-4.13 ("RcβG") were denatured in 10 µl 80% (vol/vol) deionized formamide - 20 mM PIPES buffer (pH 6.4) for 10 min at 80°C. The solution was added to an Eppendorf tube into which leukocyte poly(A) RNA (5 µg), NaCl (4 µ moles) and EDTA (10 nmoles) had been dried down. The mixture was heated for 7 h at 48°C under a layer of paraffin oil, cooled and diluted with 200 µl $H_2O$. The two samples were divided into equal parts, and one of each was heated at 100°C for 30 sec. The nucleic acids were precipitated with ethanol, dissolved in 3 µl $H_2O$ and assayed for IFN-αmRNA activity in oocytes as above:

| DNA | Le poly(A) RNA input | Treatment | IFN-$\alpha$ (IU/ml)* |
|---|---|---|---|
| Hif-2h (1.1 µg) | 2.5 µg | hybridized | 400 |
| Hif-2h (1.1 µg) | 2.5 µg | hybridized and denatured | 2000 |
| RcβG (1 g) | 2.5 µg | hybridized | 3000 |
| RcβG (1 g) | 2.5 µg | hybridized and denatured | 3000 |
| Hif-2h (0.5 µg) | 1 µg | none | 2000 |
| — | 1 µg | none | 3000 |
| — | 1 µg | none | 2000 |

* The oocyte medium was assayed after 48 h by the cytopathic effect inhibition method.

Therefore, Hif-2h, when hybridized with poly(A) RNA, inhibited the translation of the IFN-αmRNA in the poly(A) RNA; after denaturing the hybrid, the IFN-αmRNA was again translatable. This experiment confirms that Hif-2h contains sequences complementary to IFN-αmRNA.

2. Analysis By Restriction Enzyme Cleavage And Determination of Nucleotide/Amino Acid Sequences And Restriction Map

Digestions of Hif-2h with various restriction enzymes (New England Biolab) were carried out, and the resulting products analyzed by agarose gel electrophoresis. The underlined fragments are not common to pBR322. and Hif-2h:

| Restriction enzyme | Fragment sizes | |
| --- | --- | --- |
| | Hif-2h* | pBR322** |
| PstI | *885* ± 20,4361 | 4361 |
| EcoRI | *1426*,3820 | *4361* |
| BglII | *5246* | not cleaved |
| EcoRI + BglII | *336,4960* | *4361* |
| EcoRI + PstI | *209,676*,748 3611 | 748,3611 |
| BspI | *921*,587,540,504, | 587,540, 504,457, |
| | 457,434,2 × *231*, | 434,*267*, 234 |
| | +14 fragments | +14 fragments |
| | 200 bp | 200 bp |
| MBoII | *1616, 884* and others | not done |

\* Figures 8—10 display the respective fragment sizes as determined by nucleotide sequence data.
\*\* From Sutcliffe (*supra*).

In addition, 5' terminally $^{32}$P-labeled PstI cleaved Hif-2h was cleaved with several restriction enzymes and the sizes of the radioactive fragments derived from the cDNA insert in that recombinant DNA molecule were determined:

| Restriction enzyme | $^{32}$P-fragments* |
|---|---|
| *Eco*RI | 676,209 |
| *Hind*III | no cleavage |
| *Bsp*I | 799, 86 |
| *Hpa*II | no cleavage |
| *Hha*I | no cleavage |
| *Bam*HI | no cleavage |
| *Hinf* | 210, 62 |
| *Bgl*II | 545,340 |

\* Figures 8—10 display the position of these restriction sites and the respective fragment sizes as determined by analysis of nucleotide sequence data.

From these data a restriction map of Hif-2h was deduced (Figure 4). The position of the restrictions sites in Figure 4 are based on fragment sizing by agarose gel electrophoresis and may also be incomplete in regard to *Mbo*II sites within the insert. Only the sites closest to the insert are given within the pBR322 moiety. The arrow indicates the orientation of the IFN-αcDNA coding strand.

Although the actual structure of the Hif-2h fragment or other inserts in clones of this invention or the amino acid sequence or structure of the polypeptides coded therefrom is not required for one of skill in the art to make and use the invention described and claimed herein, the above data and restriction map were included in the original application hereto as the best available information on the structure of fragment at the time of filing the original application. Since that time, as expected *(supra,* p. 9, lines 27-29), these data and restriction map for the Hif-2h fragment have been refined using well-known techniques of nucleotide sequencing and restriction analysis. *E.g.,* A. M. Maxam and W. Gilbert, "A New Method For Sequencing DNA", *Proc. Natl. Acad. Sci. USA,* 74, pp.560-64 (1977). Plasmid DNA was prepared by Method B (N. M. Wilkie, *et al.,* "Hybrid Plasmids Containing An Active Thymidine Kinase Gene of Herpes Simplex Virus 1", *Nucleic Acids Research,* 7, pp. 859-7 (1979)) and restricted by various restriction enzymes essentially as recommended by the supplier, except that 200 μg/ml gelatin replaced the bovine serum albumin in the enzyme buffers. *(Eco*RI was a gift from W. Boll, *Bsp*I a gift from A. Kiss and other enzymes were obtained from New England Biolabs).

Restricted DNA (20 μg) was extracted with phenol, precipitated with ethanol, dissolved in 0.05 M Tris-HCI (pH 8), and passed over a small column of Chelex-100. Fragments with flush or 5'overhanging ends were dephosphorylated by treatment with 0.2 units calf intestinal alkaline phosphatase (Boehringer) per pmol DNA 5' ends in 200 μl 0.05 M Tris-HCI (pH 8) for 60 min at 37°C. The enzyme was inactivated by heating 60 min at 65°C. For DNA fragments with 3' overhanging ends, bacterial alkaline phosphatase (Worthington) was used as described (A. M. Maxam and W. Gilbert, *supra)* except that incubation was at 65°C for 30 min. The dephosphorylated DNA was purified by adsorption to and elution from DEAE-cellulose (W. Muller *et al.,* "Site-Directed Mutagenesis In DNA: Generation of Point Mutations In Cloned B-Globin Complementary DNA At The Position Corresponding To Amino Acids 121-123", *J. Mol. Biol.,* 124, pp. 343-58 (1978)) or subjected to polyacrylamide gel electrophoresis where required (see below). Fragments recovered from a polyacrylamide (or agarose) gel in 0.15 M NaCl, 0.05 M Tris-HCI (pH 8) were adsorbed to a 0.1-ml hydroxyapatite (Biorad HTP) column, washed 4 times with 1 ml 0.1 M potassium phosphate buffer (pH 7) and eluted with 0.3 ml 1 M potassium phosphate buffer (pH 7). The solution was diluted tenfold and the DNA adsorbed to DEAE cellulose and recovered as described (W. Muller *et al., supra).*

After ethanol precipitation, the DNA was 5'-terminally labeled with [γ-$^{32}$P] ATP (12-34 μCi per pmol DNA 5' ends) and polynucleotide kinase (New England Biolabs or P-L Biochemicals Inc.) essentially as described by A. M. Maxam and W. Gilbert, *supra,* except that the DNA was not denatured before the kinase reaction. Specific activites of 1-1-5 μCi [$^{32}$P] phosphate per pmol DNA 5' ends were obtained.

For sequencing, labeled fragments were cleaved with a second restriction enzyme and the products separated by electrophoresis through a 5% polyacrylamide gel in tris-borate-EDTA buffer. The desired fragments were extracted from the gel and purified (Muller *et al., supra).* The various fragments for sequencing were prepared as follows (the number indicates the nominal fragment chain length in base pairs, the labeled site is indicated by an asterisk):

(1) cleavage of Hif-2h with *Bsp*I, isolation of *Bsp*I-*Bsp*I-*232* and *Bsp*I-*Bsp*I-*949* by 5% polyacrylamide gel electrophoresis in Loening's buffer (U. E. Loening "The Fraction of High Molecular Weight Ribonucleic Acid By Polyacrylamide Gel Electrophoreses", *J. Biochem.* p. 102 (1967));

(2) cleavage of Hif-2h with *Bsp*I, labeling, cleavage with *Pst*I, isolation of *Bsp*I\*-*Pst*I-*83* and *Bsp*I\*- *Pst*I-827;

(3) cleavage of Hif-2h with *Bg*II. labeling, cleavage with *Pst*I, isolation of *Bg*II\*p*Pst*I-336 and *Bg*II\*-*Pst*I-570;

(4) cleavage of Hif-2h with *Mbo*II, labeling, digestion with *Pst*I and *Hind*II (to cleave an interfering 350 bp pBR322 fragment), isolation of *Mbo*II\**Pst*I-519 and *Mbo*-II\*-*Pst*I-351:

(5) cleavage of Hif-2h with *Eco*RI, labeling, cleavage with *Pst*I, isolation of *Eco*RI\*-*Pst*I-708 and *Eco*RI\*-*Pst*I-198;

(6) cleavage of Hif-2h with *Pst*I, labeling; cleavage with *Bg*II, isolation of *Pst*I\*-*Bg*II-570 and *Pst*I\**Bg*II-336;

(7) cleavage of Hif-2h with *Ava*II, labeling, cleavage with *Pst*I and *Bg*II, isolation of *Ava*II\*-*Pst*I-186 and *Ava*II\*-*Bg*II-147;

(8) cleavage of Hif-2H with *Pvu*II, labeling, cleavage with *Pst*I and *Bg*II, isolation of Pvull\* -*Pst*I-486.

The fragments were degraded according to the method of A. M. Maxam and W. Gilbert, *supra,* with the modifications described in protocols provided by the same authors in September 1978. The products were fractionated on 0.1 × 25 × 36 cm 12% polyacrylamide gels (acrylamide/bisacrylamide = 18/1) in 50 mM tris-borate, 1 mM EDTA (pH 8.3), with runs of 2, 8,18 and 26 h at 900 V following a 6 h prerun at 700 V. Best results were obtained when the gels were kept at room temperature 2-3 days before use.

Each stretch of the cDNA insert was sequenced from both strands and each restriction site which served as labeled terminus was sequenced using a fragment spanning it. The nucleotide sequence thus obtained is depicted in Figures 8-10. As is to be expected the positions of the various restriction sites in this insert are more absolutely located than those determined by restriction enzyme cleavage alone and depicted in Fig. 4.

Referring now to Figs. 8-10. the heteropolymeric part of the insert is flanked by 23G residues at the 5' end and by 7A residues (probably reflecting the poly(A) terminus of the mRNA) followed by 15C residues at the 3' terminus. For reference, the insert is numbered from the first nucleotide following the dG tail to the last nucleotide before the polyA residues. An ATG initiation triplet in position 57-59 and a TAA termination triplet at position 624-626 define a reading frame uninterrupted by nonsense codons. Both other reading frames in this region of the insert contain 18 and 12 nonsense codons respectively. Moreover, the only other sequences, *i.e.,* in different reading frames, flanked by an ATG or GTG and a termination signal, which code for a polypeptide of 25 amino acids or more, are located between nucleotides 226 and 304, 640 and 778 and 683 and 743, respectively. Therefore, the region between nucleotides 57 and 626 most likely includes the nucleotide sequence of the Hif-2h fragment that codes for a polypeptide displaying a biological or immunological activity of IFN-$\alpha$ in accordance with this invention.

It should of course be understood that cloned cDNA from polyA RNA by usual procedures (A. Efstratiadis *et al., supra)* lacks 5' terminal nucleotides and may even contain artifactual sequences (R. I. Richards *et al.,* "Molecular Cloning And Sequence Analysis Of Adult Chicken B-Globin cDNA", *Nucleic Acids Research,* 7, pp. 1137-46 (1979)). Therefore, it is not certain that the ATG located at nucleotides 57-59 is in fact the first ATG of authentic mRNA. However, for the purpose of the following description, it is assumed that the ATG at nucleotides 57-59 is the first ATG of authentic mRNA.

By comparing the polypeptide coded by this region of the insert with that sequence of 35 amino terminal amino acids of authentic human lymphoblastoid interferon -SerAspLeuProGlnThrHisSer-LeuGlyAsnArgArgAlaLeuIleLeuLeuAlaGlnMetGlyArgIleSerLeuPheSerCysLeuLysAspArgHisAsp- determined by K. C. Zoon *et al., supra* and M. Hunkapiller and L. Hood, *supra,* it appears that the chosen reading frame is correct and that nucleotides 57-124 may code for a signal sequence which precedes the nucleotide sequence coding for the "mature" polypeptide because alignment of the published sequence with the determined sequence (from the 24th amino acid onward) displays extensive coincidence *(i.e.,* 26 of 35 amino acids).

In eukaryotic mRNAs the first AUG triplet from the 5' terminus is usually the initiation site for protein synthesis (M. Kozak, "How Do Eukaryotic Ribosomes Select Initiation Regions In Messenger RNA", Cell, 15, pp. 1109-25 (1978)). The codon in the Hif-2h fragment corresponding to the first amino acid of lymphoblastoid interferon is 22 codons from the first AUG (and 14 codons from the second one) indicating that the sequence coding for interferon may be preceded by a sequence determining a signal peptide of 23 (or less likely 15) amino acids. The longer of the presumptive signal sequences contains an uninterrupted series of 1 1 hydrophobic amino acids (and the shorter one, one of 6 hydrophobic amino acids). This accumulation of hydrophobic residues is characteristic of signal sequences *(cf.,* B. D. Davis and P. C. Tai, "The Mechanism Of Protein Secretion Across Membranes", *Nature,* 283, pp. 433-38 (1980))

The nucleotide sequence apparently corresponding to "mature" IFN-a polypeptide comprises 498 nucleotides, which code for 166 amino acids. Assuming that there is no carboxyterminal processing, the molecular weight of the interferon polypeptide is 19,388. The base composition of the coding sequence is 50% GC. The codon usage within the interferon coding sequence is in reasonable agreement with that compiled for mammalian mRNAs in general (R. Grantham, *et al.,* "Codon Catalog Usage And The Genome Hypothesis", *Nucleic Acids Research,* 8, pp. 49-62 (1980)). Any deviations observed may be ascribed to the small numbers involved.

The structure of the polypeptide depicted in Figure 8-10 for the Hif-2h fragment, of course, does not take into account any modifications to the polypeptide caused by its interaction with *in vivo* enzymes, *e.g.* glycosolation. Therefore, it must be understood that this structure may not be identical with IFN-a produced *in vivo,* but it still has very similar, if not identical, biological and immunological properties. Neither, does this structure exclude the likelihood that other modifications such as mutations, including single or multiple, base substitutions, deletions, insertions, or inversions or chemical derivatizations of this structure will not produce compounds that also display IFN-$\alpha$ activity.

3. Determination Of The Plus Strand Of The Inserted IFN-$\alpha$cDNA

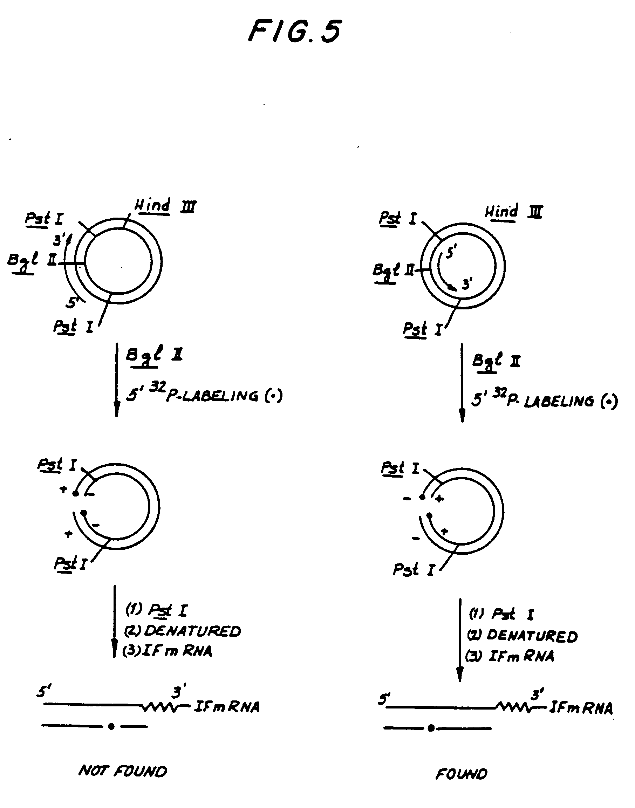

The DNA strand that has the same sequence as the mRNA is designated as plus strand, and its complement as minus strand. The plus strand of the IFN-$\alpha$cDNA insert was identified as outlined in Figure 5. Hif-2h DNA was cleaved with the restriction enzyme *Bg*/II, the termini labeled with $^{32}$P-phosphate (as described above for *Pst*I-cleaved termini) and the DNA digested with *Pst*I, to give longer (545 b.p. (570 b.p. as determined in the more refined analysis reported above)) and shorter 340 bp (336 bp as determined in the more refined analysis reported above)) radioactive fragments. These fragments were denatured and hybridized to poly(A) RNA from induced leukocytes in 80% formamide, 0.4 M NaCl, *i.e.,* under conditions where DNA-DNA reassociation does not occur *(supra).* The nucleic acids were digested with nuclease SI, which degrades all single-stranded nucleic acids, in particular the non-hybridized $^{32}$P-DNA, and the products were separated on a polyacrylamide gel (R. F. Weaver and C. Weissmann, "Mapping Of RNA By A Modification Of The Berk-Sharp Procedure", *Nucleic Acid Research,* 7, pp. 1175-93 (1979)). An autoradiogram showed that only the shorter-nucleotide fragment had been hybridized and protected by the poly(A) RNA, identifying the 5'-labeled shorter-nucleotide strand as the minus strand. The orientation of the plus strand is therefore as given in Figure 4 and Figure 5 (right hand side).

4. Demonstration That Poly(A) RNA From Non-Induced Human Leukocytes Does Not Hybridize To Hif-2h DNA

An experiment identical to that described in the proceding section was carried out, however, the poly(A) RNA was from non-induced human leukocytes, prepared by the same procedure as in the case of Sendai virus-induced leukocytes. No detectable amount of labelled DNA was protected. By comparison to the results of the preceding section the poly(A) RNA from non-induced cells contains less than about 1/20 the amount of mRNA hybridizable to Hif-2h than does poly(A) RNA from induced cells.

SYNTHESIS OF A POLYPEPTIDE WITH INTERFERON ACTIVITY BY E. COLI CONTAINING RECOMBINANT DNA MOLECULES RELATED TO Z-pBR322(Pst)/HcIF-4c

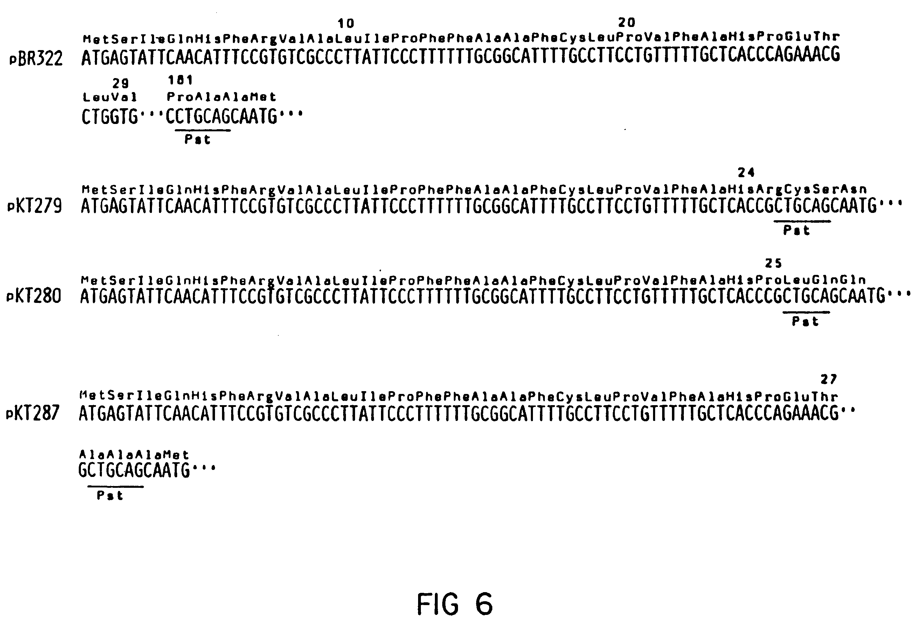

The *Pst*I sites of pB322 lies within the β-lactamase (penicillinase) gene. Therefore, when a coding DNA segment *(e.g.,* a cDNA comprising all or part of a gene) is ligated into the position in the proper orientation and proper reading frame, a fused protein may result. The protein would consist of the amino-terminal portion of β-lactamse followed by the amino acid sequence for which the inserted DNA sequence codes (L. Villa-Komaroff *et al., supra).* If the inserted DNA segment comprises a DNA sequence containing its own initiation signal, and has a sequence preceding it with a termination signal in phase with the β-lactamase sequence, termination and re-initiation may occur at the second initiation signal and a non-fused, active protein may result (A. C. Y. Chang *et al., supra).* To ensure that the DNA insert related to Hif-4c was inserted in the proper reading frame for expression within the β-lactamase gene, a set of derivatives of pBR322, namely pKT279, pKT280 and pKT287 (constructed by K. Talmadge, personal communication, 1979) was employed. Each of these derivatives has a *Pst*I site located such that a DNA insert ligated into that site will be in a different reading frame from an insert at the *Pst*I site of the other derivatives of the set (Figure 6). Therefore, the set permits the insertion of DNA into the b-lactamase gene in all three reading frames. The *Pst*I-excised insert from Hif-2h was prepared as described for the fragment Hif-4c. The Hif-2h *Pst* fragment (10 ng) was mixed with *Pst*I-cleaved

pBR322, pKT279, pKT280 or pKT287 (10 ng in each case) in 20 ml of 10 mM Tris-HCl (pH 7.5), 6 mM MgCl$_2$, 100 mM NaCl, 6 mM b-mercaptoethanol, 200 mg/ml gelatin and 0.1 mM ATP and incubated with 0.1 units T$_4$ DNA ligase (New England Biolabs) for 16 h at 10°C. The resulting recombinant DNA molecules are designated Z-pBR322(Pst)/HclF-2h, Z-pKT2 79(Pst)/HclF-2h, Z-pKT280Z(Pst)/HclF/2h and Z-pKT287(Pst)/HclF-2h. *E. coli* HB101 was transformed with each of these recombinant DNA molecules and transformed colonies were selected on tetracycline-containing agar plates as described previously. Since tetracycline-resistant clones of transformed bacteria may also contain the recyclized vector, bacterial colonies of each set were grown on Millipore filters, and colonies hybridizing to 32P-labeled Hif-4c fragment were identified and selected as described above. These strains were designated as follows,

*E. coli* HB101 (Z-pBR322(Pst)/HclF-2h-AH1) to (-AH3);

*E. coli* HB101 (Z-pKT279(Pst)/HclF-2h-AH1)

to

(-AH8):

*E. coli* HB101 (Z-pKT280(Pst)/HclF-2h-AH1) to (-AH8):

*E. coli* HB101 (Z-pKT287(Pst)/HclF-2h-AH1) to (-AH8).

Extracts of some of the above strains as well as of some of the strains Z-pBR322(Pst)/HclF-SN1 to 95 were tested for IFN-a activity. Bacteria were grown in Tryptone medium to stationary phase, harvested, washed with 1/20 vol (based on the vol of the culture) 50 mM Tris-HCl (pH 8),30 mM NaCl and frozen. After thawing, the cells were resuspended in the volume indicated below of the previous buffer and lysozyme was added to 1 mg/ml. After 60 min at 0°C the suspensions were frozen (in an ethanol-dry ice bath) and thawed (at 37°C) 5 times, and centrifuged 10 min at 12,000 rpm in a GSA Sorvall rotor. In come cases part of the supernatant (S30) was further centrifuged at 100,000xg in a Type 65 Spinco rotor and the supernatants (S100) recovered. Such supematants were screened for IFN-α activity by the cytopathic effect reduction assay (Expt.1). The colonies showing a positive response in Expt. 1 were reassayed as well as 49 clones from the set Z-pBR322/HclF-SN-1 to SN-95 described above at a lower dilution (Expt. 2).

| Source of extract: E. coli HB 101 transformed by: Expt. 1* | Preparation | IFN-$\alpha$ activity (IU/ml) |
|---|---|---|
| Z-pBR322(Pst)/HclF-2h | S30 | ? |
| Z-pBR322(Pst)/HclF-2h-AH-1 to 3 | S30 | ? |
| Z-pKT279(Pst)/HclF-2h-AH-1 to 7 | S30 | ? |
| Z-pKT279(Pst)/HclF-2h-AH-8 | S30 | positive |
| Z-pKT280(Pst)/HclF-2h-AH-2,6,7 | S30 | ? |
| Z-pKT280(Pst)/HclF-2h-AH-1,3,4,5 | S30 | positive |
| Z-pKT287(Pst)/HclF-2h-AH-1,2,3,4,5,8 | S30 | ? |
| Z-pKT287(Pst)/HclF-2h-AH-6,7 | S30 | positive |

Source of extract:
E. coli HB101 transformed by:
Expt. 2**

| | | |
|---|---|---|
| Z-pKT279/HcIF-2h-AH-8 | S30 and S100 | 300 |
| Z-pKT280/HcIF-2h-AH-1,3,4,5 | S30 and S100 | 300 |
| Z-pKT287/HcIF-2h-AH-6 and 7 | S30 and S100 | 300 |
| Z-pBR322(Pst)/HcIF-SN-4, 5, 7, 9, 10<br>11, 13, to 16 | S30 | neg (<10) |
| Z-pBR322(Pst)/HcIF-SN-18 to 22, 24,<br>25, 27, 30 to 34 | S30 | neg (<10) |
| Z-pBR322(Pst)/HcIF-SN-38 to 41,<br>43 to 48 | S30 | neg (<10) |
| Z-pBR322(Pst)/HcIF-SN-1 to 3, 6<br>8, 12, 17<br>23, 26 | S30 | 10 |
| Z-pBR322(Pst)/HcIF-SN-28, 29, 36<br>37, 49 | S30 | 10 |
| Z-pBR322(Pst)/HcIF-SN-35,42 | S30 | 200 |

\* Expt. 1: Extracts assayed at 1:150 final dilution
\** Expt. 2: Extracts assayed at 1:60 final dilution

Some of the more active producers from above were examined in more detail. Cultures were grown to late log phase (apparent $OD_{650}$ about 0.9)* and the cells lysed as above, in 1/50 of the culture volume. The following activities were found, using Z-pBR322(Pst)/HcIF-SN32 as a negative control:

| Source of extract:<br>E. coli HB101 transformed by: | Preparation | IFN-$\alpha$ activity**<br>(IU/ml)<br>(dup. assays) |
|---|---|---|
| Z-pKT279(Pst)/HcIF-2h-AH8 | S30, S100 | 100; 300 |
| Z-pKT280(Pst)/HcIF-2h-AH3 | S30, S100 | 1000; 1000 |
| Z-pKT287(Pst)/HcIF-2h-AH6 | S30, S100 | 200; 200 |
| Z-pBR322(Pst)/HcIF-SN35 | S30, S100 | 1000; 1000 |
| Z-pBR322(Pst)/HcIF-SN42 | S30, S100 | 300; 100 |
| Z-pBR322(Pst)/HcIF-SN32 | S30, S100 | 0; 0 |

\** It is to be understood that the above reported expression may reflect interferon production by genes under the control of the penicillinase expression control sequence.

It is to be understood that the actual protein produced by these strains has not been analyzed structurally to determine whether or not it is produced fused to amino acids unrelated to IFN or with all or part of IFN's signal sequence. However, whatever protein is produced, it displays an immunological or biological activity of IFN. Therefore, the protecin as expressed is useful. Most importantly, the activity of the protein demonstrates that the DNA sequence which codes for it is a DNa sequence related to HuIFN-$\alpha$. It therefore is within the skill of the art to employ that DNA sequence as is demonstrated herein to select other like HuIFN-$\alpha$ related DNA se-

\* Three thousand liters or larger cultures of IFN-$\alpha$ can be grown without loss of IFN activity.

quences and to provide the basis for other constructions that will express mature interferon or other variants thereof or will improve the yield of the particular protein expressed.

The level of production of a protein is governed by two major factors: the number of copies of its gene within the cell and the efficiency with which those gene copies are transcribed and translated. Efficiency of transcription and translation (which together comprise expression) is in turn dependent upon nucleotide sequences, normally situated ahead of the desired coding sequence. These nucleotide sequences or expression control sequences define, *inter alia,* the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for the desired protein from their adjacent nucleotide sequences and fuse them instead to other known expression control sequences so as to favor higher levels of expression. This having been achieved, the newly engineered DNA fragment may be inserted into a multicopy plasmid or a bacteriophage derivative in order to increase the number of gene copies within the cell and thereby further to improve the yield of expressed protein.

Several expression control sequences may be employed as described above. These include the operator, promoter and ribosome binding and interaction sequences (including sequences such as the Shine-Dalgarno sequences) of the lactose operon of *E. coli* ("the *lac* system"), the corresponding sequences of the tryptophan synthetase system of *E. coli* ("the *trp* system"), the major operator and promoter regions of phage $\lambda$ ($O_L P_L$ and $O_R P_R^i$ the control region of the phage fd coat protein, or other sequences which control the expression of genes of prokaryotic or eukaryotic cells and their viruses. Therefore, to improve the production of a particular polypeptide in an appropriate host, the gene coding for that polypeptide may be prepared as before and removed from a recombinant DNA molecule containing it and reinserted into a recombinant DNA molecule closer to its former expression control sequence or under the control of one of the above expression control sequences. Such methods are known in the art.

Further increases in the cellular yield of the desired products depend upon an increase in the number of genes that can be utilized in the cell. This is achieved, for illustration purposes, by insertion of recombinant DNA molecules engineered in the way described previously into the temperate bacteriophage $\lambda$ (NM989), most simply by digestion of the plasmid with a restriction enzyme, to give a linear molecule which is then mixed with a restricted phage $\lambda$ cloning vehicle *(e.g., of the type described by N. E. Murray et al.,* "Lambdoid Phages That Simplify The Recovery Of In Vitro Recombinants", *Molec. gen. Genet.* 150, pp 53-61 (1977) and N. E. Murray *et al.,* "Molecular Clonong Of The DNA Ligase Gene From Bacteriophage T4", *J. Mol. Biol.,* 132, pp. 493-505 (1979)) and the recombinant DNA molecule produced by incubation with DNA ligase. The desired recombinant phage is then selected as before and used to lysogenise a host strain of *E. coli.*

Particularly useful $\lambda$ cloning vehicles contain a temperature-sensitive mutation in the repression gene *cl* and suppressible mutations in gene *S*, the product of which is necessary for lysis of the host cell, and gene *E,* the product which is the major capsid protein of the virus. With this system the lysogenic cells are grown at 32°C and then heated to 45°C to induce excision of the prophage. Prolonged growth at 37°C leads to high levels of production of the protein, which is retained within the cells, since these are not lysed by phage gene products in the normal way, and since the phage gene insert is not encapsidated it remains available for further transcription. Artificial lysis of the cells then releases the desired product in high yield.

In an initial attempt to increase the yield of polypeptide, displaying a biological or immunological activity of human leukocyte interferon, produced from hosts transformed with Z-pBR322(Pst)/HcIF-SN35 by the process above described, a restriction map of the DNA insert in the hybrid was determined. This mapping revealed that as compared to Hif-2h, Hif-SN35 was lacking a *Pst*I site flanking the 3' end of the sequence, part of the signal sequence was missing (up to and including codon 7) and the *Ava*II site in the signal sequence had been replaced by a *Bsp*I site. Therefore, Hif-SN35 is likely a polymorphic or allelic varient of Hif-2h.

The plasmid Hif-SN25 was opened with *Pst*I and the resulting DNA strand chewed back at both ends using standard procedures and the LAC-*Alu* fragment *(infra)* inserted therein and the plasmid reclosed. The actual structure of the modified plasmid, identified as Z-pBR322(Pst)/HcIF-SN35-AHL6. and the amino acid sequence at the amino terminal end of the protein produced in *E. coli* have been determined. The nucleotide sequence of this construction reveals that the LAC-*Alu* fragment was attached one amino acid away from the first amino acid of IFN-$\alpha$I(SN35). The amino acid sequence of the amino terminal portion of the protein expressed in *E. coli* revealed that a fused protein was. produced having six amino acids fused to the IFN-$\alpha$I(SN35) sequence. However, hosts transformed with the modified plasmid produce about 100 times more polypeptide displaying a biological or immunological activity of human leukocyte interferon as compared to hosts transformed with unmodified Z-pBR322(Pst)/HcIf-SN35.

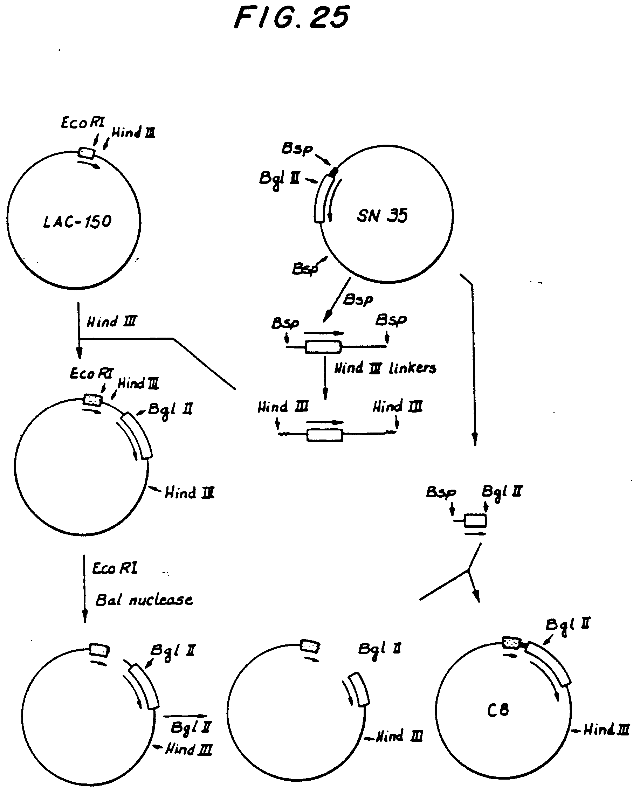

Referring now to Figure 25, another attempt to improve the yield of polypeptide, displaying an immunological or biological activity of human leukocyte interferon, produced by hosts transformed with Z-pBR322(Pst)/ HcIF-SN35 (SN35 in Figure 25) is depicted. The hybrid plasmid was cleaved using standard procedures with *Bsp*I (a gift of Dr. Kiss). After heat inactivation (65°C, 30 min) of the restriction enzyme, the mixture was adjusted to 50 mM Tris-HCl (pH 8) and heated (37°C, 30 min). Following extraction with phenol and ether, the largest. $\alpha$IcDNA fragment was isolated on low temperature gelling agarose (0.8%) and *Hind*III linkers attached. The modified fragment was then joined to *Hind*III-cleaved plasmid HS-pBR322(Eco)/lacUV5-150 ("LAC-150")* (a gist of H. Schaller) by melting the fragment-containing gel pieces (about 20 $\mu$l each) at 65°C, cooling to 37°C and adding 20 U per $\mu$l T4 DNA ligase. After 16 h at 15°C, ligation occurred in the solidified gel (H. Lehrach, personal communication 1980). One tenth vol 100 mM Tris-Hcl (pH 7.5), 100 mM $CaCl_2$, 100 mM $MgCl_2$ were added to the sample and it was heated 5 min at 65°C and cooled to 37°C. The samples were then used to transform $Ca^{+2}$ treated *E. coli* HB101, incuated at 0°C for 20 min, heated at 42°C for 1 min and for 10 min at 20°C. After addition of 1 mol tryptone medium, the samples were incubated 60 min at 37°C and plated on to agar plates containing ampicillin. Plasmid DNA was separated from these cultures, as before, and the hybrid plasmid containing the IFN-$\alpha$l insert with its 5' end adjoining the LAC fragment identified by restriction analysis. The plasmid was then cleaved with *Eco*RI using conventional procedures and digested with exonuclease BAL-31 (0.06 U/ml, 2-4 min at 30°C) to remove the over-hanging *Eco*RI tail of the LAC fragment and to shorten the $\beta$-galactosidase coding segment.

To ensure that the treated plasmid contained the complete IFN-$\alpha$l coding sequence, the plasmid was then cleaved with *Bg*/II, using conventional procedures, worked up as before and the largest fragment separated on agarose gel (0.8%). This fragment was then combined with a *Bsp*I-*Bs*/I fragment from Z-pBR322(Pst)/HcIF-SN35 and the resulting hybrid plasmid used to transform *E. coli* HB101 as before. The transformed colonies were screened for IFN activity and one clone having a high level of IFN activity was selected. This clone was designated *E. coli* HB101 (C8-IFN-$\alpha$l) and its hybrid plasmid C8-IFN-$\alpha$l.

DNA sequence analysis of C8-IFN-$\alpha$l revealed that the coding sequence following the initiator triplet determined the first seven amino acids of $\beta$-galactosidase, a Pro residue generated by the fusion, amino acids 16 to 23 of the IFN-$\alpha$l signal sequence and the IFN-$\alpha$l (SN35) sequence. *E. Coli* minicell strains (DS410)transformed with hybrid plasmid C8-IFN-$\alpha$l produce about 50 million units IFN per liter or about 2500 times more polypeptide displaying an immunological or biological activity of HuIFN as compared to minicells transformed with unmodified Z-pBR322(Pst)/Hif-SN35. Amino acid sequencing of the polypeptide produced by plasmid C8-IFN-$\alpha$l confirms that the product is a fused protein having seven amino acids from $\beta$-galactosidase. one amino acid generated from the fusion and amino acids 16-23 of the IFN-$\alpha$l signal sequence fused to IFN-$\alpha$l.

Further, examples of various constructions to improve the protein yields in accordance with this invention are discussed in connection with other forms of IFN-$\alpha$ *(infra).*

## PROPERTIES OF INTERFERON ACTIVITY PRODUCED BY E. COLI TRANSFORMED WITH HYBRID PLASMIDS

### 1. Sensitivity of IFN-$\alpha$ Activity To Trypsin

50 $\mu$l samples of authentic HuIFN-$\alpha$ (specific activity, $1.2 \times 10^6$ U/mg; 5OU), and the S100 extracts described above of *E. coli* HB101 (Z-pKT287(Pst)/HcIF-2H-AH6) ("Hif-287-6 extracts") (200 U/ml, 10 U) and of *E. coli* HB101 (Z-pBR322(Pst)/HcIF-SN35) ("Hif-35 extracts") (1000 U/ml: 50 U) were incubated with various amounts of trypsin, as indicated below, for 30 min at 37°C. Since the S100 extracts have a high protein content, while the HuIFN-$\alpha$ does not, a mixture of HuIFN-$\alpha$ and the control S100 extract Hif-32 was tested in parallel:

* This plasmid contains the lac promoter HaeIII-202 bp fragment (W. Gilbert *et al., "Lactose Operator Sequences And The Action Of Lac Repressor"* in *Protein Ligand Interactions,* H. Sung and G. Blauer, eds. (Berlin, Walter de Gruyter), pp. 193-206 and K. Backman *et al.,* "Maximizing Gene Expression On A Plasmid Using Recombination In Vitro", *Cell,* 13, 65-71 (1978)) flanked by an *Eco*RI linker at its 3' end.

| IFN-$\alpha$ preparation | Trypsin ($\mu$g) | IFN-$\alpha$ activity (units) |
|---|---|---|
| HuIFN-$\alpha$ (50 units in 50 $\mu$l Hif-32 S100 extracts) | 0 | 50 |
| | 0.1 | 50 |
| | 1 | 50 |
| | 10 | 5 |
| | 50 | 0 |
| Hif-287-6 S100 extract (10 units) | 0 | 15 |
| | 0.1 | 15 |
| | 1 | 5 |
| | 10 | 1 |
| | 50 | 0 |

Therefore the IFN-$\alpha$ of the extracts in sensitive to trypsin and hence a protein.

| | | |
|---|---|---|
| Hif-35 S100 extract 50 units) | 0 | 30 |
| | 0.1 | 20 |
| | 1 | 20 |
| | 10 | 2 |
| | 50 | 0 |

2. Behavior on Chromatography On Sephadex, G-100

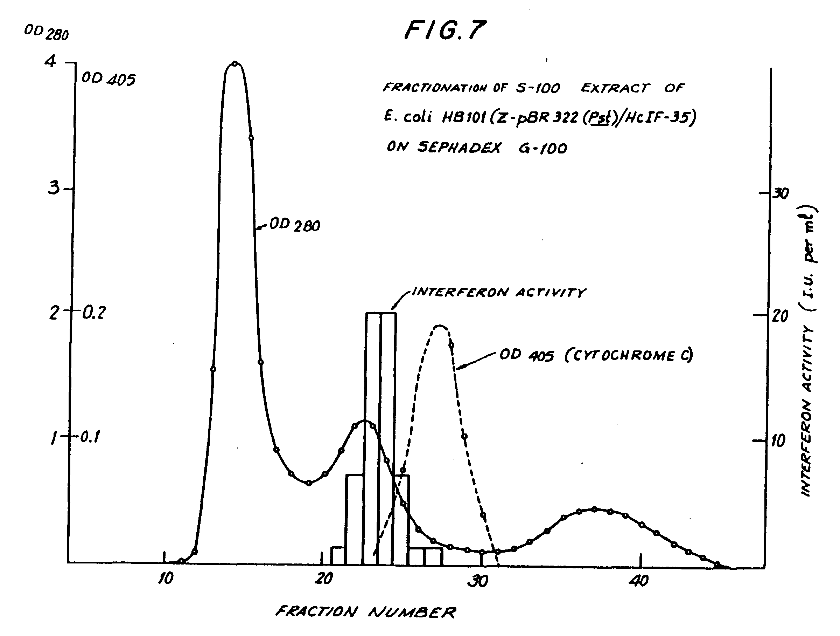

Extract Hif-35 (1 ml) and the S100 extract of *E. coli* HB101 (Z-pBR322(Pst)/HcIF-SN32) ("Hif-32 extracts") were chromatographed on a 32-ml Sephadex G-100 column at 4°C in 50mM K-phosphate buffer (pH 7.4). Cytochrome c (0.2 mg) was added as an internal marker. The flow rate was 2 ml/hr and 1.0 ml fractions were collected. The absorbance at 280 nm and 405 nm (cytochrome c), and the IFN-$\alpha$ activity were determined. As shown in Figure 7, the IFN-$\alpha$ activity of Hif-35 extracts was eluted before cytochrome c, with a $k_D$ value of about 0.45. Therefore, the apparent molecular weight of the substance was between about 20,000 and 30,000* ; no activity was detected in the fractions of control extract Hif-32.

3. Inhibition Of The Interferon Activity Of Hif-35 And Hif-287-6 By Antibody Against Human Leukocyte Interferon.

HuIFN-$\alpha$ (specific acitivity 1.2 × 10⁶ IU/mg), and the Hif-35/Hif-287-6 S100 extracts were incubated with various dilutions of sheep antiserum against HuIFN-$\alpha$ (prep. K. Cantell, Feb. 24, 1976. specific acitivity 450,000 units/ml) in 100 $\mu$l Modified Eagles Medium (MEM) with 10% calf serum for 30 min at 37°C and 45 $\mu$l were assayed for IFN-$\alpha$ activity by the cytopathic effect reduction assay. (The antibody itself did not cause a cytopathic effect):

* The molecular weight determined by nucleotide sequencing and assuming no carboxyterminal processing is 19,388.

| IFN-α Preparation (units) | | Anti-leukocyte-interferon antibody (units) | Residual IFN-α activity (IU) |
|---|---|---|---|
| IFN-α | | 0 | 5 |
| | (10) | 0.18 | ∼0.5 |
| | | 9 | <0.1 |
| | | 450 | <0.1 |
| Hif-35 extract | | 0 | 15 |
| | (25) | 0.18 | 15 |
| | | 9 | <0.1 |
| | | 450 | <0.1 |
| Hif-287-6 extract | | 0 | 15 |
| | (25) | 0.18 | 15 |
| | | 9 | <0.1 |
| | | 450 | <0.1 |
| none | | 0 | <0.1 |
| | | 450 | <0.1 |

To show that the action of the antibody was not due to an unspecific effect, such as proteolytic degradation, a similar experiment was performed with the mouse interferon system:

| IFN Preparation (Units) | Anti-leukocyte-interferon antibody (units/ml) | IFN activity (unites/ml) mouse system |
|---|---|---|
| mouse preparation | 4500 | 100 |
| (100 units) | 90 | 100 |
| | 18 | 100 |

Thus, antibodies directred against HuIFN-α specifically inhibit the IFN-α activity of polypeptides produced in E. coli transformed with certain recombinant DNA molecules containing the HcIF-2h DNA sequence. The apparently lower affinity of the antibody for the IFN-α produced in E. coli may reflect structural differences between the latter and natural HuIFN-α, for example, absence of carbohydrate moiety, presence of signal sequence, or fusion to part of the β-lactamase sequence.

4. Reduced Activity Of Hif-35 And Hif-287-6 Extracts On Mouse Cells

Human CCL23 or Mouse L929 cells were treated with E. coli extracts, HuIFN-α (prep., K. Cantell, specific activity $1.2 \times 10^6$ unites/mg) or mouse IF (N.I.H. standard), were challenged with virus (Mengo virus in the case of human cells and VSV in the case of mouse cells) and the IFN-α activity determined by the cytopathic effect reduction assay:

| | IFN activity (units/ml) | |
| Addition | human system | mouse system |
| --- | --- | --- |
| mouse-interferon (120 units/ml) | — | 120 |
| Hif-35 extracts | 100 | 13 |
| | 1000 | 120 |
| Hif-287-6 extracts | 30 | 4 |
| | 300 | 40 |
| HuIFN-$\alpha$ (100 units/ml) | 100 | 4 |
| HuIFN-$\alpha$ (1000 units/ml) | 1000 | 40 |

These results show that Hif-35 and Hif-287-6 extracts have a protective action on human cells and only a slight effect ($\sim$10%) on mouse cells, as is typical for human interferon.

5. Effect On Some Cell Function

Extracts from *E. coli* HB101 (Z-pBR322(Pst)Hif-SN35-AHL6) were compared with authentic IFN for its effect on some cell functions. The *E. coli* made IFN-$\alpha$ displayed the following properties of natural IFN-$\alpha$: (1) it enhances natural killing activity of human lymphocytes; (2) it enhances antibody-dependent cell-mediated cytotoxicity; (3) it suppresses antigen- and mitogen-induced leukocyte migration inhibition; and (4) it inhibits growth of IFN-sensitive Burkitt lymphoma cells. These properties are indicia of *E. coli* synthesized IFN-$\alpha$l's activity against human tumors and cancers.

6. Activity Of IFN-$\alpha$ Without Amino-Terminal Sequences

IFN-$\alpha$ without amino-terminal sequences has also been made in *E. coli* and shown to display activity consistent with IFN activity.

To construct the appropriate recombinant DNA molecule, plasmit Hif-2h *(supra)* was partially digested with *Eco*RI and *Bam*HI and the fragment containing the IFN-$\alpha$l coding sequence separated on agarose gel and combined with the non-IFN-$\alpha$l coding sequence obtained from an *Eco*RI/*Bam*HI restriction of plasmid Hif-SN35 which is missing a *Pst*I site adjacent the 3' end of the hybrid insert as compared to Hif-2h (supra). The resulting plasmid was then restricted with *Pst*I/*Bg*I to remove a portion of the amino terminal part of the IFN-$\alpha$l coding sequence. Inserted in its place were a series of IFN-$\alpha$l fragments prepared by digestion of plasmid Hif-2h with *Pvu*II, treatment with *Bal* exonuclease, attachment of *Pst*I linkers and restriction with *Bg*/I. The resulting plasmids thus contained a series of IFN-$\alpha$l coding sequences which lacked various portions of their amino terminal sequences. One of these (plasmid 2H-M8) was digested with *Pst*I and its nucleotide sequence determined. Sequencing revealed that the plasmid 2H-M8 contained several nucleotides between the *Pst* site and the first codon (CYS) of IFN-$\alpha$l. Therefore, the *Pst*I digested plasmid 2H-M8 was treated with T4 polynuclease/dATP, SI exonuclease and digested with *Sal*I. This precedure generated a series of fragments whose IFN-$\alpha$l coding sequences were missing portions of their amono terminal end. These fragments were then placed under LAC control by operatively linking them to a GUA-LAC fragment prepared from plasmid Lac3∇8 by digestion with *Eco*RI, treatment with exonuclease SI and digestion with *Sal*I. The resulting series of plasmids thus has IFN-$\alpha$l coding sequences lacking various portions of their amino terminal ends attached in a counterclockwise direction via an AUG to a fragment containing the LAC promoter.

Some of these plasmids were sequenced. One began at the fifth amino acid of IFN-$\alpha$l and one at the tenth amino acid of IFN-$\alpha$l. In *E. coli* minicells (DS410) both of these plasmids produced polypeptides that displayed IFN activity. Therefore, not all of the IFN-$\alpha$l protein is required for IFN activity.

IDENTIFICATION OF CLONES OF E. COLI CONTAINING RECOMBINANT DNA MOLECULES WHOSE DNA INSERTS WEAKLY CROSS-HYBRIDIZE TO THE INSERT IN Hif-4c AND HAVE A DIFFERENT RESTRICTION MAP THAN THE Hif-2h FRAGMENT

The comparison of the first 35 amino acids of authentic lymphoblastoid interferon (Zoon *et al., supra,* and M. Hunkapiller and L. Hood, *supra)* and the sequence deduced from Hif-2h fragment shows 9 differences. In all cases, the codons for the differing amino acids could be related by one-base changes. The amino acid compositions determined directly for authentic lymphoblastoid interferon on the one hand and deduced from the sequence of the Hif-2h fragment on the other, also show striking differences in regard to their content of Gly, Pro, Cys and Met. These differences are too large to be explained by polymorphism. Instead, they most likely reflect the existence of at least two non-allelic genes, because the degree of divergence of the two proteins (26% mismatch) is similar to that between, for example, human and sheep B-globin (23% mismatch). Accordingly, the clones that displayed weak hybridization to fragment Hif-4c. identified previously *(supra),* were examined and a clone *E. coli* HB101 (Z-pBR322(Pst)/HclF-II-206) was identified.

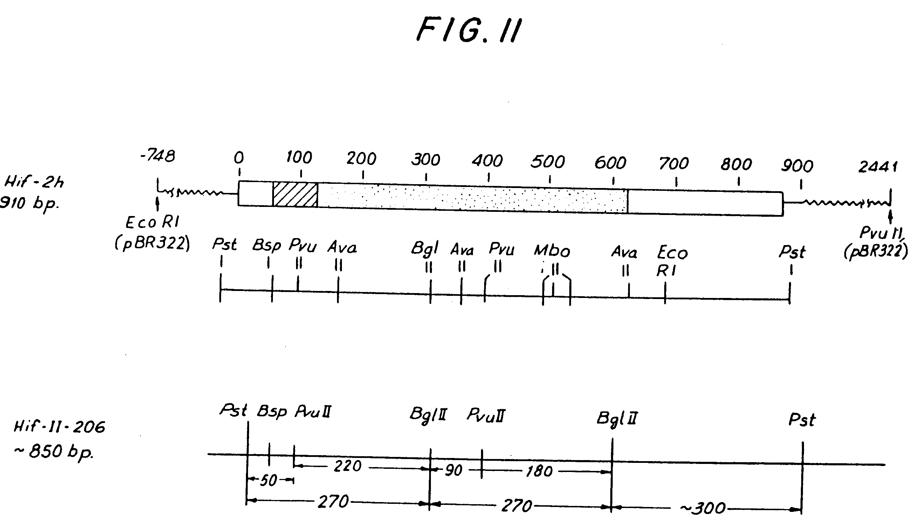

The hybrid plasmid Z-PBR322(Pst)/HclF/II/206 ("HclF-II-206") of this clone and its DNA insert "Hif-II-206 fragment" are weakly hybridizing to Hif-4c and Hif-2h fragment. *E. coli* transformed with plasmid Hif-II-206 produces polypeptides displaying a biological or immunological activity of HuIFN-α. Hif-II-206 fragment has a restriction map that is distinct from that determined for the Hif-2h fragment. A comparison of the two restriction maps is set forth in Fig. 11. Again, the absolute location of the restriction sites in the Hif-II-206 fragment is not determined by restriction mapping alone. However, sequencing of the nucleotide sequence of this insert, using the standard procedures described above, permits more absolute determination of these locations. However, because of the differences in the restriction map of the Hif-II-206 fragment as compared to the Hif-2h fragment, it is clear that the interferon genes of the two inserts have different nucleotide sequences.

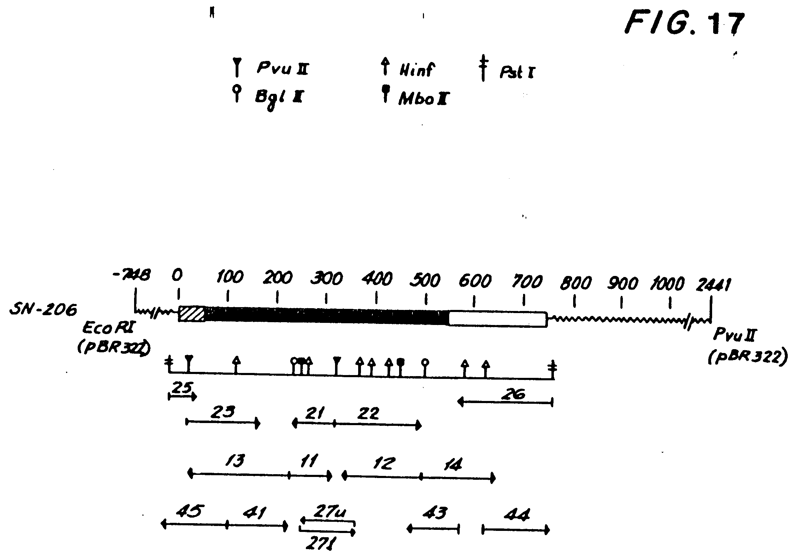

Referring now to Figures 12-16, the nucleotide sequences of the inserted DNA sequence-Hif-II-206 fragment-of culture HclF-G and the inserted DNA sequence-Hif-2h fragment-of culture HclF-E *(infra),* determined previously, and the corresponding amino acid sequences of the proteins coded for by those nucleotide sequences are displayed. The nucleotide sequence of the Hif-II-206 fragment-the *Pst* I fragment (790bp) of the plasmid DNA isolated using the procedure of Method B as described by N. M. Wilkie et al., *Nucl. Acids Res.,* 7, pp. 859-77 (1979) from culture HclF-G-was determined using the standard procedure of A. M. Maxam and W. Gilbert, *supra.* The sequencing strategy employed is shown in Figure 17.

Restricted DNA (usually about 10 µg) was 5' terminally labelled as described by N. Mantei et al., *Gene,* 10, pp. 1-10 (1980). Labeled fragments were cleaved with a second restriction enzyme, the products separated by electrophoresis through a 5% polyacrylamide gel in Tris-borate-EDTA buffer (A. C. Peacock and C. W.- Dingman, *Bioch.,* 6, pp. 1818 -27 (1967)), extracted from the gel, and purified as described by W. Muller et al., *J. Mol. Biol.,* 124, pp. 343-58 (1978).

Referring now to Figure 17, the various fragments for sequencing were prepared as follows:

25 and 26-cleavage of Hif-II-206 with *Pst*I, labelling, cleavage with *Bg*/II, isolation of a *Pst*I*-*Bg*/II fragment (257bp) ("25") and a *Pst*I*-*Bg*/II fragment (279bp) ("26");

21, 22 and 23-cleavage of Hif-II-206 with *Pvu*II, labelling, cleavage with *Bg*/II, isolation of a *Pvu*II*-*Bg*/II fragment (88bp) ("21"), a *Pvu*II*-*Bg*/II fragment (1 76bp) ("22") and a *Pvu*I*-*Bg*/II fragment (214bp) ("23");

11, 12, 13, and 14-cleavage of Hif-II-206 with *Bg*/II, labelling, cleavage with *Pst*I, isolation of a *Bg*/II*-*Pst*I fragment (279bp) ("14") and a comigrating mixture of a *Bg*/II*-*Pst*I fragment and a *Bg*/II*-*Bg*/II* fragment. Cleavage of the mixture with *Pvu*II and isolation of a *Bg*/II*-PstI fragment (257bp) ("13"), a *Bg*/II*-*Pvu*II fragment (176bp) ("12") and a *Bg*/II*-*Pvu*II fragment (88bp) ("11").

27L, 27U, 41, 43, 44 and 45-cleavage of Hif-II-206 with *Hinf*I, labelling, isolation of precursor fragments: *Hinf*I*-*Hinf*I* (11 3bp) ("27P"), *Hinf*I*-*Hinf*I* (146bp) ("28P"), *Hinf*I*-*Hinf*I* (159bp) ("30P"), *Hinf*I'*Hinf*I* (397bp) ("31P") and *Hinf*I*-*Hinf*I* (1522bp) ("32P"). Cleavage of 28P with *Mbo*II and isolation of a fragment *Hinf*I*-*Mbo*II (112bp) ("41"). Cleavage of 30P with *Mbo*II and isolation of a frag- ment *Hinf*I*-*Mbo*II (126bp) ("43"). Cleavage of 31P with *Pst*I and isolation of a fragment *Hinf*I*-*Pst*I (151 bp) ("44"). Cleavage of 32P with *Pst*I and isolation of a fragment *Hinf*I*-*Pst*I (139bp) ("45"). Strand separation of 27P to yield two strands ("27U" and "27L").

All segments, except 27U and 27L, were sequenced on both strands and across the restriction sites that served as origins for sequencing, except for the *Bg*/II site at position 185.

From a comparison of the amino acid sequence coded for by the two inserts it is apparent that the Hif-II-206 fragment codes for an interferon-like protein having one less amino acid than does the Hif- 2h fragment (amino acid 44 (Asp) present in Hif-2h is missing in Hif-II-206). Moreover, 10% of the nucleotide positions and 17% of the derived amino acid residues of the two fragments are different.

In addition, when compared to the 35 amino acids determined for the amino terminus of lymphoblastoid interferon (K. C. Zoon et al., *Science,* 207, pp. 527-28 (1980)), the insert Hif-II-206 codes for a protein that differs in 5 residues from the 35 amino acid residues determined by Zoon et al., *supra.* Therefore, at least three different IFN genes of the leukocyte type ($\alpha$ type) must exist-Hif-2h fragment, Hif-II-206 fragment and the gene coating for Zoon's IFN. In accordance with the newly proposed nomenclature for interferon, hereinafter the proteins coded for by these genes will be identified as follows:

| IFN Gene Source | Protein |
|---|---|
| Hif-2h | IFN-$\alpha$1 |
| Hif-II-206 | IFN-$\alpha$2 |
| IFN from lymphoblastoid cells (Zoon et al. (*supra*)) | IFN-$\alpha$3 |

The differences between IFN-$\alpha$1 and IFN-$\alpha$2 are also reflected in their varying activities on human CCL23 and bovine embryonic kidney (BEK) cells:

| Extract | Relative IFN Activity* | | |
|---|---|---|---|
| | CCL23 | BEK | Ratio |
| Hif-II-206 (IFN-$\alpha$2) | 1.7 | 1.0 | 1.7:1 |
| Hif-SN35** (IFN-$\alpha$1) | 0.05 | 1.0 | 1:20 |

* *E. coli* HB101 containing the hybrid plasmid were grown in tryptone medium with shaking to an $OD_{650}$ of about 1—2. The cells were harvested, weighed, resuspended in 1/100 or 1/20 of the original culture volume and lysed by the lysozyme-freeze-thaw method (S. Nagata et al., *Nature,* 284, pp. 316—20 (1980)). The S-30 supernatants were tested by CPE reduction assay in microtiter plates. Extracts from control cells ere negative ($<$1 I.U./ml). Human CCL23 cells and bovine embryonic kidney (BEK) cells (FLOW) were grown in MEM-10% fetal calf serum. Exposure to IFN-containing extracts was for 24 h. The cells were challenged with an appropriate dilution of Mengo virus and stained 24 h later. Values were estimated visually relative to partially purified leukocyte IFN (preparation PIF, a gift of K. Cantell) of known titer. This preparation was about 3x more active on human than on bovine cells.
** The comparison of the restriction maps of Hif-SN35 and Hif-2h suggest that they are polymorphic variants of each other (*supra*).

Therefore, IFN-$\alpha$1 is about 30 times less active on human cells than IFN-$\alpha$2. Yet, they are both about equally active on bovine cells. Therefore, IFNs may, in addition to their use as antiviral and antitumor or anticancer agents in humans, also be useful in treating these conditions in cattle. For example, preparations of HuIFN-$\alpha$ could be employed in a standard manner *(supra)* in treating FMDV and other well known viral infections of cattle. This is even more particularly true for IFN-$\alpha$1 since its activity in bovine cells is about 20 times greater than its activity on human cells.

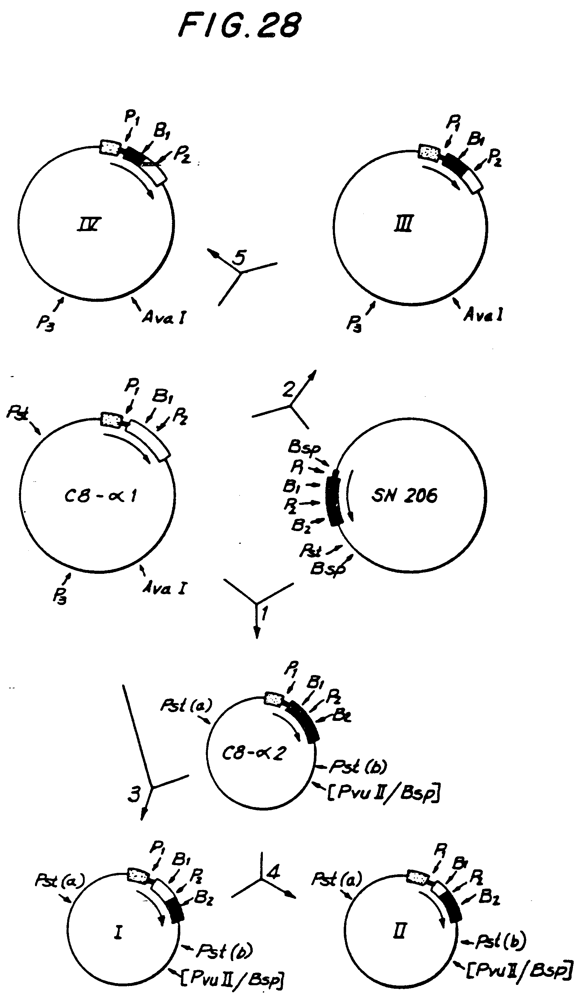

Because of the improved yield attained in the case of IFN-$\alpha$1 with construction C8 *(supra),* a similar construction was made for IFN-$\alpha$2. Z-pBR322(Pst)/Hif-11-206 was cleaved completely with *Bsp*I and partially with *Pvu*II (at PI) (Figure 28) and the 867 bp fragment was isolated from a 6% polyacrylamide gel. This fragment was then ligated to a 2590 bp *Pvu*II fragment of C8-IFN-$\alpha$1.* The resulting hybrid plasmid was used to transform *E. coli* HB101 and the clones screened for IFN activity. One clone displaying a high activity was selected and designated *E. coli* HB101 (C8-IFN-$\alpha$2).

DNA sequencing of hybrid plasmid C8-IFN-$\alpha$2 revealed that it like C8-IFN-$\alpha$1 had a coding sequence following the initiator triplet that determined the first seven amino acids of $\beta$-galactosidase, a Pro residue generated by the fusion and amino acids 16 to 23 of the IFN-$\alpha$22 signal sequence. Therefore, again a fused protein containing IFN-$\alpha$2 is likely to be expressed.

Minicells transformed with this plasmid gave 100-200 millions units per 1 of IFN or 20000 to 40000 times higher yields of IFN-$\alpha$2 than minicells transformed with unmodified Z-pBR322(Pst)/Hif-II-206.

A comparison of the relative yields of C8-IFN-$\alpha$1 ($\sim$50 $\times$ 10 units/liter) and C8-IFN-$\alpha$2 ($\sim$100-200 million units per liter) is at first surprising because IFN-($\alpha$2 has been shown to be about 30 times more active than

* C8-IFN-$\alpha$1 has three *Pvu*II restriction sites (Figure 28). The 2590 bp fragment is between $P_1$ and $P_3$.

IFN-α1 on human cells *(supra)*. However, quantitative analysis of the amount of the two proteins made by minicells transformed with the two C8 plasmids revealed that in C8-IFN-α1 about 5 to 6 times more protein than in C8-IFN-α2 was being made. Therefore, the yields measured on the basis of IFN activity had been skewed by the much greater amount of protein made in the case of C8-IFN-α1.

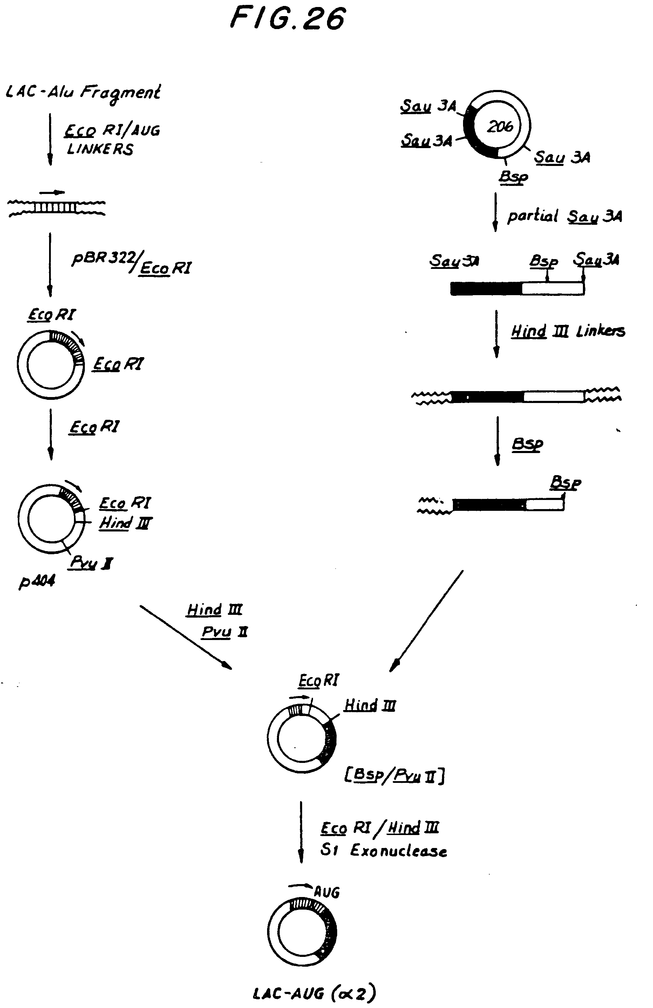

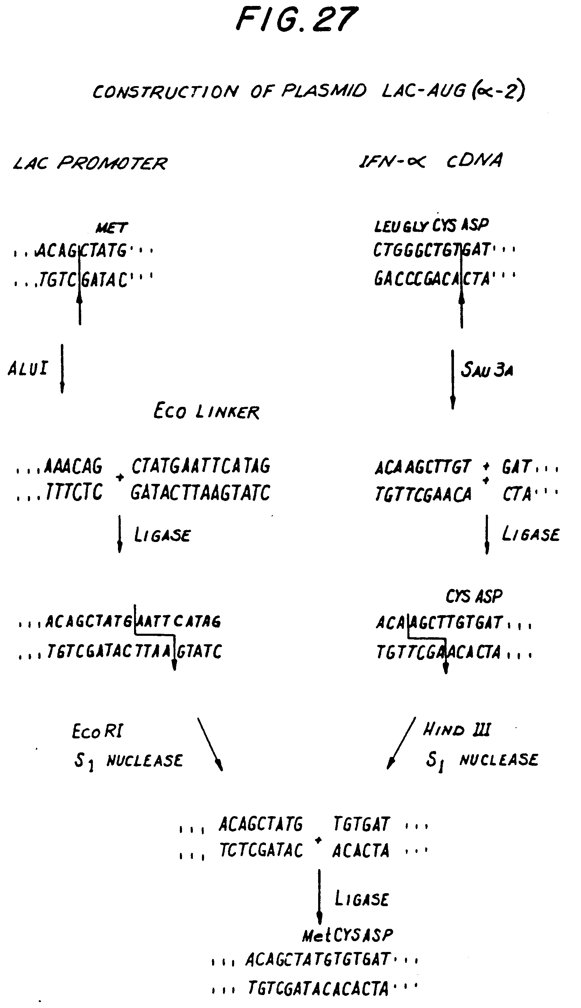

Referring now to Figure 26, another construction in an attempt to improve the yield of IFN-α2 is described. First, an expression plasmid containing the LAC *Alu* fragment was prepared by restricting the known lac promoter with *Alu*I and extending the fragment as shown (for one termini) in Figure 27 with *Eco*RI linkers (prepared by colloborative research). The extended fragment was then inserted into pBR322 at the *Eco*RI site and a small *Eco*RI-*Eco*RI fragment deleted from the construction. The resulting plasmid, designated 404 in Figure 26 was cleaved with *Hind*III and *Pvu*II for insertion of the IFN-α2 containing fragment. That IFN-α2 fragment was prepared by partial *Sau*3A restriction of Z-pBR322(Pst)/HcIF-II- 206 ("206" in Figure 26), extension of the *Sau*3A fragment with *Hind*III linkers (Figure 27) and cleavage with *Bsp*I. After insertion of this fragment into the *Hind*III-*Pvu*II cleaved plasmid 404, the resulting plasmid was restricted with *Hind*III and *Eco*RI. treated with SI nuclease to bring the LAC promoter closer to the IFN-α2 gene and religated. This construction identified as plasmid LAC-AUG(α2) has the IFN-α2 DNA sequence under the control of the LAC promoter. Moreover, the IFN-α2 sequence immediately follows the initiating AUG codon of the promoter (see Figure 27). Therefore, at least a portion of the IFN produced by these plasmids will be mature IFN, *e.g.,*IFN without any amino acids from the signal sequence. In minicells the yield of IFN-α2 obtained with plasmid LAC-AUG(α2) was 5-10 million units per liter.

Another IFN-α2 construction based on similar linking principles has also been made. Here, the penicillinase expression control sequence of pBR322 has been connected via an AUG initiator codon to the IFN-α2 gene from Hif-II-206.*  This plasmid designated as β-lac-AUG(α2) when used to transform host cells affords the production of IFN-α2 without fusion to other protein sequences. In minicells, yields of 50-100 million units per liter have been observed. This plasmid is the most preferred plasmid for use in accordance with this invention. It is also preferred for use with the other IFN-a genes disclosed herein. The preferred host in accordance with this invention is *E. coli* DS410 (ara azide TonA lac y Tsx min a min b gal λ xyl step$^R$). This strain has been deposited together with an example of the preferred plasmid β-lac-AUG(α2) as HcIF-K.

Other constructions using various promoter sequences, ribosime binding sites. Shine-Dalgarno sequences and DNA sequences between the promoter and the AUG initiator codon and using various of the IFN-α genes disclosed herein can also be constructed using similar methods and principles. These constructions are of course envisioned by this invention and are within the scope thereof.

## HYBRID MOLECULES OF IFN-α1 AND IFN-a2

A number of hybrid molecules of IFN-α1 and IFN-α2 have been constructed. Surprisingly these hybrid constructions have quantitatively different properties and activities as compared either of their parents-IFN-α1 or IFN-α2.

Referring now to Figure 28, a schematic outline of the construction of four of these hybrid molecules is displayed. For convenience these hybrid molecules are designated as plasmids I, II, III and IV. In these constructions digestions with restriction enzymes (obtained from Biolabs, with the exception of *Bsp*I, a gift of Dr. Kiss) were carried out essentially as recommended by the suppliers. Partial DNA cleavages were carried out with decreased amounts of enzyme. After heat inactivation (65°C, 30 min) of the restriction enzyme, the samples were adjusted to 50 mM Tris-HCl (pH 8) and if required calf intestine alkaline phosphatase (Boehringen) (1 vol per μg DNA) were added. After 30 min at 37°C, the samples were extracted with phenol and ether. In most cases the DNA fragments were separated on low temperature gelling agarose (0.8%). For ligation the fragment containing gel pieces (about 20 μl each) were melted at 65°C, cooled to 37°C and 20 U per μl T4 DNA ligase was added. The mixture was kept at 15°C for 16 hrs and ligation occurred in the solidified gel (H. Lehrach, personal communication (1980)). One tenth vol of 100 mM Tris-HCl (pH 7.5), 100 mM CaCl$_2$, 100 mM MgCl$_2$ was added, the sample heated 5 min at 65°C and cooled to 37°C. The samples were then added to Ca$^{+2}$-treated mini cells, incubated at 0°C for 20 min, heated 1 min at 42°C and 10 min at 20°C and 1 ml tryptone medium added. After incubation for 60 min at 37°C, the cultures were plated on to agar plates containing

*This construction could most effectively be made by partial digestion of pBR322 with *Mbo*II, treating with S1, attaching *Eco*RI linkers as before and reinserting the fragment into the *Eco*RI site of pBR322 and deleting one *Eco*RI site. The resulting plasmid (β-lac-AUG plasmid") could then be combined with an S1 treated (mild) *Hind*III linker-*Bsp*I fragment of 206 described previously. After cleavage with *Eco*RI and treating with S1 and phosphatase, the expression plasmid β-lac-AUG(α2) is isolated. Constructions with other genes could be done in a similar fashion by merely utilizing the construction β-lac-AUG plasmid for insertion of other genes or constructions or more preferably by using the *Sau*3A site in plasmid β-lac-AUG(α2) itself.

the appropriate antibiotics. All of the plasmids were characterized by nucleotide sequence analysis across the joint in the IFN sequence.

Hybrid molecule 1, an α-1(PvuII)α-2 hybrid was constructed by partially cleaving C8-IFN-α1 (supra) (C8-α1 in Figure 8) with PvuII, dephosphorylated. cleaved with PstI and the PstI-PvuII(P$_2$) 1346 bp fragment isolated. This fragment was ligated to a 2135 bp PstI(a)-PvuII(P$_2$) fragment prepared by totally cleaving C8-IFN-α2 (supra) (C8-α2 in Figure 28) with PvuII and partially cleaving it with PstI.

Hybrid molecule II, an α-1(BglII)α-2 hybrid, was constructed by cleaving hybrid molecule I with BglII. After dephosphorylation, the large BglII fragment was isolated and ligated to the small BglII fragment of C8-IFN-α2. After cloning, the hybrid plasmid having the small BglII fragment in the corresponding orientation was identified by restriction analysis.

Hybrid molecule III, an α-2(PvuII)α-1 hybrid, was constructed by partially cleaving C8-IFN-α1 with PvuII, dephosphorylating, cleaving with AvaI and isolating the 1686 bp PvuII(P$_2$)-AvaI and 3233 bp PvuII(P$_1$-AvaI fragments. These fragments were then ligated to the 300 bp PvuII(P$_1$)- PvuII(P$_2$) fragment of the HcIF-II-206 (supra) (SN206 in Figure 28) and the plasmid containing the small PvuII fragment identified by assaying transformed E. coli strains for IFN-α activity.

Hybrid molecule IV, an α-2(BglII)α-1 hybrid, was constructed by cleaving C8-IFN-α1 with BglII and AvaI and the 1776 bp fragment isolated. This fragment was then ligated to the 3543 bp BglII-AvaI fragment of hybrid molecule III.

The biological activities of the different interferon species relative to each other were also determined. Cultures of minicells (DS410) transformed with the various plasmids were grown and the bacteria harvested by centrifugation, washed with PBS, suspended in PBS (about 1/20 of the original vol), incubated for 60 min at 0°C with 1 mg/ml lysozyme, 10 mM EDTA, freeze thawed four times, sheared by passing five times through a syringe and cleaved by centrifugation.

The activities on human, mouse, guinea pig and bovine cells were as follows:

| Protein Source | Human (FS4) | Relative IFN Activity | | Guinea Pig |
| | | Bovine (BEK) | Mouse (L) | |
|---|---|---|---|---|
| C8-IFN-α2 | 1 | 1 | 0.01 | 0.03 |
| C8-IFN-α1 | 0.03 | 1 | 0.3 | 0.03 |
| Hybrid I | 0.001 | 1 | 0.003—0.008 | 0.003—0.01 |
| Hybrid II | 0.001 | 1 | 0.001 | 0.01 |
| Hybrid III | 1 | 1 | 1 | 0.3 |
| Hybrid IV | 0.1 | 1 | 2 | 0.1—0.005 |
| Negative Control | — | — | — | — |

Surprisingly, all of the interferons have about the same activity on bovine cells, yet IFN-α1 and the two hybrid IFN's (I and II) which have the amino terminal moiety of IFN-α1 have about a 10- to 1000-fold lower activity on human cells than IFN-α2 and the two hybrid IFN's (III and IV) having the amino terminal moiety of (IFN-α2. It is even more striking that the two hybrid IFN's (I and II) with the amino terminal part of IFN-α1 have more than a 10-fold lower activity on human cells than IFN-α1 itself. Yet, one of the hybrids (III) with the amino terminal part of IFN-α2 has about the same activity on human cells as IFN-α2.

IDENTIFICATION OF CHROMOSOMAL GENES FOR IFN-α

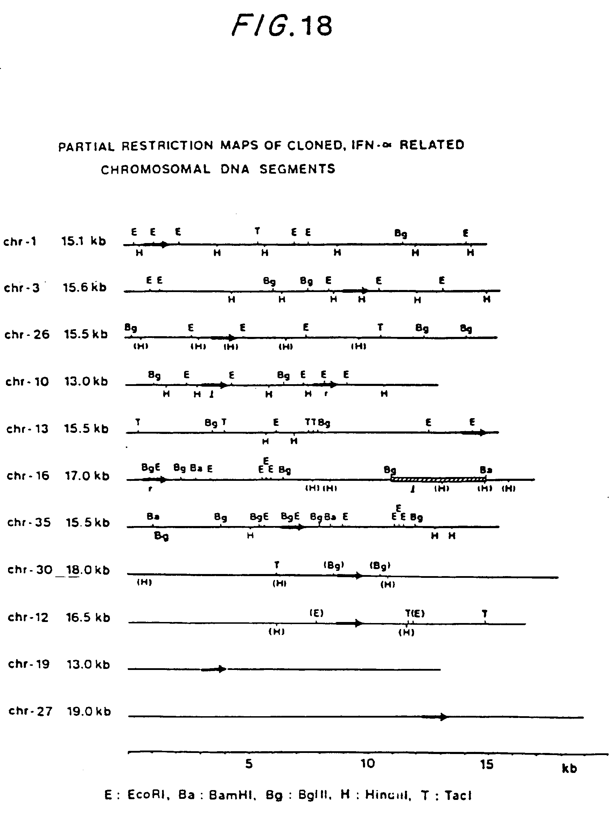

A collection of hybrid phage derived from fragments of fetal human chromosomal DNA which had been generated by partial cleavage with HaeIII and AluI, and joined with EcoRI linkers to λ Charon 4A arms has been prepared by R. M. Lawn et al., Cell, 15, pp. 1157-74 (1978). This gene bank was screened by an "in situ" procedure (W. D. Benton and R. W. Davis, Science, 196, pp. 180-82 (1977): T. Maniatis et al., Cell, 15, pp. 687-701 (1978)) using as a probe the $^{32}$P-labelled IFN-(αlcDNA insert excised from pBR322(Pst)/Hif-2h. Sixteen hybridization-positive phage clones were isolated from 240,000 plaques by repeated plaque purification (T. Maniatis et al., supra). Ten of the hybrid phage DNA preparations were cleaved with HindIII, TacI,HhaI, BamHI, EcoRI and BglII, respectively, and the fragments separated by electrophoresis on an agarose gel, transferred to a Millipore membrane (E. M. Southern, *J. Mol. Biol.,* 98, pp. 503-17 (1975)) and hybridized with the $^{32}$P-labelled Hif-2h cDNA insert. Figure 18 summarizes the results in the form of partial restriction maps and various tables. As displayed there, for each hybrid phage DNA preparation at least a few characteristic restriction sites were established and the region(s) hybridizing to the IFN-α1 gene probe delineated (black arrow).*

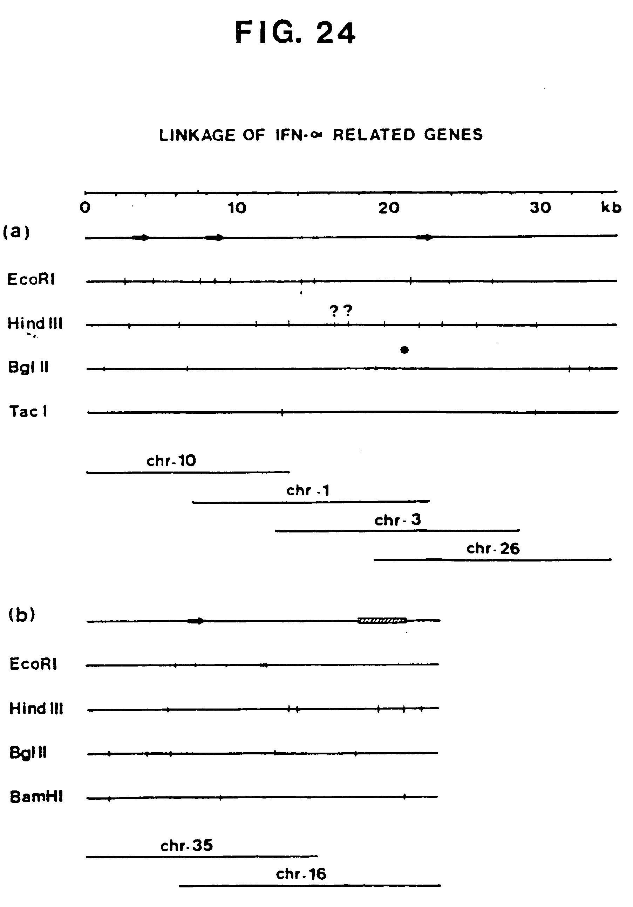

Referring now to Figures 18 and 24, it can be seen that clones chr-3 and chr-26 may represent DNA segments which overlap over much of their length because they have several *Eco*RI and *Hind*III fragments in common. In addition, the hybridizing portion of chr-1 and one of the hybridizing portions of chr-10 may be the same because the *Hind*III-*Hind*III and *Eco*RI-*Eco*RI fragments, which hybridize with the Hif-2h brobe have the same length (3.2kb and 0.95kb, respectively). It also appears that the "right-hand" hybridizing portion of chr-16 (labelled "r" in Figure 18) may be identical with the hybridizing portion of chr-35, although orientated in the opposite direction, inasmuch as each of the two clones yield a 1.4kb *Bg*/II-*Bg*/II fragment and a 2kb *Eco*RI-*Eco*RI fragment which hybridize with the Hif-2h cDNA probe. Therefore, most likely the chr-16 and chr-35 inserts overlap.

Accordingly, it appears that the 13 hybridizing portions of the 11 hybrid chromosomal DNAs fall into not less than 10 distinct classes-chr-1, chr-3, chr-12, chr-13, chr-16 (left hand, labelled "1" in Figure 18) chr-26, chr-30, chr-35, chr-19 and chr-27.

Referring now to Figure 24, the overlapping of chr-1, chr-3, chr-10 and chr-26 and that of chr-16 and chr-35 are displayed.

The above data suggests that the genome of an individual human contains not less than 10 different DNA sequences that cross-hybridize to Hif-2h. This conclusion is reinforced by the fact that the proportion of fragment Hif-2h-related sequence detected in the clone bank is about 1 in 16,000. Assuming a value of $3 \times 10^9$ bp for the haploid human genome, the expected value for a single gene copy with an average DNA fragment size of 16kb (the average value of the clones examined) is about 1:190,000. Therefore, the frequency of Hif-2h related fragments is 12 times higher than expected for a single gene.

In comparison to these data, when Lawn et al *(supra)* screened 300,000 plaques from the same gene bank with a β-globin cDNA probe, only 2 positive clones were identified-the expected value being 1.6:300,000. Therefore, there may be 10-15 distinct chromosomal DNA segments in the human genome that cross-hybridize to the Hif-2h fragment or IFN-αlcDNA.

## FURTHER CHARACTERIZATION OF Hif-chr35

As an illustration only, the hybridizing sequence of chr-35 ("Hif-chr35") was further characterized. It is to be understood that the hybridizing portions of the previously described chromosomal hybrid phages could similarly be characterized and handled without departing from the scope of this invention.

The hybridizing portion of chr-35 ("Hif-chr35") (and the right-hand segment of Hif-chr16 to which it is most likely identical, *supra)* is the only hybridizing chromosomal DNA portion with a *Bg*/II site. Since IFN-α1 and IFN-α2 cDNA's have 1 and 2 *Bg*/II sites, respectively, within their coding sequences, it seems likely that Hif-chr35 is a counterpart to one of the two previously cloned interferon genes. Hif-chr35's strong hybridization to the 3' terminal Hif-2h cDNA fragment (containing only the 3' non-coding region) compared to the weaker hybridization of the other of the chromosomal DNAs to this probe supports a likely correspondence of Hif-chr35 and Hif-2h (F. Kafatos et al., *Proc. Natl. Acad. Sci USA,* 74, pp. 5618-22 (1977)).

To analyze the Hif-chr35 fragment further, a *Hind*III-*Bam*HI fragment was excised from chr-35. This fragment (3.4kb) contains the hybridizing portion ("Hif-chr35") of chr-35. This fragment was subcloned into the *Pst*I site of pBR322 using the well-known dC-dG tailing procedures (L. Villa-Komaroff et al., *supra)* and *E. coli* HB101 transformed with the resultant recombinant DNA molecule using well-known procedures (e.g., S. Nagata et al., *Nature,* 284, pp, 316-20 (1980)).

Clones of these transformants were screened by in-situ colony hybridization (D. Hanahan and M. Meselson, *Gene,* 10, pp. 63-67 (1980)) with the $^{32}$P-labelled Hif-2h fragment *(supra)* and plasmid DNA-Z-pBr322(Pst)/HchrIF-35HB "HchrIF-35HB"-was the separated from the positive clones (N. M. Wilkie et al., *Nucleic Acids Res.,* 7, pp. 859-77 (1979); Method B). The orientation of the hybrid insert "HchrIF-35HB fragment" in the plasmid with respect to the β-lactamase gene of pBR322 was determined by *Eco*RI cleavage and sizing of the resulting fragments. The insert orientated to coincide with that of β-lactamase was designated α and the opposite orientation β.

Cultures of these positive clones were grown to an apparent $OD_{650} = 0.8$ and the bacteria harvested and lysed by the lysozyme-freeze-thaw method described in S. Nagata et al., *supra.* Seven of the 10 clones examined showed IFN-α activities of 75 to 500 units/g of cells (cytopathic effect reduction assay).

* The shaded area on chr-16 in Figures 18 and 24 represents a sequence which hybridizes weakly to Hif-2h cDNA but did not display R-looping.

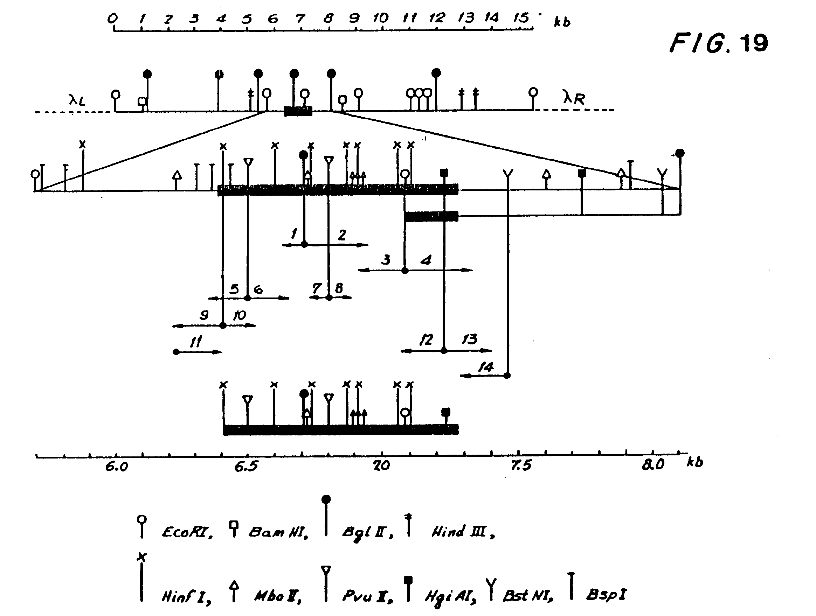

The DNA insert of one of these 7 IFN-producing clones-*E. coli* HB 101 (Z-pBR322(Pst)/HchrlF-35HBα)- was further characterized by restriction analysis and nucleotide sequence determination. Plasmid DNA (HchrlF-35HBα) was prepared from the clone as described previously and the restriction sites determined by Smith-Birnstiel mapping (H. O. Smith and M. L. Birnstiel, *Nucl. Acids Res.,* 3, pp. 2387-98 (1976)): HchrlF-35HBα was digested with *Eco*RI, labelled at the 5' termini and digested with *Bg*/II (and *Pst*I to cleave an undesired fragment of about 1 kb). The 1.04kb *Eco*RI-*Bg*/I (3' proximal) and the 0.96kb *Eco*RI-*Bg*/I (5' proximal) fragments were isolated by agarose gel electrophoresis as described by A. C. Peacock and C. W. Dingman, *Biochemistry,* 6, pp. 1818-27 (1967)). Both fragments were partially cleaved with *Hinf*I, *Bsp*I and *Mbo*II respectively and the products separated on a 1% agarose gel in Tris-acetate buffer (ph 7.8) containing 1 μg/ml ethidium bromide. After staining, the radioactive bands wer visualized by autoradiography. The *Bst*NI and *Hgi*AI sites were similarly determined on the 1.04kb (3' proximal) fragment. The results of the analysis is shown in Figure 19.

For nucleotide sequence determination, Hchrlf-35HBα was cleaved with various restriction enzymes, the products separated by electrophoresis through a 5% polyacrylamide gel in Tris-borate-EDTA buffer (A. C. Peacock and C. W. Dingman, *supra)* and extracted from the gel and purified as described by W. Muller et al., *J. Mol. Biol.,* 124. pp. 343-58 (1978).

The sequencing strategy employed is depicted in Figure 19 and described as follows:

1 and 2-cleavage of HchrlF-35HBα with *Bg*/II, labelling, cleavage with *Eco*RI and *Pst*I and isolation of a *Bg*/II*-*Eco*RI fragment (940bp) ("1") and a *Bg*/II*-*Eco*RI (360bp) ("2");

3 and 4-cleavage of HchrlF-35HBα with *Eco*RI, labelling, cleavage with *Bsp*I and isolation of an *Eco*RI*-*Bsp*I fragment (680bp) ("3") and an *Eco*RI*-*Bsp*I, fragment (880 bp) ("4"):

5,6,7, and 8-cleavage of HchrlF-35HBα with *Pvu*II, labelling, cleavage with *Bg*/II and *Eco*RI and isolation of *Pvu*II*-*Eco*RI fragment (780bp) ("5"), a *Pvu*II*-*Bg*/II (215bp) ("6"). a *Pvu*II*-*Bg*/II fragment (90bp) ("7"), and a *Pvu*II*-*Eco*RI fragment (290bp) ("8");

9 and 10-cleavage of HchrlF-35HBα with *Eco*RI, isolation by 1% agarose gel electrophoresis in Tris-borate EDTA buffer of a 1300 bp *Eco*RI-*Eco*RI fragment, further cleavage with *Hinf*I and isolation of a *Hinf*I-*Hinf*I fragment (450bp), and a *Hinf*I-*Hinf*I fragment (180bp). Labelling, of the larger *Hinf*I-*Hinf*I fragment and cleavage with *Mbo*II permitted isolation of a *Hinf*I*-*Mbo*II (190bp) ("9"). Labelling of the shorter *Hinf*I-*Hinf*I fragment and cleavage with Ava II permitted isolation of a *Hinf*I*-*Ava*II fragment (150bp) ("10");

11-cleavage of HchrlF-35HBα with *Mbo*II, labelling, cleavage with *Bg*/II, and isolation of a *Mbo*II*-*Bg*/II fragment (465bp) ("11");

12, 13 and 14-cleavage of HchrlF-35HBα with *Bsp*I and *Bg*/II, isolation by agarose electrophoresis as above of a 1200bp *Bsp*I-*Bg*/II fragment and (a) cleavage with *Hgi*AI, labelling, cleavage with *Mbo*II and isolation of an *Hgi*AI*-*Mbo*II fragment (300bp) ("12") and an *Hgi*AI*-*Mbo*II fragment (360bp) ("13"), or (b) cleavage with *Bst*NI, labelling, cleavage with *Eco*RI and isolation of a *Bst*NI*-*Eco*RI fragment (380bp) ("14").

The various fragments were sequenced by the Maxam-Gilbert procedure *(supra).* All fragments were sequenced on both strands and across the restriction sites that served as origins for sequencing.

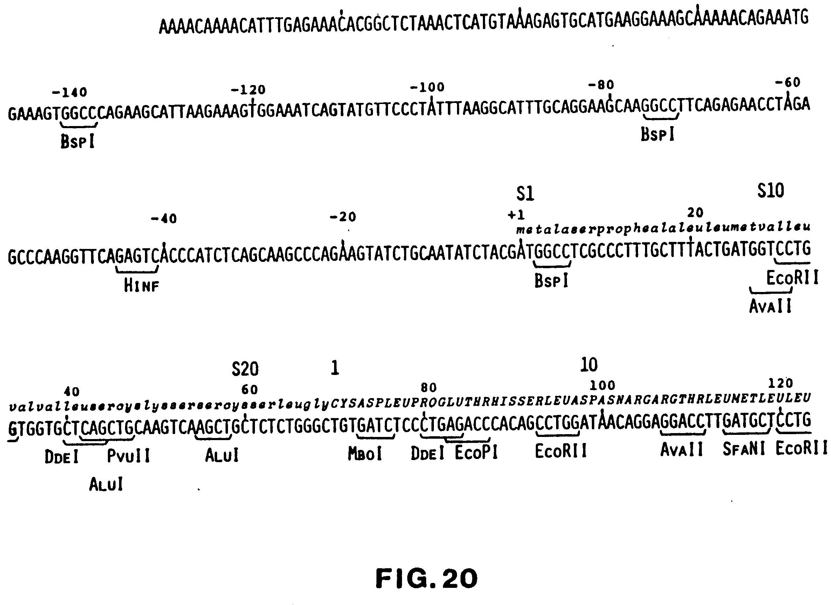

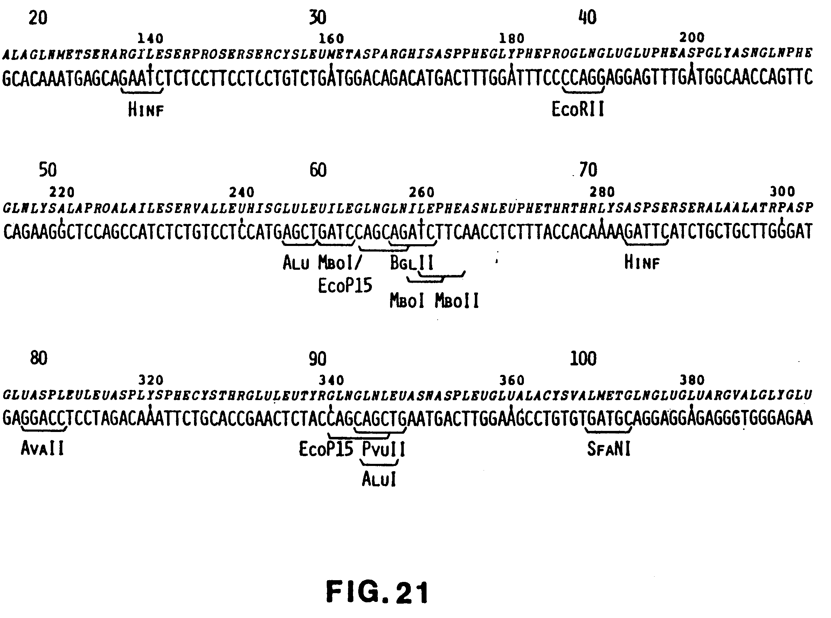

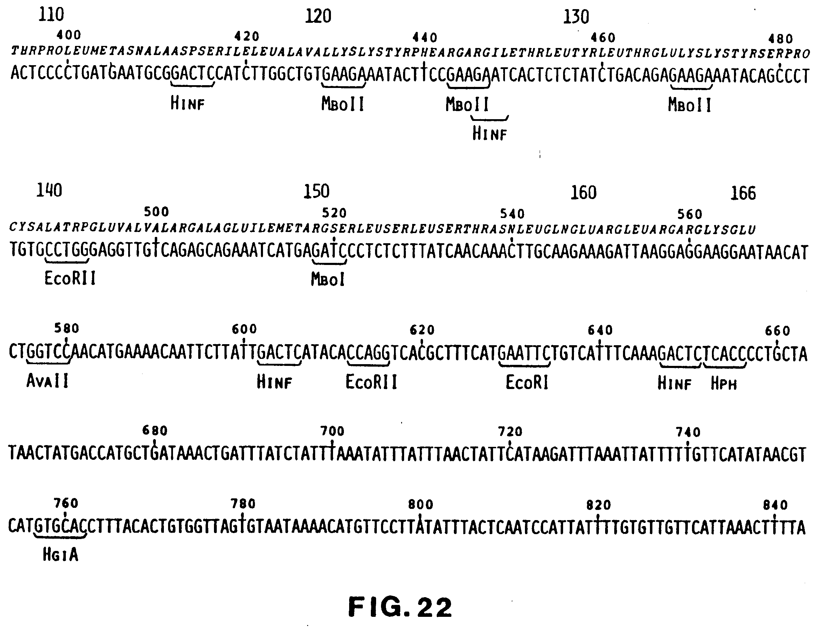

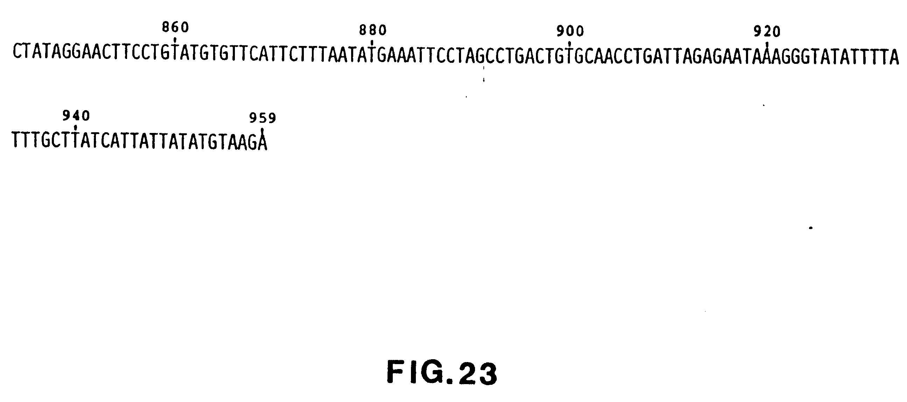

A comparison of the nucleotide sequence of the coding region of HchrlF-35HBα and that of Hif-2h (coding region) Figures 8-10 compared to Figures 20-23) reveals that they are identical. In particular, it is surprising that there is no indication of the presence of introns within the coding sequence of the HchrlF-35HBα fragment, i.e., between the *Hinf*I site in the 5' non-coding region and the *Eco*RI site in the 3' non-coding region. Thus, no intron could be detected in the chromosomal sequence corresponding to mature IFN-αmRNA.

## FURTHER CHARACTERIZATION OF Hif-chr 26 AND Hif-chr 3

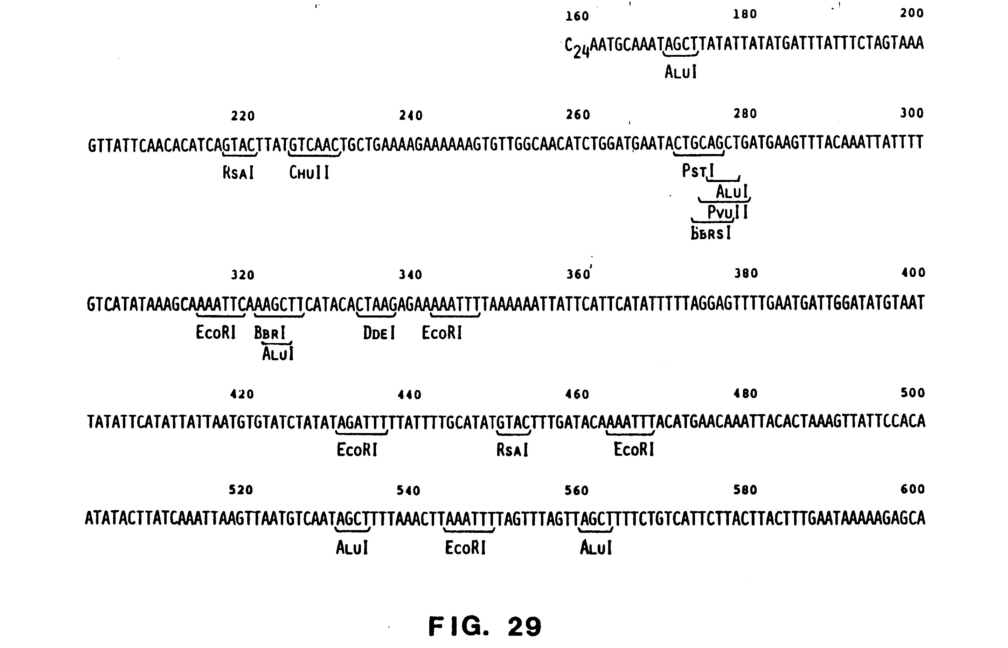

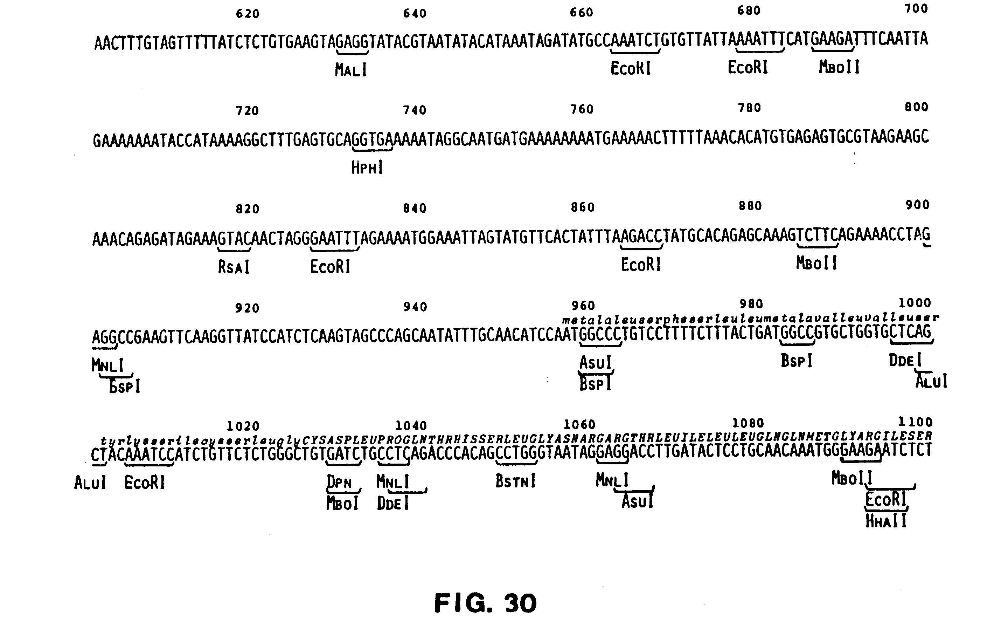

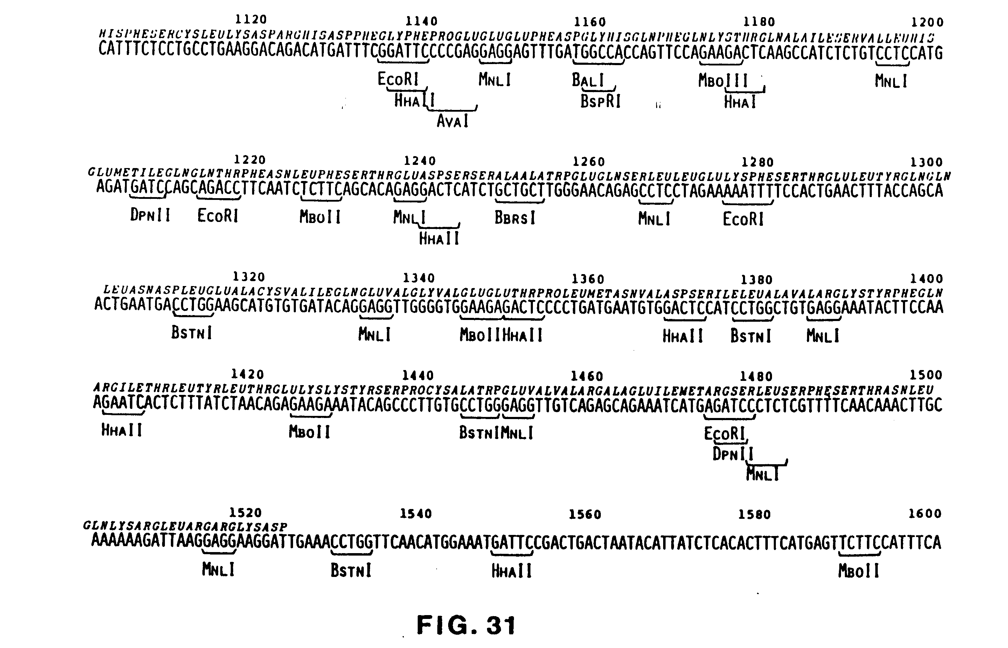

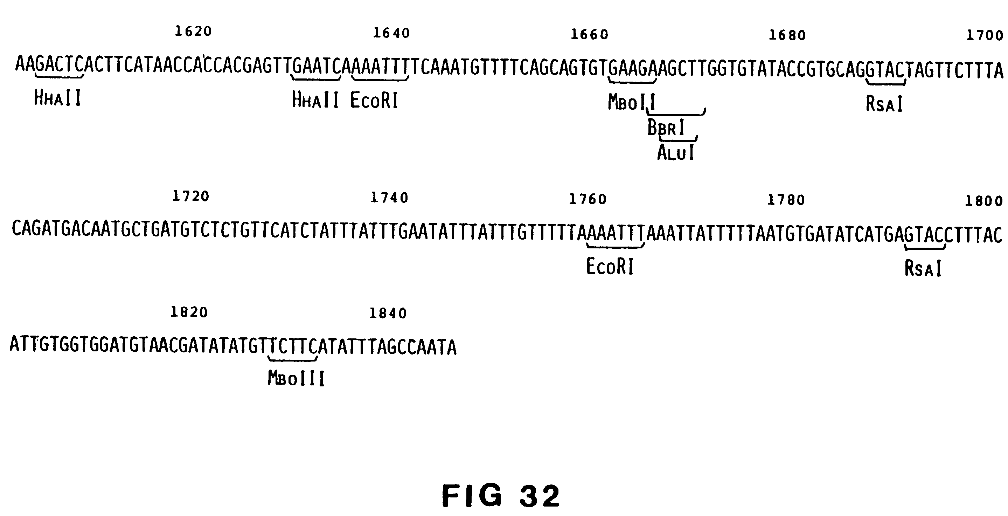

The gene-containing segments of chr-3 and chr-26, which appear identical by heteroduplex analysis but differ in at least one *Bg*/II restriction site were examined by nucleotide sequencing. Five nucleotide differences in 725 base pairs were founds. Only two of these appear in the coding sequences. Since not only the genes, but at least 3.5 Kbp preceding and 6.0 Kbp following them formed a perfect heteroduplex and because of the relatively low sequence divergence which entails only 2 amino acid changes, it appears that Hif-chr3 and Hif-chr26 are allenlic forms of the same gene. These are designated IFN-α4a (Hif-chr3) and IFN-α4b (Hif-chr26). The nucleotide sequence and corresponding amino acid sequence of IFN-α4b determined by conventional sequencing techniques described previously is displayed in Figures 29-32.

A comparison of Figures 29-32 with Figures 8-10, 12-16 and 20-23 reveals that the proteins encoded by each of the sequences differ from each other in about 15% of their residues. This divergence is typical for products of non-allelic genes which have diverged 20-90 million years ago.

Expression Of Hif-chr35 In Mouse Cells

Plasmid Z-pBR322(Pst)/HchrlF-35HBα *(supra)* was used as a source of a Hif-chr35 fragment for expression in mouse cells. The plasmid was restricted with *Pst*I and treated with 5' exonuclease to remove the 5' dG tails. This fragment was then inserted into a 5' dG-tailed *Kpn*I fragment of a plasmid prepared by joining the *Bam*HI-*Bam*HI fragments of pBR322 and polynoma DNA. The resulting vector was used to transform mouse 3T3 cells using the calcium phosphate technique (N. Mantel *et al., Nature,* 281 pp. 40-46 (1979)). These transformed cells are designated for convenience Mouse 3T3 (polynoma-Hif-chr35). After 20-40 hours, assays revealed an IFN-α activity of 300 units/ml of IFN-α on human cells and about 3000 units/ml of IFN-α on bovine cells.

It should of course be understood that the nucleotide sequences depicted in Figures 8-10, 12-16, 20-23 and 29-32 do not take into account any modifications to the nucleotide sequences such as mutation, including single or multiple, base substitutions, insertions, inversions or deletions which may have already taken place or which may subsequently be employed. Moreover, the sequence also does not take into account the possible substitution of other codons coding for the same amino acid as a codon depicted in these figures. Therefore, it should be understood that such modified sequences as code for polypeptides displaying an immunological or biological activity of IFN-α are also within this invention.

In addition, it is to be understood that the amino acid sequences depited in Figures 8-10, 12-16, 20-23 and 29-32 do not take into account any modifications to the polypeptides caused by their interaction with *in vivo* or *in vitro* agents e.g. *in vivo* glycosolation enzymes. Therefore, it must be understood that fragments and derivatives of these polypeptides that display an immunological or biological activity of IFN-α are also part of this invention.

PRODUCTION OF POLYPEPTIDES DISPLAYING AN IMMUNOLOGICAL OR BIOLOGICAL ACTIVITY OF INTERFERON IN BACTERIAL HOSTS

Since the cytopathic effort reduction assay (W. E. Stewart II and S. E. Sulkin,*S. E. Proc. Soc. Exp. Biol. Med..* 123, pp. 650-53 (1966)) can detect minute amounts of IFN-less than one active molecule per bacterial cell-lysates of *E. coli* HB101 infected with the ten hybrid A phages, described previously, were assayed for the presence of IFN. Seven of the eleven phages (all except chr-10. chr-12. chr-19 and chr-27) gave lysates containing IFN activity ranging from 3 to 50 units/ml. In the case of chr-10 and chr-12, the hybridizing (to Hif-2h) *Hind*III-*Hind*III or *Eco*RI-*Eco*RI fragments, subcloned into the *Pst*I site of pBR322. as described previously, expressed IFN-α activity in *E. coli.* Since *E. coli* is believed to be incapable of splicing mRNA (O. Mercereau-Puijalon and P. Kourilsky, *Nature,* 279, pp. 647-49 (1979)), these IFN-α chromosomal genes most likely do not contain introns in their coding region.

FINAL CONCLUSIONS

We have isolated a set of recombinant DNA molecules containing cDNA prepared from poly(A) RNA from Sendai-virus-treated (induced) human leukocytes, representatives of which have the following properties:
(1) They hybridize to poly(A) RNA from induced but not from non-induced human leukocytes.
(2) They hybridize to IFN-αmRNA as shown by their capacity to select this RNA from a mixture of RNAs. and by their capacity to inhibit (reversibly) translation of interfon mRNA in the hybrid arrested translation assay.
(3) *E. coli* containing certain members of the set product a compound with the following properties:
(a) It is sensitive to trypsin
(b) It exhibits IFN-α activity in a human cell system and only slight activity in a mouse cell system
(c) It has a molecular weight between 20,000 and 30,000 (19,388 based on the nucleotide sequencing of Figs. 8-10
(d) The iFN-α activity is specifically inhibited by antibody to human leukocyte interferon.
(4) The DNA inserts of the hybrid plasmids of this invention are able. in addition to their ability to select IFN-αmRNA from a mixture of RNAs, to select IFN-αDNA from mixtures of various sources including cDNAs and from hybrid phage gene banks of chromosomal DNA.
(5) A number of different chromosomal genes for IFN-α exist. It is unexpected that these genes lack introns and permit direct expression of interferon and interferon-like polypeptides in appropriate hosts.
(6) At least three of the nucleotide sequences of the DNA inserts of these recombinant DNA molecules are different and suggest the existence of at least three non-allelic genes for IFN-α.
(7) The proteins coded for by these three nucleotide sequences are different from the 35 amino acids determined from authentic lymphoblastoid interferon.

(8) Hybrid proteins prepared for various combinations of IFN-α gene segments display quantitatively different properties than each other or their parents and proteins having additional amino acids fused to IFN-α or proteins comprising IFN-α without a portion of its amino terminal sequence display IFN activity.

These properties demonstrate that the recombinant DNA molecules described by this invention contain at least a part of the coding sequence for human leukocyte interferon and that some of these plasmids lead to expression in *E. coli* of a polypeptide with an immunological or biological activity of human leukocyte interferon. It should also be evident that the polypeptides disclosed herein may be fragmented, modified or derivatized, as is well known in the protein art, without departing from the scope or disclosure of this invention.

Micro-organisms and recombinant DNA molecules prepared by the processes described herein are exemplified by cultures deposited in the culture collection Deutsche Sammlung von Mikroorganismen, in Gottingen, West Germany on January 7, 1980. and identified as HclF-A to E:

A: *E. coli* HB101 (Z-pBR322(Pst)/HclF-4c)
B: *E. coli* HB101 (Z-pBR322(Pst)/HclF-2h)
C: *E. coli* HB101 (Z-pBR322(Pst)/HclF-SN35)
D: *E. coli* HB101 (Z-pBR322(Pst)/HclF-SN42)
E: *E. coli* HB101 (Z-pKT287(Pst)/HclF-2h-AH6)

These cultures were assigned accession numbers DSM 1699-1703, respectively.

In addition, micro-organisms and recombinant DNA molecules prepared by the processes described herein are exemplified by cultures deposited in the culture collection of the American Type Culture Collection, Rockville, Maryland on March 27, 1980 and identified as HclF-G to H, and assigned ATCC accession numbers 31633 and 31634 respectively:

G: *E. coli* HB101 (Z-pBR322(Pst)/HclF-II-206)
H. *E. coli* HB101 (Z-pBR322(Pst)/HclF-SN35-AHL6)

Other micro-organisms prepared by the processes described herein are exemplified by cultures deposited in the culture collection Deutsche Sammlung von Mikroorganismem, in Gottingen, West Germany on October 1, 1980 and identified as HchrIF-A through J, and assigned accession numbers DSM 1941-1923:

A. subcloned *Hind*III fragment of chr 3 in *E. coli* HB101;
B. subcloned *Eco*RI fragment of chr 12 in *E. coli* HB101;
C. subcloned *Hind*III fragment of chr 12 in *E. coli* HB101;
D. subcloned *Eco*RI fragment of chr 13 in *E. coli* HB101;
E. subcloned *Eco*RI fragment of chr 23 in *E. coli* HB101;
F. subcloned *Hind*III fragment of chr 23 in *E. coli* HB101;
G. subcloned *Eco*RI fragment of chr 26 in *E. coli* HB101;
H. subcloned *Hind*III fragment of chr 26 in *E. coli* HB101;
I. subcloned *Hind*III/*Bam*HI fragment of chr 35 in *E. coli* HB101;
J. subcloned *Bam*HI fragment of chr 35 in *E. coli* HB101;

Finally, micro-organisms prepared by the processes described herein are exemplified by cultures deposited in the American Type Culture Collection, Rockville, Maryland, on December 15, 1980 and identified as HchrIF-K through HchrIF-Q and HclF-I through HclF-K, and assigned ATTC accession numbers as follows:

31760 HchrIF-K
31761 HchrIF-L
31762 HchrIF-M
31763 HchrIF-N
31764 HchrIF-Q
31765 HchrIF-P
31766 HchrIF-Q
31767 HclF-I
31768 HclF-J
31769 HclF-K

K. subcloned *Tac-Tac* fragment of chr 23 in *E. coli* HB101.
L. subcloned *Bg*/II-*Bg*/II fragment of chr 101 in *E. coli* HB101.
M. subcloned *Hind*III-*Hind*III fragment of chr 10r in *E. coli* HB101.
N. subcloned *Bg*/II-*Bg*/II fragment of chr 26 in *E. coli* HB101.
O. subcloned *Hind*III-*Hind*III fragment of chr 30 in *E. coli* HB101.
P. subcloned *Bg*/I-*Tac* fragment of chr 13 in *E. coli* HB101.

Q. subcloned *Bg/*II-Tac fragment of chr 16 I in *E. coli* HB101.

HcIF-I: *E. coli* DS410 (C8-IFN-α2)

HcIF-J: *E. coli* DS410 (LAC-AUG(α2))

HcIF-K: *E. coli* DS410 (β-Lac-AUG(α2))

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto rather than the specific embodiments which have been presented hereinbefore by way of example.

**Claims**

**Claims for the following Contracting States : BE, CH, DE, FR, GB, IT, LI, LU, NL, SE**

1. A recombinant DNA molecule for use in cloning a DNA sequence in bacteria, yeasts or animal cells, said recombinant DNA molecule comprising a DNA sequence selected from:

   (a) the DNA inserts of Z-pBR322(Pst)/HcIF-4c, Z-pBR322(Pst)/HcIF-2h, Z- pBR322(Pst)/HcIF-SN35, Z-pBR322(Pst)/HcIF-SN42 and Z- pKT287 (Pst)/HcIF-2h-AH6, said DNA inserts being the DNA inserts of the recombinant DNA molecules carried by the microorganisms identified by accession numbers DSM 1699-1703, respectively,

   (b) DNA sequences which hybridize to any of the foregoing DNA inserts and which code for a polypeptide of the IFN-α type, and

   (c) DNA sequences which are degenerate as a result of the genetic code to the DNA sequences and inserts defined in (a) and (b) and which code for a polypeptide of the IFN-α type.

2. A recombinant DNA molecule according to claim 1, wherein said DNA sequence (b) which hybridizes to said DNA insert (a) is selected from:

   (d) the DNA inserts of Z-pBR322(Pst)/HcIF-II-206 and Z-pBR322(Pst)/Hclf-SN35- AHL6, said DNA inserts being the DNA inserts of the recombinant DNA molecules carried by the microorganisms identified by accession numbers ATCC 31633-31634, respectively,

   (e) DNA sequences which hybridize to any or the foregoing DNA inserts and which code for a polypeptide of the IFN-α, type, and

   (f) DNA sequences which are degenerate as a result of the genetic code to the DNA sequences and inserts defined in (d) and (e) and which code for a polypeptide of the IFN-α type.

3. A recombinant DNA molecule according to claim 1 or 2, wherein said DNA sequence (b) or (e) which hybridizes to said DNA insert (a) or (d) is selected from:

   (g) the hybridizing portion of each of: HchrIF-A, the subcloned <u>Hind</u>III fragment of chr 3 in <u>E.coli</u> HB101 (DSM 1914); HchrIF-B, the subcloned <u>EcoRI</u> fragment of chr 12 in <u>E.coli</u> HB101 (DSM 1915); HchrIF-C, the subcloned <u>Hind</u>III fragment of chr 12 in <u>E.coli</u> HB101 (DSM 1916); HchrIF-D, the subcloned <u>EcoRI</u> fragment of chr 13 in <u>E.coli</u> HB101 (DSM 1917); HchrIF-E, the subcloned <u>EcoRI</u> fragment of chr 23 in <u>E.coli</u> HB101 (DSM 1918); HchrIF-F, the subcloned <u>Hind</u>III fragment of chr 23 in <u>E.coli</u> HB101 (DSM 1919); HchrIF-G, the subcloned <u>EcoRI</u> fragment of chr 26 in <u>E.coli</u> HB101 (DSM 1920); and Hchrlf-H, the subcloned <u>Hind</u>III fragment of chr 26 in <u>E.coli</u> HB101 (DSM 1921).

4. A recombinant DNA molecule according to claim 1, wherein said DNA sequence is selected from DNA sequences of the formula:

ATGGCCTCGCCCTTTG

CTTTACTGATGGTCCTGGTGGTGCTCAGCTGCAAGTCAAGCTGCTCTCTGGG

CTGTGATCTCCCTGAGACCCACAGCCTGGATAACAGGAGGACCTTGATGCTC

CTGGCACAAATGAGCAGAATCTCTCCTTCCTCCTGTCTGATGGACAGACATG

ACTTTGGATTTCCCCAGGAGGAGTTTGATGGCAACCAGTTCCAGAAGGCTCC

AGCCATCTCTGTCCTCCATGAGCTGATCCAGCAGATCTTCAACCTCTTTACC

ACAAAAGATTCATCTGCTGCTTGGGATGAGGACCTCCTAGACAAATTCTGCA
CCGAACTCTACCAGCAGCTGAATGACTTGGAAGCCTGTGTGATGCAGGAGGA
GAGGGTGGGAGAAACTCCCCTGATGAATGCGGACTCCATCTTGGCTGTGAAG
AAATACTTCCGAAGAATCACTCTCTATCTGACAGAGAAGAAATACAGCCCTT
GTGCCTGGGAGGTTGTCAGAGCAGAAATCATGAGATCCCTCTCTTTATCAAC
AAACTTGCAAGAAAGATTAAGGAGGAAGGAA and TGTGATCTCCCTGAGA
CCCACAGCCTGGATAACAGGAGGACCTTGATGCTCCTGGCACAAATGAGCAG
AATCTCTCCTTCCTCCTGTCTGATGGACAGACATGACTTTGGATTTCCCCAG
GAGGAGTTTGATGGCAACCAGTTCCAGAAGGCTCCAGCCATCTCTGTCCTCC
ATGAGCTGATCCAGCAGATCTTCAACCTCTTTACCACAAAAGATTCATCTGC
TGCTTGGGATGAGGACCTCCTAGACAAATTCTGCACCGAACTCTACCAGCAG
CTGAATGACTTGGAAGCCTGTGTGATGCAGGAGGAGAGGGTGGGAGAAACTC
CCCTGATGAATGCGGACTCCATCTTGGCTGTGAAGAAATACTTCCGAAGAAT
CACTCTCTATCTGACAGAGAAGAAATACAGCCCTTGTGCCTGGGAGGTTGTC
AGAGCAGAAATCATGAGATCCCTCTCTTTATCAACAAACTTGCAAGAAAGAT
TAAGGAGGAAGGAA.

5. A recombinant DNA molecule according to claim 3, wherein said DNA sequence is selected from DNA sequences of the formula:

                    ATGGCCCTGTCCTTTT
CTTTACTGATGGCCGTGCTGGTGCTCAGCTACAAATCCATCTGTTCTCTGGG
CTGTGATCTGCCTCAGACCCACAGCCTGGGTAATAGGAGGACCTTGATACTC
CTGCAACAAATGGGAAGAATCTCTCATTTCTCCTGCCTGAAGGACAGACATG
ATTTCGGATTCCCCGAGGAGGAGTTTGATGGCCACCAGTTCCAGAAGACTCA
AGCCATCTCTGTCCTCCATGAGATGATCCAGCAGACCTTCAATCTCTTCAGCA
CAGAGGACTCATCTGCTGCTTGGGAACAGAGCCTCCTAGAAAAATTTTCCACT
GAACTTTACCAGCAACTGAATGACCTGGAAGCATGTGTGATACAGGAGGTTGG
GGTGGAAGAGACTCCCCTGATGAATGTGGACTCCATCCTGGCTGTGAGGAAAT
ACTTCCAAAGAATCACTCTTTATCTAACAGAGAAGAAATACAGCCCTTGTGCC
TGGGAGGTTGTCAGAGCAGAAATCATGAGATCCCTCTCGTTTTCAACAAACTT
GCAAAAAAGATTAAGGAGGAAGGAT and TGTGATCTGCCTCAGACCCACAG
CCTGGGTAATAGGAGGACCTTGATACTCCTGCAACAAATGGGAAGAATCTCTC
ATTTCTCCTGCCTGAAGGACAGACATGATTTCGGATTCCCCGAGGAGGAGTTT
GATGGCCACCAGTTCCAGAAGACTCAAGCCATCTCTGTCCTCCATGAGATGAT
CCAGCAGACCTTCAATCTCTTCAGCACAGAGGACTCATCTGCTGCTTGGGAAC

AGAGCCTCCTAGAAAAATTTTCCACTGAACTTTACCAGCAACTGAATGACCTG
GAAGCATGTGTGATACAGGAGGTTGGGGTGGAAGAGACTCCCCTGATGAATGT
GGACTCCATCCTGGCTGTGAGGAAATACTTCCAAAGAATCACTCTTTATCTAA
CAGAGAAGAAATACAGCCCTTGTGCCTGGGAGGTTGTCAGAGCAGAAATCATG
AGATCCCTCTCGTTTTCAACAAACTTGCAAAAAAGATTAAGGAGGAAGGAT.

6. A recombinant DNA molecule according to any one of claims 1 to 5 for use in expressing said cloned DNA sequence in bacteria, yeasts or animal cells, characterized in that said DNA sequence is operatively linked to an expression control sequence in said recombinant DNA molecule.

7. A recombinant DNA molecule according to claim 6, wherein said expression control sequence is selected from a lac system, a β-lac system, a trp system, major operator and promotor regions of phage λ, the control region of fd coat protein, and other sequences which control the expression of genes in bacteria, yeasts or animal cells.

8. A recombinant DNA molecule according to claim 6 or 7 selected from C8-IFN-α2, LAC-AUG(α2) and β-lac-AUG(α2), said recombinant DNA molecules being the recombinant DNA molecules carried by the microorganisms identified by accession numbers ATCC 31767- 31769.

9. A host transformed with at least one recombinant DNA molecule according to any one of claims 1 to 8, said host being selected from bacteria, yeasts and animal cells.

10. The host according to claim 9, wherein said bacteria, yeasts on animal cells are selected from strains of E.coli, Pseudomonas, Bacillus subtilis, Bacillus stearothermophilus, other bacilli and mouse cells.

11. The transformed host according to claim 10, selected from E.coli HB101 (Z-pBR322 (Pst)/HcIF-4c)[DSM 1699], E.coli HB101 (Z-pBR322 (Pst)/HcIF-2h)[DSM 1700], E.coli HB101 (Z-pBR322 (Pst)/HcIF-SN35)[DSM 1701], E.coli HB101 (Z-pBR322 (Pst)/HcIF-SN42)[DSM 1702] and E.coli HB101 (Z-pKT287(Pst)/HcIF-2h-AH6)[DSM 1703].

12. The transformed host according to claim 10, selected from E.coli HB101 (Z-pBR322 (Pst)/HcIF-II-206)[ATCC 31633] and E.coli HB101 (Z- pBR322(Pst)/HcIF-SN35-AHL6)[ATCC 31634].

13. The transformed host according to claim 10, selected from HchrIF-A [DSM 1914], HchrIF-B[DSM 1915], HchrIF-C[DSM 1916], HchrIF-D[DSM 1917], HchrIF-E[DSM 1918], HchrIF-F[DSM 1919], HchrIF-G[DSM 1920] and HchrIF-H[DSM 1921].

14. The transformed host according to claim 10, selected from E.coli DS410 (C8-IFN-α2) [ATCC 31767], E.coli DS410 (LAC-AUG(α2))[ATCC 31768], and E.coli DS410 HB101 (β-lac-AUG(α2))[ATCC 31769].

15. A process for producing a polypeptide of the IFN-α type which comprises culturing a transformed host according to any one of claims 9 to 14, insofar as those claims depend from claims 6 to 8 and collecting said polypeptide.

16. A polypeptide of the IFN-α type produced according to the process of claim 15, selected from polypeptides of the formula:

44

## METALASER

PROPHEALALEULEUMETVALLEUVALVALLEUSERCYSLYSSERSERCYSSER
LEUGLYCYSASPLEUPROGLUTHRHISSERLEUASPASNARGARGTHRLEUMET
LEULEUALAGLNMETSERARGILESERPROSERSERCYSLEUMETASPARGHIS
ASPPHEGLYPHEPROGLNGLUGLUPHEASPGLYASNGLNPHEGLNLYSALAPRO
ALAILESERVALLEUHISGLULEUILEGLNGLNILEPHEASNLEUPHETHRTHR
LYSASPSERSERALAALAATRPASPGLUASPLEULEUASPLYSPHECYSTHRGLU
LEUTYRGLNGLNLEUASNASPLEUGLUALACYSVALMETGLNGLUGLUARGVAL
GLYGLUTHRPROLEUMETASNALAASPSERILELEUALAVALLYSLYSTYRPHE
ARGARGILETHRLEUTYRLEUTHRGLULYSLYSTYRSERPROCYSALATRPGLU
VALVALARGALAGLUILEMETARGSERLEUSERLEUSERTHRASNLEUGLNGLU
ARGLEUARGARGLYSGLU and CYSASPLEUPROGLUTHRHISSERLEUASPA
SNARGARGTHRLEUMETLEULEUALAGLNMETSERARGILESERPROSERSERC
YSLEUMETASPARGHISASPPHEGLYPHEPROGLNGLUGLUPHEASPGLYASNG
LNPHEGLNLYSALAPROALAILESERVALLEUHISGLULEUILEGLNGLNILEP
HEASNLEUPHETHRTHRLYSASPSERSERALAALAATRPASPGLUASPLEULEUA
SPLYSPHECYSTHRGLULEUTYRGLNGLNLEUASNASPLEUGLUALACYSVALM
ETGLNGLUGLUARGVALGLYGLUTHRPROLEUMETASNALAASPSERILELEUA
LAVALLYSLYSTYRPHEARGARGILETHRLEUTYRLEUTHRGLULYSLYSTYRS
ERPROCYSALATRPGLUVALVALARGALAGLUILEMETARGSERLEUSERLEUS
ERTHRASNLEUGLNGLUARGLEUARGARGLYSGLU.

17. A polypeptide of the IFN-α type produced according to the process of claim 15, selected from polypeptides of the formula:

## METALA

LEUSERPHESERLEULEUMETALAVALLEUVALLEUSERTYRLYSSERILE
CYSSERLEUGLYCYSASPLEUPROGLNTHRHISSERLEUGLYASNARGARG
THRLEUILELEULEULEUGLNGLNMETGLYARGILESERHISPHESERCYSLEU
LYSASPARGHISASPPHEGLYPHEPROGLUGLUGLUPHEASPGLYHISGLN
PHEGLNLYSTHRGLNALAILESERVALLEUHISGLUMETILEGLNGLNTHR
PHEASNLEUPHESERTHRGLUASPSERSERALAALAATRPGLUGLNSERLEU
LEUGLULYSPHESERTHRGLULEUTYRGLNGLNLEUASNASPLEUGLUALA
CYSVALILEGLNGLUVALGLYVALGLUGLUTHRPROLEUMETASNVALALASP

SERILELEUALAVALARGLYSTYRPHEGLNARGILETHRLEUTYRLEUTHR

GLULYSLYSTYRSERPROCYSALATRPGLUVALVALARGALAGLUILEMET

ARGSERLEUSERPHESERTHRASNLEUGLNLYSARGLEUARGARGLYSASP

and CYSASPLEUPROGLNTHRHISSERLEUGLYASNARGARG

THRLEUILELEULEUGLNGLNMETGLYARGILESERHISPHESERCYSLEU

LYSASPARGHISASPPHEGLYPHEPROGLUGLUGLUPHEASPGLYHISGLN

PHEGLNLYSTHRGLNALAILESERVALLEUHISGLUMETILEGLNGLNTHR

PHEASNLEUPHESERTHRGLUASPSERSERALAALATRPGLUGLNSERLEU

LEUGLULYSPHESERTHRGLULEUTYRGLNGLNLEUASNASPLEUGLUALA

CYSVALILEGLNGLUVALGLYVALGLUGLUTHRPROLEUMETASNVALALASP

SERILELEUALAVALARGLYSTYRPHEGLNARGILETHRLEUTYRLEUTHR

GLULYSLYSTYRSERPROCYSALATRPGLUVALVALARGALAGLUILEMET

ARGSERLEUSERPHESERTHRASNLEUGLNLYSARGLEUARGARGLYSASP.

**18.** A method for producing a recombinant DNA molecule according to any one of claims 1 to 5, comprising the step of introducing into a cloning vehicle a DNA sequence that characterizes the recombinant DNA molecules of any of claims 1 to 5.

**19.** A method for producing a recombinant DNA molecule according to any one of claims 6 to 8, comprising the steps of introducing into a cloning vehicle a DNA sequence that characterizes the recombinant DNA molecules of any of claims 1 to 5 and introducing into said cloning vehicle an expression control sequence according to claim 7, said espression control sequence being introduced into said cloning vehicle so as to control and to regulate the expression of said DNA sequence.

**20.** A method for transforming a host selected from bacteria, yeasts or animal cells, comprising the step of introducing into said host a recombinant DNA molecule according to any one of claims 1 to 8.

**21.** A method for producing a polypeptide of the IFN-α type comprising the steps of transforming a host selected from bacteria, yeasts or animal cells, with a recombinant DNA molecule according to any of claims 6 to 8; culturing said host; and collecting said polypeptide.

**22.** The method according to claim 21, wherein said host is selected from strains of <u>E.coli</u>, <u>Pseudomonas</u>, <u>Bacillus</u> <u>subtilis</u>, <u>Bacillus</u> <u>stearothermophilus</u>, and other bacilli.

**23.** A method for producing a polypeptide of the IFN-α type comprising the steps of culturing a host selected from bacteria, yeasts or animal cells and transformed by a recombinant DNA molecule according to any of claims 6 to 8 and collecting said polypeptide.

**24.** A process for selecting a DNA sequence coding for a polypeptide of the IFN-α type from a group of DNA sequences comprising the step of determining which of said DNA sequences hybridize to a DNA sequence that characterizes the recombinant DNA molecules according to any one of claims 1 to 8.

**25.** The process according to claim 24, wherein said DNA sequence screened is selected from DNA sequences from natural sources, synthetic DNA sequences, DNA sequences from recombinant DNA molecules and DNA sequences which are a combination of any of the foregoing DNA sequences.

**26.** A composition for treating human viruses or treating human cancers or tumors which comprises as a sole IFN-α a polypeptide selected from a polypeptide according to any one of claims 16 to 17 or produced according to the process of any one of claims 15, 21, 22 and 23.

46

27. A composition for treating bovine viral infections which comprises as a sole IFN-α a polypeptide selected from a polypeptide according to any one of claims 16 to 17 or produced according to the process of any one of claims 15, 21, 22, and 23.

28. A DNA sequence selected from DNA sequences of the formula:

ATGGCCTCGCCCTTTGCTTTACTGA
TGGTCCTGGTGGTGCTCAGCTGCAAGTCAAGCTGCTCTCTGGGCTGTGATCT
CCCTGAGACCCACAGCCTGGATAACAGGAGGACCTTGATGCTCCTGGCACAA
ATGAGCAGAATCTCTCCTTCCTCCTGTCTGATGGACAGACATGACTTTGGAT
TTCCCCAGGAGGAGTTTGATGGCAACCAGTTCCAGAAGGCTCCAGCCATCTC
TGTCCTCCATGAGCTGATCCAGCAGATCTTCAACCTCTTTACCACAAAAGAT
TCATCTGCTGCTTGGGATGAGGACCTCCTAGACAAATTCTGCACCGAACTCT
ACCAGCAGCTGAATGACTTGGAAGCCTGTGTGATGCAGGAGGAGAGGGTGGG
AGAAACTCCCCTGATGAATGCGGACTCCATCTTGGCTGTGAAGAAATACTTC
CGAAGAATCACTCTCTATCTGACAGAGAAGAAATACAGCCCTTGTGCCTGGG
AGGTTGTCAGAGCAGAAATCATGAGATCCCTCTCTTTATCAACAAACTTGCA
AGAAAGATTAAGGAGGAAGGAA and TGTGATCTCCCTGAGACCCACAGCC
TGGATAACAGGAGGACCTTGATGCTCCTGGCACAAATGAGCAGAATCTCTCC
TTCCTCCTGTCTGATGGACAGACATGACTTTGGATTTCCCCAGGAGGAGTTT
GATGGCAACCAGTTCCAGAAGGCTCCAGCCATCTCTGTCCTCCATGAGCTGA
TCCAGCAGATCTTCAACCTCTTTACCACAAAAGATTCATCTGCTGCTTGGGA
TGAGGACCTCCTAGACAAATTCTGCACCGAACTCTACCAGCAGCTGAATGAC
TTGGAAGCCTGTGTGATGCAGGAGGAGAGGGTGGGAGAAACTCCCCTGATGA
ATGCGGACTCCATCTTGGCTGTGAAGAAATACTTCCGAAGAATCACTCTCTA
TCTGACAGAGAAGAAATACAGCCCTTGTGCCTGGGAGGTTGTCAGAGCAGAA
ATCATGAGATCCCTCTCTTTATCAACAAACTTGCAAGAAAGATTAAGGAGGA

AGGAA, said DNA sequence being for use in cloning or expressing said sequence in bacteria, yeasts or animal cells and in selecting DNA sequences that code for a polypeptide of the IFN-α type.

29. A DNA sequence selected from DNA sequences of the formula:

47

ATGGCCCTGTCCTTTTCTTTACTGA

TGGCCGTGCTGGTGCTCAGCTACAAATCCATCTGTTCTCTGGGCTGTGATCT

GCCTCAGACCCACAGCCTGGGTAATAGGAGGACCTTGATACTCCTGCAACAA

ATGGGAAGAATCTCTCATTTCTCCTGCCTGAAGGACAGACATGATTTCGGAT

TCCCCGAGGAGGAGTTTGATGGCCACCAGTTCCAGAAGACTCAAGCCATCTC

TGTCCTCCATGAGATGATCCAGCAGACCTTCAATCTCTTCAGCACAGAGGAC

TCATCTGCTGCTTGGGAACAGAGCCTCCTAGAAAAATTTTCCACTGAACTTT

ACCAGCAACTGAATGACCTGGAAGCATGTGTGATACAGGAGGTTGGGGTGGA

AGAGACTCCCCTGATGAATGTGGACTCCATCCTGGCTGTGAGGAAATACTTC

CAAAGAATCACTCTTTATCTAACA  GAGAAGAAATACAGCCCTTGTGCCTG

GGAGGTTGTCAGAGCAGAAATCATGAGATCCCTCTCGTTTTCAACAAACTTG

CAAAAAAGATTAAGGAGGAAGGAT and TGTGATCTGCCTCAGACCCACAG

CCTGGGTAATAGGAGGACCTTGATACTCCTGCAACAAATGGGAAGAATCTCT

CATTTCTCCTGCCTGAAGGACAGACATGATTTCGGATTCCCCGAGGAGGAGT

TTGATGGCCACCAGTTCCAGAAGACTCAAGCCATCTCTGTCCTCCATGAGAT

GATCCAGCAGACCTTCAATCTCTTCAGCACAGAGGACTCATCTGCTGCTTGG

GAACAGAGCCTCCTAGAAAAATTTTCCACTGAACTTTACCAGCAACTGAATG

ACCTGGAAGCATGTGTGATACAGGAGGTTGGGGTGGAAGAGACTCCCCTGAT

GAATGTGGACTCCATCCTGGCTGTGAGGAAATACTTCCAAAGAATCACTCTT

TATCTAACAGAGAAGAAATACAGCCCTTGTGCCTGGGAGGTTGTCAGAGCAG

AAATCATGAGATCCCTCTCGTTTTCAACAAACTTGCAAAAAAGATTAAGGAG

GAAGGAT,

said DNA sequence being for use in cloning or expressing said sequence in bacteria, yeasts or animal cells and in selecting DNA sequences that code for a polypeptide of the IFN-α type.

**Claims for the following Contracting State : AT**

1. A method for producing a recombinant DNA molecule for use in cloning a DNA sequence in bacteria, yeasts or animal cells, characterized by the step of introducing into a cloning vehicle a DNA sequence selected from:
(a) the DNA inserts of Z-pBR322(Pst)/HcIF-4c, Z-pBR322(Pst)/HcIF-2h, Z-pBR322(Pst)/HcIF-SN35, Z-pBR322 (Pst)/HcIF-SN42 and Z-pKT287 (Pst)/HcIF-2h-AH6, said DNA inserts being the DNA inserts of the recombinant DNA molecules carried by the microorganisms identified by accession numbers DSM 1699-1703, respectively,
(b) DNA sequences which hybridize to any of the foregoing DNA inserts and which code for a polypeptide of the IFN-α type, and
(c) DNA sequences which are degenerate as a result of the genetic code to the DNA sequences and inserts defined in (a) and (b) and which code for a polypeptide of the IFN-α type.

2. The method according to claim 1, characterized in that said DNA sequence (b) which hybridizes to said DNA insert (a) is selected from:
(d) the DNA inserts of Z-pBR322(Pst)/HcIF-II-206 and Z-pBR322(Pst)/HcIF-SN35-AHL6, said DNA inserts being the DNA inserts of the recombinant DNA molecules carried by the microorganisms identified by accession numbers ATCC 31633-31634, respectively,
(e) DNA sequences which hybridize to any of the foregoing DNA inserts and which code for a polypeptide of the IFN-α type, and

48

(f) DNA sequences which are degenerate as a result of the genetic code to the DNA sequences and inserts defined in (d) and (e) and which code for a polypeptide of the IFN-α type.

3. The method according to claim 1 or 2, characterized in that said DNA sequence (b) or (e) which hybridizes to said DNA insert (a) or (d) is selected from:
     (g) the hybridizing portion of each of:
HchrIF-A, the subcloned HindIII fragment of chr 3 in E.coli HB101 (DSM 1914);
HchrIF-B, the subcloned EcoRI fragment of chr 12 in E.coli HB101 (DSM 1915);
HchrIF-C, the subcloned HindIII fragment of chr 12 in E.coli HB101 (DSM 1916);
HchrIF-D, the subcloned EcoRI fragment of chr 13 in E.coli HB101 (DSM 1917);
HchrIF-E, the subcloned EcoRI fragment of chr 23 in E.coli HB101 (DSM 1918);
HchrIF-F, the subcloned HindIII fragment of chr 23 in E.coli HB101 (DSM 1919);
HchrIF-G, the subcloned EcoRI fragment of chr 26 in E.coli HB101 (DSM 1920); and
HchrIF-H, the subcloned HindIII fragment of chr 26 in E.coli HB101 (DSM 1921).

4. The method according to claim 1, characterized in that said DNA sequence is selected from DNA sequences of the formula:

ATGGCCTCGCCCTTTGCTTTACTGATGGTCCTGGTGGTGCTCAGCTGCAAGTCAAGC

TGCTCTCTGGGCTGTGATCTCCCTGAGACCCACAGCCTGGATAACAGGAGGACCTTG

ATGCTCCTGGCACAAATGAGCAGAATCTCTCCTTCCTCCTGTCTGATGGACAGACAT

GACTTTGGATTTCCCCAGGAGGAGTTTGATGGCAACCAGTTCCAGAAGGCTCCAGCC

ATCTCTGTCCTCCATGAGCTGATCCAGCAGATCTTCAACCTCTTTACCACAAAAGAT

TCATCTGCTGCTTGGGATGAGGACCTCCTAGACAAATTCTGCACCGAACTCTACCAG

CAGCTGAATGACTTGGAAGCCTGTGTGATGCAGGAGGAGAGGGTGGGAGAAACTCCC

CTGATGAATGCGGACTCCATCTTGGCTGTGAAGAAATACTTCCGAAGAATCACTCTC

TATCTGACAGAGAAGAAATACAGCCCTTGTGCCTGGCAGGTTGTCAGAGCAGAAATC

ATGAGATCCCTCTCTTTATCAACAAACTTGCAAGAAAGATTAAGGAGGAAGGAA and

TGTGATCTCCCTGAGACCCACAGCCTGGATAACAGGAGGACCTTGATGCTCCTGGCA

CAAATGAGCAGAATCTCTCCTTCCTCCTGTCTGATGGACAGACATGACTTTGGATTT

CCCCAGGAGGAGTTTGATGGCAACCAGTTCCAGAAGGCTCCAGCCATCTCTGTCCTC

CATGAGCTGATCCAGCAGATCTTCAACCTCTTTACCACAAAAGATTCATCTGCTGCT

TGGGATGAGGACCTCCTAGACAAATTCTGCACCGAACTCTACCAGCAGCTGAATGAC

TTGGAAGCCTGTGTGATGCAGGAGGAGAGGGTGGGAGAAACTCCCCTGATGAATGCG

GACTCCATCTTGGCTGTGAAGAAATACTTCCGAAGAATCACTCTCTATCTGACAGAG

AAGAAATACAGCCCTTGTGCCTGGGAGGTTGTCAGAGCAGAAATCATGAGATCCCTC

TCTTTATCAACAAACTTGCAAGAAAGATTAAGGAGGAAGGAA.

5. The method according to claim 3, characterized in that said DNA sequence is selected from DNA sequences of the formula:

```
ATGGCCCTGTCCTTTTCTTTACTGATGGCCGTGCTGGTGCTCAGCTACAAATCCATC

TGTTCTCTGGGCTGTGATCTGCCTCAGACCCACAGCCTGGGTAATAGGAGGACCTTG

ATACTCCTGCAACAAATGGGAAGAATCTCTCATTTCTCCTGCCTGAAGGACAGACAT

GATTTCGGATTCCCCGAGGAGGAGTTTGATGGCCACCAGTTCCAGAAGACTCAAGCC

ATCTCTGTCCTCCATGAGATGATCCAGCAGACCTTCAATCTCTTCAGCACAGAGGAC

TCATCTGCTGCTTGGGAACAGAGCCTCCTAGAAAAATTTTCCACTGAACTTTACCAG

CAACTGAATGACCTGGAAGCATGTGTGATACAGGAGGTTGGGGTGGAAGAGACTCCC

CTGATGAATGTGGACTCCATCCTGGCTGTGAGGAAATACTTCCAAAGAATCACTCTT

TATCTAACAGAGAAGAAATACAGCCCTTGTGCCTGGGAGGTTGTCAGAGCAGAAATC

ATGAGATCCCTCTCGTTTTCAACAAACTTGCAAAAAAGATTAAGGAGGAAGGAT and

TGTGATCTGCCTCAGACCCACAGCCTGGGTAATAGGAGGACCTTGATACTCCTGCAA

CAAATGGGAAGAATCTCTCATTTCTCCTGCCTGAAGGACAGACATGATTTCGGATTC

CCCGAGGAGGAGTTTGATGGCCACCAGTTCCAGAAGACTCAAGCCATCTCTGTCCTC

CATGAGATGATCCAGCAGACCTTCAATCTCTTCAGCACAGAGGACTCATCTGCTGCT

TGGGAACAGAGCCTCCTAGAAAAATTTTCCACTGAACTTTACCAGCAACTGAATGAC

CTGGAAGCATGTGTGATACAGGAGGTTGGGGTGGAAGAGACTCCCCTGATGAATGTG

GACTCCATCCTGGCTGTGAGGAAATACTTCCAAAGAATCACTCTTTATCTAACAGAG

AAGAAATACAGCCCTTGTGCCTGGGAGGTTGTCAGAGCAGAAATCATGAGATCCCTC

TCGTTTTCAACAAACTTGCAAAAAAGATTAAGGAGGAAGGAT.
```

6. The method according to any one of claims 1 to 5 characterized in that said recombinant DNA molecule includes an expression control sequence to which said DNA sequence is operatively linked.

7. The method according to claim 6 characterized in that the expression control sequence is selected from a lac system, a β-lac system, a trp system, major operator and promoter region of phage λ, the control region of fd coat proteins and other sequences which control the expression of genes in bacteria, yeasts or animal cells.

8. The method according to claim 6 or 7, characterized in that the produced recombinant DNA molecule is selected from C8-IFN-α2, LAC-AUG(α2) and β-lac-AUG(α2), said recombinant DNA molecules being the recombinant DNA molecules carried by the microorganisms identified by accession numbers ATCC 31767-31769.

9. A method for transforming a host selected from bacteria, yeasts or animal cells, characterized by the step of introducing into said host a recombinant DNA molecule produced by the method of any of claims 1 to 8.

10. A method for producing a polypeptide of the IFN-α type, characterized by the steps of transforming a host selected from bacteria, yeasts or animal cells, with a recombinant DNA molecule produced by the method according to any one of claims 6 to 8, culturing said host, and collecting said polypeptide.

11. The method according to claim 9 or 10, characterized in that the untransformed host is selected from strains of E.coli, Pseudomonas, Bacillus subtilis, Bacillus stearothermophilus, other bacilli and mouse cells.

12. The method according to claim 10 or 11, characterized by the transformed host being selected from E.coli HB101 (Z-pR322(Pst)/HcIF-4c) [DSM 1699], E.coliHB101 (Z-pBR322(Pst)/HcIF-2h) [DSM 1700], E.coli HB101 (ZpBR322(Pst)/Hclf-SN35) [DSM 1701], E.coli HB101 (Z-pBR322 (Pst)/HcIF-SN42) [DSM 1702], and E.coli HB101 (Z-pKT287 (Pst)/HcIF-2h-AH6)[DSM 1703].

13. The method according to claim 10 or 11, characterized by the transformed host being selected from E.coli HB101 (Z-pBR322(Pst)/HcIF-II-206) [ATCC 31633] and E.coli HB101 (Z-pBR322(Pst)/HcIF-SN35-AHL6) [ATCC 31634].

14. The method according to claim 10 or 11, characterized by the transformed host being selected from HchrIF-A [DSM 1914], HchrIF-B [DSM 1915], HchrIF-C [DSM 1916], HchrIF-D [DSM 1917], HchrIF-E [DSM 1918], HchrIF-F [DSM 1919], HchrIF-G [DSM 1920] and HchrIF-H [DSM 1921].

15. The method according to claim 10 or 11, characterized by the transformed host being selected from E.coli DS410 (C8-IFN-α2) [ATCC 31767], E.coli DS410 (LAC- AUG(α2) [ATCC 31768] and E.coli DS410 (β-lac-AUG(α2)) [ATCC 31769).

16. A method for producing a polypeptide of the IFN-α type comprising the steps of culturing a host selected from bacteria, yeasts and animal cells and transformed by a recombinant DNA molecule produced by the method according to any one of claims 6 to 8 and collecting said polypeptide.

17. The method according to any one of claims 10, 11 and 16, characterized in that the polypeptide is selected from polypeptides of the formula:

MetAlaSerProPheAlaLeuLeuMetValLeuValValLeuSerCysLysSerSer

CysSerLeuGlyCysAspLeuProGluThrHisSerLeuAspAsnArgArgThrLeu

MetLeuLeuAlaGlnMetSerArgIleSerProSerSerCysLeuMetAspArgHis   .

AspPheGlyPheProGlnGluGluPheAspGlyAsnGlnPheGlnLysAlaProAla

IleSerValLeuHisGluLeuIleGlnGlnIlePheAsnLeuPheThrThrLysAsp

SerSerAlaAlaTrpAspGluAspLeuLeuAspLysPheCysThrGluLeuTyrGln

GlnLeuAsnAspLeuGluAlaCysValMetGlnGluGluArgValGlyGluThrPro

LeuMetAsnAlaAspSerIleLeuAlaValLysLysTyrPheArgArgIleThrLeu

TyrLeuThrGluLysLysTyrSerProCysAlaTrpGluValValArgAlaGluIle

MetArgSerLeuSerLeuSerThrAsnLeuGlnGluArgLeuArgArgLysGlu and

CysAspLeuProGluThrHisSerLeuAspAsnArgArgThrLeuMetLeuLeu

AlaGlnMetSerArgIleSerProSerSerCysLeuMetAspArgHisAspPheGly

PheProGlnGluGluPheAspGlyAsnGlnPheGlnLysAlaProAlaIleSerVal

LeuHisGluLeuIleGlnGlnIlePheAsnLeuPheThrThrLysAspSerSerAla

AlaTrpAspGluAspLeuLeuAspLysPheCysThrGluLeuTyrGlnGlnLeuAsn

AspLeuGluAlaCysValMetGlnGluGluArgValGlyGluThrProLeuMetAsn

AlaAspSerIleLeuAlaValLysLysTyrPheArgArgIleThrLeuTyrLeuThr

GluLysLysTyrSerProCysAlaTrpGluValValArgAlaGluIleMetArgSer

LeuSerLeuSerThrAsnLeuGlnGluArgLeuArgArgLysGlu.

18. The method according to any one of claims 10, 11 and 16, characterized in that the polypeptide is selected from polypeptides of the formula:

                                                MetAlaLeuSerPheSerLeu

LeuMetAlaValLeuValLeuSerTyrLysSerIleCysSerLeuGlyCysAspLeu

ProGlnThrHisSerLeuGlyAsnArgArgThrLeuIleLeuLeuGlnGlnMetGly

ArgIleSerHisPheSerCysLeuLysAspArgHisAspPheGlyPheProGluGlu

GluPheAspGlyHisGlnPheGlnLysThrGlnAlaIleSerValLeuHisGluMet

IleGlnGlnThrPheAsnLeuPheSerThrGluAspSerSerAlaAlaTrpGluGln

SerLeuLeuGluLysPheSerThrGluLeuTyrGlnGlnLeuAsnAspLeuGluAla

CysValIleGlnGluValGlyValGluGluThrProLeuMetAsnValAspSerIle

LeuAlaValArgLysTyrPheGlnArgIleThrLeuTyrLeuThrGluLysLysTyr

SerProCysAlaTrpGluValValArgAlaGluIleMetArgSerLeuSerPheSer

ThrAsnLeuGlnLysArgLeuArgArgLysAsp  and  CysAspLeuProGlnThr

HisSerLeuGlyAsnArgArgThrLeuIleLeuLeuGlnGlnMetGlyArgIleSer

HisPheSerCysLeuLysAspArgHisAspPheGlyPheProGluGluGluPheAsp

GlyHisGlnPheGlnLysThrGlnAlaIleSerValLeuHisGluMetIleGlnGln

ThrPheAsnLeuPheSerThrGluAspSerSerAlaAlaTrpGluGlnSerLeuLeu

GluLysPheSerThrGluLeuTyrGlnGlnLeuAsnAspLeuGluAlaCysValIle

GlnGluValGlyValGluGluThrProLeuMetAsnValAspSerIleLeuAlaVal

ArgLysTyrPheGlnArgIleThrLeuTyrLeuThrGluLysLysTyrSerProCys

AlaTrpGluValValArgAlaGluIleMetArgSerLeuSerPheSerThrAsnLeu

GlnLysArgLeuArgArgLysAsp.

19. A process for selecting a DNA sequence coding for a polypeptide of the IFN-$\alpha$ type from a group of DNA sequences characterized by the step of determining which of said DNA sequences hybridize to a DNA sequence that characterizes a recombinant DNA molecule produced according to the method of any one of claims 1 to 8.

20. The process of claim 19, characterized in that the DNA sequence screened is selected from DNA sequences from natural sources, synthetic DNA sequences, DNA sequences from recombinant DNA molecules and DNA sequences which are a combination of any of the foregoing DNA sequences.

**Patentansprüche**

**Patentansprüche für folgende Vertragsstaaten : BE, CH, DE, FR, GB, IT, LI, LU, NL, SE**

1. Rekombinantes DNA-Molekül zur Verwendung bei der Clonierung einer DNA-Sequenz in Bakterien, Hefen oder tierischen Zellen, wobei das rekombinante DNA-Molekül eine DNA-Sequenz enthält, ausgewählt aus
   (a) den DNA-Insertionen von Z-pBR322(Pst)/HcIF-4c, Z-pBR322(Pst)/HcIF-2h, Z-pBR322(Pst) /HcIF-SN35, Z-pBR322(Pst)/HcIF-SN42 und Z-pKT287(Pst)/HcIF-2h-AH6, wobei die DNA-Insertionen die DNA-Insertionen der rekombinanten DNA-Moleküle sind, die von den Mikroorganismen getragen werden, die durch die Hinterlegungsnummern DSM 1699-1703 gekennzeichnet sind,
   (b) DNA-Sequenzen, die mit einer der vorgenannten DNA-Insertionen hybridisieren und ein Polypeptid des IFN-$\alpha$-Typs codieren, und
   (c) DNA-Sequenzen, die infolge des genetischen Codes zu den DNA-Sequenzen und Insertionen gemäß (a) und (b) degeneriert sind und die ein Polypeptid des IFN-$\alpha$-Typs codieren.

2. Rekombinantes DNA-Molekül nach Anspruch 1, wobei die mit der DNA-Insertion (a) hybridisierende DNA-Sequenz (b) ausgewählt ist aus:
   (d) den DNA-Insertionen von Z-pBR322(Pst)/HcIF-II-206 und Z-pBR322(Pst)/HcIF-SN35-AHL6, wobei die DNA-Insertionen die DNA-Insertionen der rekombinanten DNA-Moleküle sind, die von den Mikroorganismen getragen werden, die durch die Hinterlegungsnummern ATCC 31633-31634 gekennzeichnet sind,
   (e) DNA-Sequenzen, die mit einer der vorgenannten DNA-Insertionen hybridisieren und die ein Polypeptid des IFN-$\alpha$-Typs codieren, und
   (f) DNA-Sequenzen, die infolge des genetischen Codes zu den DNA-Sequenzen und Insertionen gemäß (d) und (e) degeneriert sind und die ein Polypeptid des IFN-$\alpha$-Typs codieren.

3. Rekombinantes DNA-Molekül nach Anspruch 1 oder 2, wobei die mit der DNA-Insertion (a) oder (d) hybridisierende DNA-Sequenz (b) oder (e) ausgewählt ist aus:
   (g) dem hybridisierenden Bereich von:
   HchrIF-A, dem subclonierten HindIII-Fragment von chr 3 in E.coli HB101 (DSM 1914);
   HchrIF-B, dem subclonierten EcoRI-Fragment von chr 12 in E.coli HB101 (DSM 1915);
   HchrIF-C, dem subclonierten HindIII-Fragment von chr 12 in E.coli HB101 (DSM 1916);
   HchrIF-D, dem subclonierten EcoRI-Fragment von chr 13 in E.coli HB101 (DSM 1917);

HchrIF-E, dem subclonierten <u>EcoRI</u>-Fragment von chr 23 in <u>E.coli</u> HB101 (DSM 1918);
HchrIF-F, dem subclonierten <u>HindIII</u>-Fragment von chr 23 in <u>E.coli</u> HB101 (DSM 1919);
HchrIF-G, dem subclonierten <u>EcoRI</u>-Fragment von chr 26 in <u>E.coli</u> HB101 (DSM 1920); und
HchrIF-H, dem subclonierten <u>HindIII</u>-Fragment von chr 26 in <u>E.coli</u> HB101 (DSM 1921).

4. Rekombinantes DNA-Molekül nach Anspruch 1, wobei die DNA-Sequenz ausgewählt ist aus DNA-Sequenzen der Formel:

```
                                    ATGGCCTCGCCCTTTG
CTTTACTGATGGTCCTGGTGGTGCTCAGCTGCAAGTCAAGCTGCTCTCTGGG
CTGTGATCTCCCTGAGACCCACAGCCTGGATAACAGGAGGACCTTGATGCTC
CTGGCACAAATGAGCAGAATCTCTCCTTCCTCCTGTCTGATGGACAGACATG
ACTTTGGATTTCCCCAGGAGGAGTTTGATGGCAACCAGTTCCAGAAGGCTCC
AGCCATCTCTGTCCTCCATGAGCTGATCCAGCAGATCTTCAACCTCTTTACC
```

```
ACAAAAGATTCATCTGCTGCTTGGGATGAGGACCTCCTAGACAAATTCTGCA
CCGAACTCTACCAGCAGCTGAATGACTTGGAAGCCTGTGTGATGCAGGAGGA
GAGGGTGGGAGAAACTCCCCTGATGAATGCGGACTCCATCTTGGCTGTGAAG
AAATACTTCCGAAGAATCACTCTCTATCTGACAGAGAAGAAATACAGCCCTT
GTGCCTGGGAGGTTGTCAGAGCAGAAATCATGAGATCCCTCTCTTTATCAAC
AAACTTGCAAGAAAGATTAAGGAGGAAGGAA und TGTGATCTCCCTGAGA
CCCACAGCCTGGATAACAGGAGGACCTTGATGCTCCTGGCACAAATGAGCAG
AATCTCTCCTTCCTCCTGTCTGATGGACAGACATGACTTTGGATTTCCCCAG
GAGGAGTTTGATGGCAACCAGTTCCAGAAGGCTCCAGCCATCTCTGTCCTCC
ATGAGCTGATCCAGCAGATCTTCAACCTCTTTACCACAAAAGATTCATCTGC
TGCTTGGGATGAGGACCTCCTAGACAAATTCTGCACCGAACTCTACCAGCAG
CTGAATGACTTGGAAGCCTGTGTGATGCAGGAGGAGAGGGTGGGAGAAACTC
CCCTGATGAATGCGGACTCCATCTTGGCTGTGAAGAAATACTTCCGAAGAAT
CACTCTCTATCTGACAGAGAAGAAATACAGCCCTTGTGCCTGGGAGGTTGTC
AGAGCAGAAATCATGAGATCCCTCTCTTTATCAACAAACTTGCAAGAAAGAT
TAAGGAGGAAGGAA.
```

5. Rekombinantes DNA-Molekül nach Anspruch 3, wobei die DNA-Sequenz ausgewählt ist aus DNA-Sequenzen der Formel:

ATGGCCCTGTCCTTTT

CTTTACTGATGGCCGTGCTGGTGCTCAGCTACAAATCCATCTGTTCTCTGGG

CTGTGATCTGCCTCAGACCCACAGCCTGGGTAATAGGAGGACCTTGATACTC

CTGCAACAAATGGGAAGAATCTCTCATTTCTCCTGCCTGAAGGACAGACATG

ATTTCGGATTCCCCGAGGAGGAGTTTGATGGCCACCAGTTCCAGAAGACTCA

AGCCATCTCTGTCCTCCATGAGATGATCCAGCAGACCTTCAATCTCTTCAGCA

CAGAGGACTCATCTGCTGCTTGGGAACAGAGCCTCCTAGAAAAATTTTCCACT

GAACTTTACCAGCAACTGAATGACCTGGAAGCATGTGTGATACAGGAGGTTGG

GGTGGAAGAGACTCCCCTGATGAATGTGGACTCCATCCTGGCTGTGAGGAAAT

ACTTCCAAAGAATCACTCTTTATCTAACAGAGAAGAAATACAGCCCTTGTGCC

TGGGAGGTTGTCAGAGCAGAAATCATGAGATCCCTCTCGTTTTCAACAAACTT

GCAAAAAGATTAAGGAGGAAGGAT u.nd TGTGATCTGCCTCAGACCCACAG

CCTGGGTAATAGGAGGACCTTGATACTCCTGCAACAAATGGGAAGAATCTCTC

ATTTCTCCTGCCTGAAGGACAGACATGATTTCGGATTCCCCGAGGAGGAGTTT

GATGGCCACCAGTTCCAGAAGACTCAAGCCATCTCTGTCCTCCATGAGATGAT

CCAGCAGACCTTCAATCTCTTCAGCACAGAGGACTCATCTGCTGCTTGGGAAC

AGAGCCTCCTAGAAAAATTTTCCACTGAACTTTACCAGCAACTGAATGACCTG

GAAGCATGTGTGATACAGGAGGTTGGGGTGGAAGAGACTCCCCTGATGAATGT

GGACTCCATCCTGGCTGTGAGGAAATACTTCCAAAGAATCACTCTTTATCTAA

CAGAGAAGAAATACAGCCCTTGTGCCTGGGAGGTTGTCAGAGCAGAAATCATG

AGATCCCTCTCGTTTTCAACAAACTTGCAAAAAGATTAAGGAGGAAGGAT.

**6.** Rekombinantes DNA-Molekül nach einem der Ansprüche 1 bis 5 zur Verwendung bei der Expression der clonierten DNA-Sequenz in Bakterien, Hefen oder tierischen Zellen, dadurch gekennzeichnet, daß die DNA-Sequenz in dem rekombinanten DNA-Molekül funktionell mit einer Expressionskontrollsequenz verbunden ist.

**7.** Rekombinantes DNA-Molekül nach Anspruch 6, wobei die Expressionskontrollsequenz ausgewählt ist aus einem lac-System, einem β-lac-System, einem trp-System, den Hauptoperator- und Promotorbereichen des Phagen λ, dem Kontrollbereich des fd-Hüllproteins und anderen Sequenzen, die die Expression von Genen in Bakterien, Hefen oder tierischen Zellen steuern.

**8.** Rekombinantes DNA-Molekül nach Anspruch 6 oder 7, ausgewählt aus C8-IFN-α2, LAC-AUG(α2) und β-lac-AUG(α2), wobei die rekombinanten DNA-Moleküle die rekombinanten DNA-Moleküle sind, die von den durch die Hinterlegungsnummern ATCC 31767-31769 gekennzeichneten Mikroorganismen getragen werden.

**9.** Wirt, transformiert mit mindestens einem rekombinanten DNA-Molekül nach einem der Ansprüche 1 bis 8, wobei der Wirt aus Bakterien, Hefen und tierischen Zellen ausgewählt ist.

**10.** Wirt nach Anspruch 9, wobei die Bakterien, Hefen oder tierischen Zellen ausgewählt sind aus Stämmen von E.coli, Pseudomonas, Bacillus subtilis, Bacillus stearo- thermophilus, anderen Bacillen und Mauszellen.

**11.** Transformierter Wirt nach Anspruch 10, ausgewählt aus E.coli HB101 (Z-pBR322(Pst)/HcIF-4c)[DSM 1699], E.coli HB101 (Z-pBR322(Pst)/HcIF-2h)[DSM 1700], E.coli HB101 (Z-pBR322(Pst)/HcIF-SN35)[DSM 1701], E.coli HB101 (Z-pBR322(Pst)/HcIF-SN42)[DSM 1702] und E.coli HB101 (Z-pKT287(Pst)/HcIF-2h-AH6) [DSM 1703].

12. Transformierter Wirt nach Anspruch 10, ausgewählt aus E.coli HB101 (Z-pBR322(Pst)/HcIF-II-206)[ATCC 31633] und E.coli HB101 (Z-pBR322(Pst)/HcIF-SN35-AHL6) [ATCC 31634].

13. Transformierter Wirt nach Anspruch 10, ausgewählt aus HchrIF-A [DSM 1914], HchrIF-B[DSM 1915], HchrIF-C[DSM 1916] , HchrIF-D[DSM 1917], HchrIF-E[DSM 1918] , HchrIF-F[DSM 1919], HchrIF-G[DSM 1920] und HchrIF-H[DSM 1921].

14. Transformierter Wirt nach Anspruch 10, ausgewählt aus E.coli DS410 (C8-IFN-α2) [ATCC 31767], E.coli DS410 (LAC-AUG(α2))[ATCC 31768] und E.coli DS410 HB101 (β-lac-AUG(α2)) [ATCC 31769].

15. Verfahren zur Herstellung eines Polypeptids des IFN-α-Typs, wobei ein transformierter Wirt nach einem der Ansprüche 9 bis 14, soweit diese Ansprüche von den Ansprüchen 6 bis 8 abhängen, gezüchtet und das Polypeptid gesammelt wird.

16. Polypeptid des IFN-α-Typs, hergestellt nach dem Verfahren von Anspruch 15 und ausgewählt aus Polypeptiden der Formel:

**METALASER**

PROPHEALALEULEUMETVALLEUVALVALLEUSERCYSLYSSERSERCYSSER
LEUGLYCYSASPLEUPROGLUTHRHISSERLEUASPASNARGARGTHRLEUMET
LEULEUALAGLNMETSERARGILESERPROSERSERCYSLEUMETASPARGHIS
ASPPHEGLYPHEPROGLNGLUGLUPHEASPGLYASNGLNPHEGLNLYSALAPRO
ALAILESERVALLEUHISGLULEUILEGLNGLNILEPHEASNLEUPHETHRTHR
LYSASPSERSERALAALAATRPASPGLUASPLEULEUASPLYSPHECYSTHRGLU

LEUTYRGLNGLNLEUASNASPLEUGLUALACYSVALMETGLNGLUGLUARGVAL
GLYGLUTHRPROLEUMETASNALAASPSERILELEUALAVALLYSLYSTYRPHE
ARGARGILETHRLEUTYRLEUTHRGLULYSLYSTYRSERPROCYSALAATRPGLU
VALVALALARGALAGLUILEMETARGSERLEUSERLEUSERTHRASNLEUGLNGLU
ARGLEUARGARGLYSGLU und CYSASPLEUPROGLUTHRHISSERLEUASPA
SNARGARGTHRLEUMETLEULEUALAGLNMETSERARGILESERPROSERSERC
YSLEUMETASPARGHISASPPHEGLYPHEPROGLNGLUGLUPHEASPGLYASNG
LNPHEGLNLYSALAPROALAILESERVALLEUHISGLULEUILEGLNGLNILEP
HEASNLEUPHETHRTHRLYSASPSERSERALAALAATRPASPGLUASPLEULEUA
SPLYSPHECYSTHRGLULEUTYRGLNGLNLEUASNASPLEUGLUGLUALACYSVALM
ETGLNGLUGLUARGVALGLYGLUTHRPROLEUMETASNALAASPSERILELELEUA
LAVALLYSLYSTYRPHEARGARGILETHRLEUTYRLEUTHRGLULYSLYSTYRS
ERPROCYSALAATRPGLUVALVALALARGALAGLUILEMETARGSERLEUSERLEUS
ERTHRASNLEUGLNGLUARGLEUARGARGLYSGLU.

17. Polypeptid des IFN-α-Typs, hergestellt nach dem Verfahren von Anspruch 15 und ausgewählt aus Poly-

peptiden der Formel:

METALA

LEUSERPHESERLEULEUMETALAVALLEUVALLEUSERTYRLYSSERILE
CYSSERLEUGLYCYSASPLEUPROGLNTHRHISSERLEUGLYASNARGARG
THRLEUILELEULEUGLNGLNMETGLYARGILESERHISPHESERCYSLEU
LYSASPARGHISASPPHEGLYPHEPROGLUGLUGLUPHEASPGLYHISGLN
PHEGLNLYSTHRGLNALAILESERVALLEUHISGLUMETILEGLNGLNTHR
PHEASNLEUPHESERTHRGLUASPSERSERALAALATRPGLUGLNSERLEU
LEUGLULYSPHESERTHRGLULEUTYRGLNGLNLEUASNASPLEUGLUALA
CYSVALILEGLNGLUVALGLYVALGLUGLUTHRPROLEUMETASNVALASP
SERILELEUALAVALARGLYSTYRPHEGLNARGILETHRLEUTYRLEUTHR
GLULYSLYSTYRSERPROCYSALATRPGLUVALVALARGALAGLUILEMET
ARGSERLEUSERPHESERTHRASNLEUGLNLYSARGLEUARGARGLYSASP
und CYSASPLEUPROGLNTHRHISSERLEUGLYASNARGARG
THRLEUILELEULEUGLNGLNMETGLYARGILESERHISPHESERCYSLEU
LYSASPARGHISASPPHEGLYPHEPROGLUGLUGLUPHEASPGLYHISGLN
PHEGLNLYSTHRGLNALAILESERVALLEUHISGLUMETILEGLNGLNTHR
PHEASNLEUPHESERTHRGLUASPSERSERALAALATRPGLUGLNSERLEU
LEUGLULYSPHESERTHRGLULEUTYRGLNGLNLEUASNASPLEUGLUALA

CYSVALILEGLNGLUVALGLYVALGLUGLUTHRPROLEUMETASNVALASP
SERILELEUALAVALARGLYSTYRPHEGLNARGILETHRLEUTYRLEUTHR
GLULYSLYSTYRSERPROCYSALATRPGLUVALVALARGALAGLUILEMET
ARGSERLEUSERPHESERTHRASNLEUGLNLYSARGLEUARGARGLYSASP.

18. Verfahren zur Herstellung eines rekombinanten DNA-Moleküls nach einem der Ansprüche 1 bis 5, wobei man in einen Clonierungsvektor eine DNA-Sequenz einfügt, die die rekombinanten DNA-Moleküle nach einem der Ansprüche 1 bis 5 kennzeichnet.

19. Verfahren zur Herstellung eines rekombinanten DNA-Moleküls nach einem der Ansprüche 6 bis 8, wobei man eine DNA-Sequenz, die die rekombinanten DNA-Moleküle nach einem der Ansprüche 1 bis 5 kennzeichnet, in einen Clonierungsvektor einfügt und eine Expressionskontrollsequenz nach Anspruch 7 in den Clonierungsvektor einfügt, wobei die Expressionskontrollsequenz so in den Clonierungsvektor eingefügt wird, daß sie die Expression der DNA-Sequenz steuert und reguliert.

20. Verfahren zur Transformation eines Wirtes aus der Gruppe der Bakterien, Hefen oder tierischen Zellen, wobei man ein rekombinantes DNA-Molekül nach einem der Ansprüche 1 bis 8 in den Wirt einschleust.

21. Verfahren zur Herstellung eines Polypeptids des IFN-$\alpha$-Typs, wobei ein Wirt aus der Gruppe der Bakterien, Hefen oder tierischen Zellen mit einem rekombinanten DNA-Molekül nach einem der Ansprüche 6 bis 8 transformiert wird, der Wirt gezüchtet und das Polypeptid gesammelt wird.

**22.** Verfahren nach Anspruch 21, wobei der Wirt ausgewählt ist aus Stämmen von <u>E.coli</u>, <u>Pseudomonas</u>, <u>Bacillus subtilis</u>, <u>Bacillus</u> <u>stearothermophilus</u> und anderen Bacillen.

**23.** Verfahren zur Herstellung eines Polypeptids des IFN-$\alpha$- Typs, wobei ein mit einem rekombinanten DNA-Molekül nach einem der Ansprüche 6 bis 8 transformierter Wirt aus der Gruppe der Bakterion, Hefen oder tierischen Zellen gezüchtet wird und das Polypeptid gesammelt wird.

**24.** Verfahren zur Auswahl einer ein Polypeptid des IFN-$\alpha$-Typs codierenden DNA-Sequenz aus einer Gruppe von DNA-Sequenzen, wobei festgestellt wird, welche der DNA-Sequenzen mit einer DNA-Sequenz hybridisiert, die die rekombinanten DNA-Moleküle nach einem der Ansprüche 1 bis 8 kennzeichnet.

**25.** Verfahren nach Anspruch 24, wobei die ermittelte DNA-Sequenz ausgewählt ist aus DNA-Sequenzen aus natürlichen Quellen, synthetischen DNA-Sequenzen, DNA-Sequenzen aus rekombinanten DNA-Molekülen und DNA-Sequenzen, die eine Kombination irgendwelcher der vorgenannten DNA-Sequenzen sind.

**26.** Mittel zur Behandlung menschlicher Viren oder zur Behandlung menschlicher Krebserkrankungen oder Tumoren, das als einziges IFN-$\alpha$ ein Polypeptid enthält, das aus einem Polypeptid nach einem der Ansprüche 16 oder 17 ausgewählt ist oder nach dem Verfahren nach einem der Ansprüche 15, 21, 22 oder 23 hergestellt wird.

**27.** Mittel zur Behandlung viraler Rinderinfektionen, das als einziges IFN-$\alpha$ ein Polypeptid enthält, das aus einem Polypeptid nach einem der Ansprüche 16 oder 17 ausgewählt ist oder nach dem Verfahren nach einem der Ansprüche 15, 21, 22 oder 23 hergestellt wird.

**28.** DNA-Sequenz, ausgewählt aus DNA-Sequenzen der Formel:

ATGGCCTCGCCCTTTGCTTTACTGA
TGGTCCTGGTGGTGCTCAGCTGCAAGTCAAGCTGCTCTCTGGGCTGTGATCT
CCCTGAGACCCACAGCCTGGATAACAGGAGGACCTTGATGCTCCTGGCACAA
ATGAGCAGAATCTCTCCTTCCTCCTGTCTGATGGACAGACATGACTTTGGAT
TTCCCCAGGAGGAGTTTGATGGCAACCAGTTCCAGAAGGCTCCAGCCATCTC
TGTCCTCCATGAGCTGATCCAGCAGATCTTCAACCTCTTTACCACAAAAGAT
TCATCTGCTGCTTGGGATGAGGACCTCCTAGACAAATTCTGCACCGAACTCT
ACCAGCAGCTGAATGACTTGGAAGCCTGTGTGATGCAGGAGGAGAGGGTGGG
AGAAACTCCCCTGATGAATGCGGACTCCATCTTGGCTGTGAAGAAATACTTC
CGAAGAATCACTCTCTATCTGACAGAGAAGAAATACAGCCCTTGTGCCTGGG
AGGTTGTCAGAGCAGAAATCATGAGATCCCTCTCTTTATCAACAAACTTGCA
AGAAAGATTAAGGAGGAAGGAA und TGTGATCTCCCTGAGACCCACAGCC
TGGATAACAGGAGGACCTTGATGCTCCTGGCACAAATGAGCAGAATCTCTCC
TTCCTCCTGTCTGATGGACAGACATGACTTTGGATTTCCCCAGGAGGAGTTT
GATGGCAACCAGTTCCAGAAGGCTCCAGCCATCTCTGTCCTCCATGAGCTGA
TCCAGCAGATCTTCAACCTCTTTACCACAAAAGATTCATCTGCTGCTTGGGA
TGAGGACCTCCTAGACAAATTCTGCACCGAACTCTACCAGCAGCTGAATGAC
TTGGAAGCCTGTGTGATGCAGGAGGAGAGGGTGGGAGAAACTCCCCTGATGA
ATGCGGACTCCATCTTGGCTGTGAAGAAATACTTCCGAAGAATCACTCTCTA
TCTGACAGAGAAGAAATACAGCCCTTGTGCCTGGGAGGTTGTCAGAGCAGAA
ATCATGAGATCCCTCTCTTTATCAACAAACTTGCAAGAAAGATTAAGGAGGA

AGGAA, wobei die DNA-Sequenz geeignet ist zur Clonierung oder Expression der Sequenz in Bakterien, Hefen oder tierischen Zellen und zur Auswahl von DNA-Sequenzen, die ein Polypeptid des IFN-α-Typs codieren.

**29.** DNA-Sequenz, ausgewählt aus DNA-Sequenzen der Formel:

ATGGCCCTGTCCTTTTCTTTACTGA
TGGCCGTGCTGGTGCTCAGCTACAAATCCATCTGTTCTCTGGGCTGTGATCT
GCCTCAGACCCACAGCCTGGGTAATAGGAGGACCTTGATACTCCTGCAACAA
ATGGGAAGAATCTCTCATTTCTCCTGCCTGAAGGACAGACATGATTTCGGAT
TCCCCGAGGAGGAGTTTGATGGCCACCAGTTCCAGAAGACTCAAGCCATCTC
TGTCCTCCATGAGATGATCCAGCAGACCTTCAATCTCTTCAGCACAGAGGAC

TCATCTGCTGCTTGGGAACAGAGCCTCCTAGAAAAATTTTCCACTGAACTTT

ACCAGCAACTGAATGACCTGGAAGCATGTGTGATACAGGAGGTTGGGGTGGA

AGAGACTCCCCTGATGAATGTGGACTCCATCCTGGCTGTGAGGAAATACTTC

CAAAGAATCACTCTTTATCTAACAGAGAAGAAATACAGCCCTTGTGCCTG

GGAGGTTGTCAGAGCAGAAATCATGAGATCCCTCTCGTTTTCAACAAACTTG

CAAAAAAGATTAAGGAGGAAGGAT und TGTGATCTGCCTCAGACCCACAG

CCTGGGTAATAGGAGGACCTTGATACTCCTGCAACAAATGGGAAGAATCTCT

CATTTCTCCTGCCTGAAGGACAGACATGATTTCGGATTCCCCGAGGAGGAGT

TTGATGGCCACCAGTTCCAGAAGACTCAAGCCATCTCTGTCCTCCATGAGAT

GATCCAGCAGACCTTCAATCTCTTCAGCACAGAGGACTCATCTGCTGCTTGG

GAACAGAGCCTCCTAGAAAAATTTTCCACTGAACTTTACCAGCAACTGAATG

ACCTGGAAGCATGTGTGATACAGGAGGTTGGGGTGGAAGAGACTCCCCTGAT

GAATGTGGACTCCATCCTGGCTGTGAGGAAATACTTCCAAAGAATCACTCTT

TATCTAACAGAGAAGAAATACAGCCCTTGTGCCTGGGAGGTTGTCAGAGCAG

AAATCATGAGATCCCTCTCGTTTTCAACAAACTTGCAAAAAAGATTAAGGAG

GAAGGAT, wobei die DNA-Sequenz geeignet ist zur Clonierung oder Expression der DNA-Sequenz in Bakterien, Hefen oder tierischen Zellen und zur Selektion von DNA-Sequenzen, die ein Polypeptid des IFN-α-Typs codieren.

**Patentansprüche für folgenden Vertragsstaat : AT**

1. Verfahren zur Herstellung eines rekombinanten DNA-Moleküls zur Verwendung bei der Clonierung einer DNA-Sequenz in Bakterien, Hefen oder tierischen Zellen, dadurch gekennzeichnet, daß in einen Clonierungsvektor eine DNA-Sequenz eingefügt wird, die ausgewählt ist aus
   (a) den DNA-Insertionen von Z-pBR322(Pst)/HcIF-4c, Z-pBR322(Pst)/HcIF-2h, Z- pBR322(Pst)/HcIF-SN35, Z-pBR322(Pst)/HcIF-SN42 undZ-pKT287(Pst)/HcIF-2h-AH6, wobei die DNA-Insertionen die DNA-Insertionen der rekombinanten DNA-Moleküle sind, die von den Mikroorganismen getragen werden, die durch die Hinterlegungsnummern DSM 1699-1703 identifiziert sind,
   (b) DNA-Sequenzen, die mit einer der vorgenannten DNA-Insertionen hybridisieren und die für ein Polypeptid des IFN-α-Typs codieren, und
   (c) DNA-Sequenzen, die infolge des genetischen Codes zu den DNA-Sequenzen und Insertionen gemäß (a) und (b) degeneriert sind und die für ein Polypeptid des IFN-α-Typs codieren.

2. Verfahren nach Anspruch 1, dadurch gekennzeichnet, daß die DNA-Sequenz (b), die mit der DNA-Insertion (a) hybridisiert, ausgewählt ist aus:
   (d) den DNA-Insertionen von Z-pBR322(Pst)/HcIF-II-206 und Z-pBR322(Pst)/HcIF-SN35- AHL6, wobei die DNA-Insertionen die DNA-Insertionen der rekombinanten DNA-Moleküle sind, die von den Mikroorganismen getragen werden, die durch die Hinterlegungsnummern ATCC 31633-31634 identifiziert sind,
   (e) DNA-Sequenzen, die mit einer der vorgenannten DNA-Insertionen hybridisieren und die für ein Polypeptid des IFN-α-Typs codieren, und
   (f) DNA-Sequenzen, die infolge des genetischen Codes zu den DNA-Sequenzen und Insertionen gemäß (d) und (e) degeneriert sind und die für ein Polypeptid des IFN-α-Typs codieren.

3. Verfahren nach Anspruch 1 oder 2. dadurch gekennzeichnet, daß die DNA-Sequenz (b) oder (e), die mit der DNA-Insertion (a) oder (d) hybridisiert, ausgewählt ist aus:
   (g) dem hybridisierenden Teil von:

HcrIF-A, dem subclonierten <u>HindIII</u>-Fragment von chr 3 in <u>E.coli</u> HB101 (DSM 1914);
HcrIF-B, dem subclonierten <u>EcoRI</u>-Fragment von chr 12 in <u>E.coli</u> HB101 (DSM 1915);
HchrIF-C, dem subclonierten <u>HindIII</u>-Fragment von chr 12 in <u>E.coli</u> HB101 (DSM 1916);
HchrIF-D, dem subclonierten <u>EcoRI</u>-Fragment von chr 13 in <u>E.coli</u> HB101 (DSM 1917);
HchrIF-E, dem subclonierten <u>EcoRI</u>-Fragment von chr 23 in <u>E.coli</u> HB101 (DSM 1918);
HchrIF-F, dem subclonierten <u>HindIII</u>-Fragment von chr 23 in <u>E.coli</u> HB101 (DSM 1919);
HchrIF-G, dem subclonierten <u>EcoRI</u>-Fragment von chr 26 in <u>E.coli</u> HB101 (DSM 1920); und
HchrIF-H, dem subclonierten <u>HindIII</u>-Fragment von chr 26 in <u>E.coli</u> HB101 (DSM 1921).

4.  Verfahren nach Anspruch 1, dadurch gekennzeichnet, daß die DNA-Sequenz ausgewählt ist aus DNA-Sequenzen der Formel:

ATGGCCTCGCCCTTTGCTTTACTGATGGTCCTGGTGGTGCTCAGCTGCAAGTCAAGC

TGCTCTCTGGGCTGTGATCTCCCTGAGACCCACAGCCTGGATAACAGGAGGACCTTG

ATGCTCCTGGCACAAATGAGCAGAATCTCTCCTTCCTCCTGTCTGATGGACAGACAT

GACTTTGGATTTCCCCAGGAGGAGTTTGATGGCAACCAGTTCCAGAAGGCTCCAGCC

ATCTCTGTCCTCCATGAGCTGATCCAGCAGATCTTCAACCTCTTTACCACAAAAGAT

TCATCTGCTGCTTGGGATGAGGACCTCCTAGACAAATTCTGCACCGAACTCTACCAG

CAGCTGAATGACTTGGAAGCCTGTGTGATGCAGGAGGAGAGGGTGGGAGAAACTCCC

CTGATGAATGCGGACTCCATCTTGGCTGTGAAGAAATACTTCCGAAGAATCACTCTC

TATCTGACAGAGAAGAAATACAGCCCTTGTGCCTGGGAGGTTGTCAGAGCAGAAATC

ATGAGATCCCTCTCTTTATCAACAAACTTGCAAGAAAGATTAAGGAGGAAGGAA   und

TGTGATCTCCCTGAGACCCACAGCCTGGATAACAGGAGGACCTTGATGCTCCTGGCA

CAAATGAGCAGAATCTCTCCTTCCTCCTGTCTGATGGACAGACATGACTTTGGATTT

CCCCAGGAGGAGTTTGATGGCAACCAGTTCCAGAAGGCTCCAGCCATCTCTGTCCTC

CATGAGCTGATCCAGCAGATCTTCAACCTCTTTACCACAAAAGATTCATCTGCTGCT

TGGGATGAGGACCTCCTAGACAAATTCTGCACCGAACTCTACCAGCAGCTGAATGAC

TTGGAAGCCTGTGTGATGCAGGAGGAGAGGGTGGGAGAAACTCCCCTGATGAATGCG

GACTCCATCTTGGCTGTGAAGAAATACTTCCGAAGAATCACTCTCTATCTGACAGAG

AAGAAATACAGCCCTTGTGCCTGGGAGGTTGTCAGAGCAGAAATCATGAGATCCCTC

TCTTTATCAACAAACTTGCAAGAAAGATTAAGGAGGAAGGAA.

5.  Verfahren nach Anspruch 3, dadurch gekennzeichnet, daß die DNA-Sequenz ausgewählt ist aus DNA-Sequenzen der Formel:

ATGGCCCTGTCCTTTTCTTTACTGATGGCCGTGCTGGTGCTCAGCTACAAATCCATC

TGTTCTCTGGGCTGTGATCTGCCTCAGACCCACAGCCTGGGTAATAGGAGGACCTTG

ATACTCCTGCAACAAATGGGAAGAATCTCTCATTTCTCCTGCCTGAAGGACAGACAT

GATTTCGGATTCCCCGAGGAGGAGTTTGATGGCCACCAGTTCCAGAAGACTCAAGCC

ATCTCTGTCCTCCATGAGATGATCCAGCAGACCTTCAATCTCTTCAGCACAGAGGAC

TCATCTGCTGCTTGGGAACAGAGCCTCCTAGAAAAAATTTTCCACTGAACTTTACCAG

CAACTGAATGACCTGGAAGCATGTGTGATACAGGAGGTTGGGGTGGAAGAGACTCCC

CTGATGAATGTGGACTCCATCCTGGCTGTGAGGAAATACTTCCAAAGAATCACTCTT

EP 0 032 134 B2

TATCTAACAGAGAAGAAATACAGCCCTTGTGCCTGGGAGGTTGTCAGAGCAGAAATC

ATGAGATCCCTCTCGTTTTCAACAAACTTGCAAAAAAGATTAAGGAGGAAGGAT und

TGTGATCTGCCTCAGACCCACAGCCTGGGTAATAGGAGGACCTTGATACTCCTGCAA

CAAATGGGAAGAATCTCTCATTTCTCCTGCCTGAAGGACAGACATGATTTCGGATTC

CCCGAGGAGGAGTTTGATGGCCACCAGTTCCAGAAGACTCAAGCCATCTCTGTCCTC

CATGAGATGATCCAGCAGACCTTCAATCTCTTCAGCACAGAGGACTCATCTGCTGCT

TGGGAACAGAGCCTCCTAGAAAAATTTTCCACTGAACTTTACCAGCAACTGAATGAC

CTGGAAGCATGTGTGATACAGGAGGTTGGGGTGGAAGAGACTCCCCTGATGAATGTG

GACTCCATCCTGGCTGTGAGGAAATACTTCCAAAGAATCACTCTTTATCTAACAGAG

AAGAAATACAGCCCTTGTGCCTGGGAGGTTGTCAGAGCAGAAATCATGAGATCCCTC

TCGTTTTCAACAAACTTGCAAAAAAGATTAAGGAGGAAGGAT.

**6.** Verfahren nach einem der Ansprüche 1 bis 5, dadurch gekennzeichnet, daß das rekombinante DNA-Molekül eine Expressionskontrollsequenz enthält, mit der die DNA-Sequenz funktionell verbunden ist.

**7.** Verfahren nach Anspruch 6, dadurch gekennzeichnet, daß die Expressionskontrollsequenz ausgewählt ist aus einem lac-System, einem β-lac-System, einem trp-System, den Hauptoperator- und Promotorbereichen des Phagen λ, dem Kontrollbereich der fd-Hüllproteine und anderen Sequenzen, die die Expression von Genen in Bakterien, Hefen oder tierischen Zellen steuem.

**8.** Verfahren nach Anspruch 6 oder 7, dadurch gekennzeichnet, daß das hergestellte rekombinante DNA-Molekül ausgewählt ist aus C8-IFN-α2, LAC-AUG(α2) und β-lac-AUG(α2), wobei die rekombinanten DNA-Moleküle die rekombinanten DNA-Moleküle sind, die von den durch die Hinterlegungsnummern ATCC 31767-31769 identifizierten Mikroorganismen getragen werden.

**9.** Verfahren zur Transformation eines Wirts, ausgewählt aus der Gruppe der Bakterien, Hefen und tierischen Zellen, dadurch gekennzeichnet, daß man in den Wirt ein nach dem Verfahren nach einem der Ansprüche 1 bis 8 hergestelltes rekombinantes DNA-Molekül einbringt.

**10.** Verfahren zur Herstellung eines Polypeptids des IFN-α-Typs, dadurch gekennzeichnet, daß man einen Wirt ausgewählt aus der Gruppe der Bakterien, Hefen oder tierischen Zellen mit einem nach einem Verfahren nach einem der Ansprüche 6 bis 8 hergestellten rekombinanten DNA-Molekül transformiert, den Wirt züchtet und das Polypeptid sammelt.

**11.** Verfahren nach Anspruch 9 oder 10, dadurch gekennzeichnet, daß der nicht-transformierte Wirt ausgewählt ist aus Stämmen von E.coli, Pseudomonas, Bacillus subtilis, Bacillus stearothermophilus anderen Bacillen und Mauszellen.

**12.** Verfahren nach Anspruch 10 oder 11, dadurch gekennzeichnet, daß der transformierte Wirt ausgewählt ist aus E.coli HB101 (Z-pBR322(Pst)/HcIF-4c) [DSM 1699], E.coli HB101 (Z- pBR322(Pst)/HcIF-2h)[DSM 1700], E.coli HB101 (Z-pBR322(Pst)/HcIF-SN35)[DSM 1701], E.coli HB101 (Z-pBR322(Pst)/HcIF-SN42)[DSM 1702] und E.coli HB101 (Z- pKT287(Pst)/HcIF-2h-AH6) [DSM 1703].

**13.** Verfahren nach Anspruch 10 oder 11, dadurch gekennzeichnet, daß der transformierte Wirt ausgewählt ist aus E.coli HB101 (Z-pBR322(Pst)/Hcl F-II-206)[ATCC 31633] und E.coli HB101 (Z-pBR322(Pst)/HcIF-SN35-AHL6) [ATCC 31634].

**14.** Verfahren nach Anspruch 10 oder 11, dadurch gekennzeichnet, daß der transformierte Wirt ausgewählt ist aus HchrIF-A [DSM 19141, HchrIF-B [DSM 1915], HchrIF-C [DSM 1916], HchrIF-D [DSM 1917], HchrIF-E [DSM 1918], HchrIF-F [DSM 1919], HchrIF-G [DSM 1920] und HchrIF-H [DSM 1921].

**15.** Verfahren nach Anspruch 10 oder 11, dadurch gekennzeichnet, daß der transformierte Wirt ausgewählt ist aus E.coli DS410 (C8-IFN-α2) [ATCC 31767], E.coli DS410 (LAC-AUG(α2)) [ATCC 31768] und E.coli

62

DS410 (β-lacAUG(α2)) [ATCC 31769].

16. Verfahren zur Herstellung eines Polypeptids des IFN-α-Typs, wobei man einen Wirt ausgewählt aus der Gruppe der Bakterien, Hefen und tierischen Zellen züchtet, der durch ein nach dem Verfahren nach einem der Ansprüche 6 bis 8 hergestelltes rekombinantes DNA-Molekül transformiert worden ist, und das Polypeptid sammelt.

17. Verfahren nach einem der Ansprüche 10, 11 und 16, dadurch gekennzeichnet, daß das Polypeptid ausgewählt ist aus Polypeptiden der Formel:

MetAlaSerProPheAlaLeuLeuMetValLeuValValLeuSerCysLysSerSer

CysSerLeuGlyCysAspLeuProGluThrHisSerLeuAspAsnArgArgThrLeu

MetLeuLeuAlaGlnMetSerArgIleSerProSerSerCysLeuMetAspArgHis

AspPheGlyPheProGlnGluGluPheAspGlyAsnGlnPheGlnLysAlaProAla

IleSerValLeuHisGluLeuIleGlnGlnIlePheAsnLeuPheThrThrLysAsp

SerSerAlaAlaTrpAspGluAspLeuLeuAspLysPheCysThrGluLeuTyrGln

GlnLeuAsnAspLeuGluAlaCysValMetGlnGluGluArgValGlyGluThrPro

LeuMetAsnAlaAspSerIleLeuAlaValLysLysTyrPheArgArgIleThrLeu

TyrLeuThrGluLysLysTyrSerProCysAlaTrpGluValValArgAlaGluIle

MetArgSerLeuSerLeuSerThrAsnLeuGlnGluArgLeuArgArgLysGlu   und

CysAspLeuProGluThrHisSerLeuAspAsnArgArgThrLeuMetLeuLeu

AlaGlnMetSerArgIleSerProSerSerCysLeuMetAspArgHisAspPheGly

PheProGlnGluGluPheAspGlyAsnGlnPheGlnLysAlaProAlaIleSerVal

LeuHisGluLeuIleGlnGlnIlePheAsnLeuPheThrThrLysAspSerSerAla

AlaTrpAspGluAspLeuLeuAspLysPheCysThrGluLeuTyrGlnGlnLeuAsn

AspLeuGluAlaCysValMetGlnGluGluArgValGlyGluThrProLeuMetAsn

AlaAspSerIleLeuAlaValLysLysTyrPheArgArgIleThrLeuTyrLeuThr

GluLysLysTyrSerProCysAlaTrpGluValValArgAlaGluIleMetArgSer

LeuSerLeuSerThrAsnLeuGlnGluArgLeuArgArgLysGlu.

18. Verfahren nach einem der Ansprüche 10, 11 oder 16, dadurch gekennzeichnet, daß das Polypeptid ausgewählt ist aus Polypeptiden der Formel:

MetAlaLeuSerPheSerLeu

LeuMetAlaValLeuValLeuSerTyrLysSerIleCysSerLeuGlyCysAspLeu

ProGlnThrHisSerLeuGlyAsnArgArgThrLeuIleLeuLeuGlnGlnMetGly

ArgIleSerHisPheSerCysLeuLysAspArgHisAspPheGlyPheProGluGlu

GluPheAspGlyHisGlnPheGlnLysThrGlnAlaIleSerValLeuHisGluMet

IleGlnGlnThrPheAsnLeuPheSerThrGluAspSerSerAlaAlaTrpGluGln

SerLeuLeuGluLysPheSerThrGluLeuTyrGlnGlnLeuAsnAspLeuGluAla

CysValIleGlnGluValGlyValGluGluThrProLeuMetAsnValAspSerIle

LeuAlaValArgLysTyrPheGlnArgIleThrLeuTyrLeuThrGluLysLysTyr

SerProCysAlaTrpGluValValArgAlaGluIleMetArgSerLeuSerPheSer

ThrAsnLeuGlnLysArgLeuArgArgLysAsp  und  CysAspLeuProGlnThr

HisSerLeuGlyAsnArgArgThrLeuIleLeuLeuGlnGlnMetGlyArgIleSer

HisPheSerCysLeuLysAspArgHisAspPheGlyPheProGluGluGluPheAsp

GlyHisGlnPheGlnLysThrGlnAlaIleSerValLeuHisGluMetIleGlnGln

ThrPheAsnLeuPheSerThrGluAspSerSerAlaAlaTrpGluGlnSerLeuLeu

GluLysPheSerThrGluLeuTyrGlnGlnLeuAsnAspLeuGluAlaCysValIle

GlnGluValGlyValGluGluThrProLeuMetAsnValAspSerIleLeuAlaVal

ArgLysTyrPheGlnArgIleThrLeuTyrLeuThrGluLysLysTyrSerProCys

AlaTrpGluValValArgAlaGluIleMetArgSerLeuSerPheSerThrAsnLeu

GlnLysArgLeuArgArgLysAsp.

19. Verfahren zur Auswahl einer ein Polypeptid des IFN-α-Typs codierenden DNA-Sequenz aus einer Gruppe von DNA-Sequenzen, dadurch gekennzeichnet, daß festgestellt wird, welche der DNA-Sequenzen mit einer DNA-Sequenz hybridisiert, die ein rekombinantes DNA-Molekül kennzeichnet, das nach dem Verfahren nach einem der Ansprüche 1 bis 8 hergestellt worden ist.

20. Verfahren nach Anspruch 19, dadurch gekennzeichnet, daß die ermittelte DNA-Sequenz ausgewählt ist aus DNA-Sequenzen aus natürlichen Quellen, synthetischen DNA-Sequenzen, DNA-Sequenzen aus rekombinanten DNA-Molekülen und DNA-Sequenzen, die eine Kombination irgendwelcher der vorgenannten DNA-Sequenzen sind.

**Revendications**

**Revendications pour les Etats contractants suivants : BE, CH, DE, FR, GB, IT, LI, LU, NL, SE**

1. Molécule d'ADN recombinante destinée à cloner une séquence d'ADN dans des bactéries, des levures ou des cellules animales, la molécule d'ADN recombinante étant caractérisée en ce qu'elle est constituée par une séquence d'ADN sélectionnée parmi les :
   (a) les inserts d'ADN de Z-pBR322(Pst)/HcIF-4c, Z- pBR322(Pst)/HcIF-2h, Zp-BR322(Pst)/HcIF-SN35, Z- pBR322(Pst)/HcIF-SN42 et Z-pKT287 (Pst)/HcIF-2h- AH6, ces inserts d'ADN étant les inserts d'ADN de molécules d'ADN recombinantes portés par les microorganismes identifiés par les N° DMS 1699-1703, respectivement,
   (b) des séquences d'ADN qui produisent par hybridation l'un quelconque des inserts d'ADN précédents, et qui codent pour un polypeptide du type IFN-α, et
   (c) des séquences d'ADN qui sont des éléments dégénérés, résultant du code génétique pour les séquences et inserts d'ADN définis en (a) et (b), et qui codent pour un polypeptide du type IFN-α.

2. Molécule recombinante d'ADN selon la revendication 1, caractérisée en ce que la séquence d'ADN (b) qui donne par hybridation l'insert d'ADN (a), est sélectionnée parmi :

(d) les inserts d'ADN de Z-pBR322(Pst)/HcIF-II-206 et Z-pBR322(Pst)/HcIF-SN35-AHL6, ces inserts d'ADN étant des inserts d'ADN de molécules d'ADN recombinantes portés par les microorganismes identifiés par les numéros ATCC 31633-31634, respectivement,

(e) des séquences d'ADN qui produisent par hybridation l'un quelconque des inserts d'ADN précèdents et qui codent pour un polypeptide du type IFN-α, et

(f) des séquences d'ADN qui sont des éléments dégénérés résultant du code génétique sur les séquences et inserts d'ADN définis en (d) et (e) et qui codent pour un polypeptide du type IFN-α.

3. Molécule recombinante d'ADN selon l'une des revendications 1 ou 2, caractérisée en ce que la séquence d'ADN (b) ou (e) qui produit par hybridation l'insert d'ADN (a) ou (d), est sélectionnée parmi les :

(g) les portions d'hybridation de chacun des segments de Hchr IF-A, le fragment sous-cloné Hind III de chr 3 dans E. Coli HB 101 (DSM 1914); Hchr IF-B, le fragment sous-cloné Eco RI de chr 12 dans E. Coli HB 101 (DSM 1915) ; Hchr IF-C, le fragment sous-cloné Hind III de Chr 12 dans E. Coli HB 101 (DSM 1916) ; Hchr IF-D, le fragment sous-cloné Ecori de Chr 13 dans E. Coli HB 101 (DSM 1917) ; Hchr IF.E, le fragment sous-cloné Eco RI de chr 23 dans E. Coli HB 101 (DSM 1918) ; Hchr IF-F, le fragment sous-cloné Hind III de Chr 23 dans E. Coli HB 101 (DSM 1919) ; Hchr IF-G le fragment sous cloné Eco RI de Chr 26 dans E. Coli HB 101 (DSM 1920) ;

et Hchr IF-H le fragment sous-cloné Hind III de chr 26 dans E. Coli HB 101 (DSM 1921).

4. Molécule recombinante d'ADN selon la revendication 1, caractérisée en ce que cette séquence d'ADN est choisie parmi les séquences d'ADN ayant les formules :

```
            ATGGCCTCGCCCTTTG
CTTTACTGATGGTCCTGGTGGTGCTCAGCTGCAAGTCAAGCTGCTCTCTGGG
CTGTGATCTCCCTGAGACCCACAGCCTGGATAACAGGAGGACCTTGATGCTC
CTGGCACAAATGAGCAGAATCTCTCCTTCCTCCTGTCTGATGGACAGACATG
ACTTTGGATTTCCCCAGGAGGAGTTTGATGGCAACCAGTTCCAGAAGGCTCC
AGCCATCTCTGTCCTCCATGAGCTGATCCAGCAGATCTTCAACCTCTTTACC
ACAAAAGATTCATCTGCTGCTTGGGATGAGGACCTCCTAGACAAATTCTGCA
CCGAACTCTACCAGCAGCTGAATGACTTGGAAGCCTGTGTGATGCAGGAGGA
```

```
GAGGGTGGGAGAAACTCCCCTGATGAATGCGGACTCCATCTTGGCTGTGAAG
AAATACTTCCGAAGAATCACTCTCTATCTGACAGAGAAGAAATACAGCCCTT
GTGCCTGGGAGGTTGTCAGAGCAGAAATCATGAGATCCCTCTCTTTATCAAC
AAACTTGCAAGAAAGATTAAGGAGGAAGGAA  et  TGTGATCTCCCTGAGA
CCCACAGCCTGGATAACAGGAGGACCTTGATGCTCCTGGCACAAATGAGCAG
AATCTCTCCTTCCTCCTGTCTGATGGACAGACATGACTTTGGATTTCCCCAG
GAGGAGTTTGATGGCAACCAGTTCCAGAAGGCTCCAGCCATCTCTGTCCTCC
ATGAGCTGATCCAGCAGATCTTCAACCTCTTTACCACAAAAGATTCATCTGC
TGCTTGGGATGAGGACCTCCTAGACAAATTCTGCACCGAACTCTACCAGCAG
CTGAATGACTTGGAAGCCTGTGTGATGCAGGAGGAGAGGGTGGGAGAAACTC
CCCTGATGAATGCGGACTCCATCTTGGCTGTGAAGAAATACTTCCGAAGAAT
CACTCTCTATCTGACAGAGAAGAAATACAGCCCTTGTGCCTGGGAGGTTGTC
AGAGCAGAAATCATGAGATCCCTCTCTTTATCAACAAACTTGCAAGAAAGAT
TAAGGAGGAAGGAA .
```

**5.** Une molécule d'ADN recombinante selon la revendication 3, caractérisée en ce que cette séquence d'ADN est choisie parmi les séquences d'ADN, de formule :

```
                     ATGGCCCTGTCCTTTT                                    |
      CTTTACTGATGGCCGTGCTGGTGCTCAGCTACAAATCCATCTGTTCTCTGGG
      CTGTGATCTGCCTCAGACCCACAGCCTGGGTAATAGGAGGACCTTGATACTC
      CTGCAACAAATGGGAAGAATCTCTCATTTCTCCTGCCTGAAGGACAGACATG
      ATTTCGGATTCCCCGAGGAGGAGTTTGATGGCCACCAGTTCCAGAAGACTCA
      AGCCATCTCTGTCCTCCATGAGATGATCCAGCAGACCTTCAATCTCTTCAGCA
      CAGAGGACTCATCTGCTGCTTGGGAACAGAGCCTCCTAGAAAAATTTTCCACT
      GAACTTTACCAGCAACTGAATGACCTGGAAGCATGTGTGATACAGGAGGTTGG
      GGTGGAAGAGACTCCCCTGATGAATGTGGACTCCATCCTGGCTGTGAGGAAAT
      ACTTCCAAAGAATCACTCTTTATCTAACAGAGAAGAAATACAGCCCTTGTGCC
      TGGGAGGTTGTCAGAGCAGAAATCATGAGATCCCTCTCGTTTTCAACAAACTT
      GCAAAAAAGATTAAGGAGGAAGGAT    et  TGTGATCTGCCTCAGACCCACAG
      CCTGGGTAATAGGAGGACCTTGATACTCCTGCAACAAATGGGAAGAATCTCTC
      ATTTCTCCTGCCTGAAGGACAGACATGATTTCGGATTCCCCGAGGAGGAGTTT
      GATGGCCACCAGTTCCAGAAGACTCAAGCCATCTCTGTCCTCCATGAGATGAT
      CCAGCAGACCTTCAATCTCTTCAGCACAGAGGACTCATCTGCTGCTTGGGAAC
      AGAGCCTCCTAGAAAAATTTTCCACTGAACTTTACCAGCAACTGAATGACCTG
      GAAGCATGTGTGATACAGGAGGTTGGGGTGGAAGAGACTCCCCTGATGAATGT
```

```
      GGACTCCATCCTGGCTGTGAGGAAATACTTCCAAAGAATCACTCTTTATCTAA
      CAGAGAAGAAATACAGCCCTTGTGCCTGGGAGGTTGTCAGAGCAGAAATCATG
      AGATCCCTCTCGTTTTCAACAAACTTGCAAAAAAGATTAAGGAGGAAGGAT .
```

**6.** Molécule d'ADN recombinante selon l'une quelconque des revendications 1 à 5, destinée à être utilisée pour exprimer cette séquence d'ADN clonée dans des bactéries, des levures ou des cellules animales, caractérisée en ce que la séquence d'ADN est fonctionnellement reliée à une séquence de contrôle de l'expression dans la molécule recombinante d'ADN.

**7.** Molécule d'ADN recombinante selon la revendication 6, caractérisée en ce que la séquence de contrôle de l'expression est sélectionnée entre un système lac, un système β-lac, et un système trp, les principales régions d'opération et de promotion de phage λ, la région de contrôle de la protéine de revêtement fd, et d'autres séquences qui commandent l'expression des gènes dans les bactéries, levures et cellules animales.

**8.** Molécule d'ADN recombinante selon les revendications 6 ou 7, caractérisée en ce qu'elle est sélectionnée parmi C8-IFN-α2, LAC-AUG(α2) et β-lac-AUG(α2), ces molécules recombinantes d'ADN étant des molécules recombinantes d'ADN portées par les microorganismes identifiés par les numéros ATCC 31767-31769.

**9.** Hôte transformé avec au moins une molécule recombinante d'ADN selon l'une quelconque des revendications 1 à 8, cet hôte étant sélectionné parmi les bactéries, levures et cellules animales.

**10.** Hôte transformé selon la revendication 9, caractérisé en ce que les bactéries, levures et cellules animales sont choisis parmi les souches de E. Coli, Pseudomonas, Bacillus subtilis, Bacillus stearothermophilus, d'autres bacilles et des cellules de souris.

11. Hôte transformé selon la revendication 10, sélectionné parmi les :

E. Coli HB101 (Z-pBR322(Pst)/HcIF-4c) [DSM 1699],

E. Coli HB101 (Z-pBR322(Pst)/HcIF-2h) [DSM 1700],

E. Coli HB101 (Z-pBR322(Pst)/HcIF-SN35) [DSM 1701],

E. Coli HB101 (Z-pBR322(Pst)/HcIF-SN42 [DSM 1702] et

E. COLI HB101 (Z-pKT287(Pst)/HcIF-2h-AH6) [DSM 1703].

12. Hôte transformé selon la revendication 10, sélectionné parmi les E. Coli HB101 (Z- pBR322(Pst)/HcIF-II-206) [ATCC 31633] et E. Coli HB101 (Z-pBR322(Pst)/HcIF-SN35-AHL6) [ATCC 31634].

13. Hôte transformé selon la revendication 10, sélectionné parmi les HchrIF-A [DSM 1914], HchrIF- B [DSM 1915], HchrIF-C [DSM 1916], HchrIF-D [DSM 1917], HchrIF-E [DSM 1918], HchrIF-F [DSM 1919], HchrIF-G [DSM 1920] et HchrIF-H [DSM 1921].

14. Hôte transformé selon la revendication 10, sélectionné parmi les E. Coli DS410 (C8-IFN-α2) [ATCC 31767], E. Coli DS410 (LAC-AUG(α2)) [ATCC 31768] et E. Coli DS410 HB101 (β-lac-AUG(α2)) [ATCC 31769].

15. Un procédé de production d'un polypeptide du type IFN-α qui comprend la mise en culture d'un hôte transformé selon l'une quelconque des revendications 9 à 14, pour autant que ces revendications dépendent des revendications 6 à 8, et à recueillir ce polypeptide.

16. Un polypeptide du type IFN-α produit selon le procédé de la revendication 15, choisi parmi les polypeptides ayant la formule :

```
                                              METALASER
PROPHEALALEULEUMETVALLEUVALVALLEUSERCYSLYSSERSERCYSSER
LEUGLYCYSASPLEUPROGLUTHRHISSERLEUASPASNARGARGTHRLEUMET
LEULEUALAGLNMETSERARGILESERPROSERSERCYSLEUMETASPARGHIS
ASPPHEGLYPHEPROGLNGLUGLUPHEASPGLYASNGLNPHEGLNLYSALAPRO
ALAILESERVALLEUHISGLULEUILEGLNGLNILEPHEASNLEUPHETHRTHR
```

```
LYSASPSERSERALAALAATRPASPGLUASPLEULEUASPLYSPHECYSTHRGLU
LEUTYRGLNGLNLEUASNASPLEUGLUALACYSVALMETGLNGLUGLUARGVAL
GLYGLUTHRHRPROLEUMETASNALAASPSERILELEUALAVALLYSLYSTYRPHE
ARGARGILETHRLEUTYRLEUTHRHRGLULYSLYSTYRSERPROCYSSALATRPGLU
VALVALARGALAGLUILEMETARGSERLEUSERLEUSERTHRASNLEUGLNGLU
ARGLEUARGARGGLYSGLU    et  CYSASPLEUPROGLUTHRHISSERLEUASPA
SNARGARGTHRLEUMETLEULEUALAGLNMETSERARGILESERPROSERSERC
YSLEUMETASPARGHISASPPHEGLYPHEPROGLNGLUGLUPHEASPGLYASNG
LNPHEGLNLYSALAPROALAAILESERVALLEUEUHISGLULEUILEGLNGLNILEP
HEASNLEUPHETHRTHRLYSASPSERSERALAALAATRPASPGLUASPLEULEUA
SPLYSPHECYSTHRGLULEUTYRGLNGLNLNEUASNASPLEUGLUGLUALACYSVALM
ETGLNGLUGLUARGVALGLYGLUTHRHRPROLEUMETASNALAASPSERILELEUA
LAVALLYSLYSTYRPHEARGARGILETHRHRLEUTYRLEUTHRHRGLULYSLYSTYRS
ERPROCYSSALATRPGLUVALVALARGALAGLUILEMETARGSERLEUSERLEUS
ERTHRASNLEUGLNGLNGLUARGLEUARGARGGLYSGLU.
```

17. Polypeptide du type IFN-α produit selon le procédé de la revendication 15, choisi parmi les polypeptides

de formule :

```
METALALEUSERPHESERLEULEUMETALAVALLEUVALLEUSERTYRLYS
SERILECYSSERLEUGLYCYSASPLEUPROGLNTHRHISSERLEUGLYASN
ARGARGTHRLEUILELEULEUGLNGLNMETGLYARGILESERHISPHESER
CYSLEULYSASPARGHISASPPHEGLYPHEPROGLUGLUGLUPHEASPGLY
HISGLNPHEGLNLYSTHRGLNALAILESERVALLEUHISGLUMETILEGLN
GLNTHRPHEASNLEUPHESERTHRGLUASPSERSERALAALATRPGLUGLN
SERLEULEUGLULYSPHESERTHRGLULEUTYRGLNGLNLEUASNASPLEU
GLUALACYSVALILEGLNGLUVALGLYVALGLUGLUTHRPROLEUMETASN
VALASPSERILELEUALAVALARGLYSTYRPHEGLNARGILETHRLEUTYR
LEUTHRGLULYSLYSTYRSERPROCYSALATRPGLUVALVALARGALAGLU
ILEMETARGSERLEUSERPHESERTHRASNLEUGLNLYSARGLEUARGARG
LYSASP  ET   CYSASPLEUPROGLNTHRHISSERLEUGLYASNARGARGT
HRLEUILELEULEUGLNGLNMETGLYARGILESERHISPHESERCYSLEUL
YSASPARGHISASPPHEGLYPHEPROGLUGLUGLUPHEASPGLYHISGLNP
HEGLNLYSTHRGLNALAILESERVALLEUHISGLUMETILEGLNGLNTHRP
HEASNLEUPHESERTHRGLUASPSERSERALAALATRPGLUGLNSERLEUL
EUGLULYSPHESERTHRGLULEUTYRGLNGLNLEUASNASPLEUGLUALAC


YSVALILEGLNGLUVALGLYVALGLUGLUTHRPROLEUMETASNVALASPS
ERILELEUALAVALARGLYSTYRPHEGLNARGILETHRLEUTYRLEUTHRG
LULYSLYSTYRSERPROCYSALATRPGLUVALVALARGALAGLUILEMETA
RGSERLEUSERPHESERTHRASNLEUGLNLYSARGLEUARGARGLYSASP.
```

**18.** Procédé d'obtention d'une molécule recombinante d'ADN selon l'une quelconque des revendications 1 à 5, caractérisé en ce qu'il comprend l'étape d'introduction, dans un véhicule de clonage, une séquence d'ADN qui caractérise les molécules recombinante d'ADN selon l'une quelconque des revendications 1 à 5.

**19.** Procédé d'obtention d'une molécule recombinante d'ADN selon l'une quelconque des revendications 6 à 8, caractérisé en ce qu'il comprend les étapes consistant à introduire, dans un véhicule de clonage, une séquence d'ADN qui caractérise les molécules recombinante d'ADN selon l'une quelconque des revendications 1 à 5, et à introduire dans ce véhicule de clonage une séquence de contrôle de l'expression selon la revendication 7, cette séquence de contrôle de l'expression étant introduite dans le véhicule de clonage de façon à contrôler et réguler l'expression de la séquence d'ADN.

**20.** Procédé de transformation d'un hôte choisi parmi les bactéries, levures et les cellules animales, caractérisé en ce qu'il comprend l'étape consistant à introduire dans cet hôte une molécule recombinante d'ADN selon l'une quelconque des revendications 1 à 8.

**21.** Procédé d'obtention d'un polypeptide du type IFNα, caractérisé en ce qu'il comprend les étapes consistant à transformer un hôte sélectionné parmi les bactéries, les levures et les cellules animales, au moyen d'une molécule recombinante d'ADN selon l'une quelconque des revendications 6 à 8, à cultiver cet hôte et à recueillir ce polypeptide.

**22.** Procédé selon la revendication 21, caractérisé en ce que cet hôte est sélectionné parmi les souches de E. Coli, Pseudomonas, Bacillus subtilis, Bacillus stéarothermophilus et autre bacilles.

**23.** Procédé d'obtention d'un polypeptide du type IFN-α, caractérisé par les étapes de culture d'un hôte sélectionné parmi les bactéries, les levures et les cellules animales et transformé par une molécule recombinante d'ADN selon l'une quelconque des revendications 6 à 8, et à recueillir ce polypeptide.

24. Procédé de sélection d'une séquence d'ADN codant pour un polypeptide du type IFNα à partir d'un groupe de séquence d'ADN, caractérisé par l'étape qui consiste à déterminer la séquence d'ADN qui subit l'hybridation en une séquence d'ADN qui caractérise les molécules recombinantes d'ADN selon l'une quelconque des revendications 1 à 8.

25. Procédé selon la revendication 24, caractérisé en ce que la séquence d'ADN essayée est sélectionnée parmi les séquences d'ADN de sources naturelles, de séquences d'ADN synthétiques, de séquences d'ADN de molécules recombinantes d'ADN et de séquences d'ADN qui sont une combinaison quelconque des séquences d'ADN précédentes.

26. Composition pour le traitement de virus humains ou de traitement de cancers ou tumeurs humains, caractérisée en ce qu'elle comprend comme IFNα unique, un polypeptide choisi parmi un polypeptide selon l'une quelconque des revendications 16 à 17 ou produit selon le procédé de l'une quelconque des revendications 15, 21, 22 et 23.

27. Composition pour le traitement des infections virales bovines, caractérisée en ce qu'elle comprend comme IFN-α unique, un polypeptide choisi parmi un polypeptide selon l'une quelconque des revendications 16 à 17 ou produit selon le procédé de l'une quelconque des revendications 15, 21, 22 et 23.

28. Séquence d'ADN sélectionnée parmi les séquences d'ADN de formule :

```
                                        ATGGCCTCGCCCTTTGCTTTACTGA
TGGTCCTGGTGGTGCTCAGCTGCAAGTCAAGCTGCTCTCTGGGCTGTGATCT
CCCTGAGACCCACAGCCTGGATAACAGGAGGACCTTGATGCTCCTGGCACAA
ATGAGCAGAATCTCTCCTTCCTCCTGTCTGATGGACAGACATGACTTTGGAT
TTCCCCAGGAGGAGTTTGATGGCAACCAGTTCCAGAAGGCTCCAGCCATCTC
TGTCCTCCATGAGCTGATCCAGCAGATCTTCAACCTCTTTACCACAAAAGAT
TCATCTGCTGCTTGGGATGAGGACCTCCTAGACAAATTCTGCACCGAACTCT
ACCAGCAGCTGAATGACTTGGAAGCCTGTGTGATGCAGGAGGAGAGGGTGGG
AGAAACTCCCCTGATGAATGCGGACTCCATCTTGGCTGTGAAGAAATACTTC
CGAAGAATCACTCTCTATCTGACAGAGAAGAAATACAGCCCTTGTGCCTGGG
AGGTTGTCAGAGCAGAAATCATGAGATCCCTCTCTTTATCAACAAACTTGCA
AGAAAGATTAAGGAGGAAGGAA  ET TGTGATCTCCCTGAGACCCACAGCC
TGGATAACAGGAGGACCTTGATGCTCCTGGCACAAATGAGCAGAATCTCTCC
TTCCTCCTGTCTGATGGACAGACATGACTTTGGATTTCCCCAGGAGGAGTTT
GATGGCAACCAGTTCCAGAAGGCTCCAGCCATCTCTGTCCTCCATGAGCTGA
TCCAGCAGATCTTCAACCTCTTTACCACAAAAGATTCATCTGCTGCTTGGGA
TGAGGACCTCCTAGACAAATTCTGCACCGAACTCTACCAGCAGCTGAATGAC
TTGGAAGCCTGTGTGATGCAGGAGGAGAGGGTGGGAGAAACTCCCCTGATGA
ATGCGGACTCCATCTTGGCTGTGAAGAAATACTTCCGAAGAATCACTCTCTA
TCTGACAGAGAAGAAATACAGCCCTTGTGCCTGGGAGGTTGTCAGAGCAGAA
ATCATGAGATCCCTCTCTTTATCAACAAACTTGCAAGAAAGATTAAGGAGGA
AGGAA,
```

cette séquence d'ADN étant destinée au clonage ou à l'expression de séquence dans les bactéries, les levures et les cellules animales et à la sélection de séquences d'ADN qui codent pour un polypeptide du type IFN-α.

**29.** Séquence d'ADN sélectionnée parmi les séquences d'ADN de formule:

```
                                    ATGGCCCTGTCCTTTTCTTTACTGA
        TGGCCGTGCTGGTGCTCAGCTACAAATCCATCTGTTCTCTGGGCTGTGATCT
        GCCTCAGACCCACAGCCTGGGTAATAGGAGGACCTTGATACTCCTGCAACAA


        ATGGGAAGAATCTCTCATTTCTCCTGCCTGAAGGACAGACATGATTTCGGAT
        TCCCCGAGGAGGAGTTTGATGGCCACCAGTTCCAGAAGACTCAAGCCATCTC
        TGTCCTCCATGAGATGATCCAGCAGACCTTCAATCTCTTCAGCACAGAGGAC
        TCATCTGCTGCTTGGGAACAGAGCCTCCTAGAAAAATTTTCCACTGAACTTT
        ACCAGCAACTGAATGACCTGGAAGCATGTGTGATACAGGAGGTTGGGGTGGA
        AGAGACTCCCCTGATGAATGTGGACTCCATCCTGGCTGTGAGGAAATACTTC
        CAAAGAATCACTCTTTATCTAACAGAGAAGAAATACAGCCCTTGTGCCTG
        GGAGGTTGTCAGAGCAGAAATCATGAGATCCCTCTCGTTTTCAACAAACTTG
        CAAAAAAGATTAAGGAGGAAGGAT ET TGTGATCTGCCTCAGACCCACAG
        CCTGGGTAATAGGAGGACCTTGATACTCCTGCAACAAATGGGAAGAATCTCT
        CATTTCTCCTGCCTGAAGGACAGACATGATTTCGGATTCCCCGAGGAGGAGT
        TTGATGGCCACCAGTTCCAGAAGACTCAAGCCATCTCTGTCCTCCATGAGAT
        GATCCAGCAGACCTTCAATCTCTTCAGCACAGAGGACTCATCTGCTGCTTGG
        GAACAGAGCCTCCTAGAAAAATTTTCCACTGAACTTTACCAGCAACTGAATG
        ACCTGGAAGCATGTGTGATACAGGAGGTTGGGGTGGAAGAGACTCCCCTGAT
        GAATGTGGACTCCATCCTGGCTGTGAGGAAATACTTCCAAAGAATCACTCTT
        TATCTAACAGAGAAGAAATACAGCCCTTGTGCCTGGGAGGTTGTCAGAGCAG
        AAATCATGAGATCCCTCTCGTTTTCAACAAACTTGCAAAAAAGATTAAGGAG
        GAAGGAT,
```

séquence d'ADN étant utilisée pour le clonage ou l'expression de cette séquence dans des bactéries, les levures et les cellules animales, et dans la sélection de séquences d'ADN qui codent pour un polypeptide du type IFN-α.

**Revendications pour l'Etat contractant suivant : AT**

**1.** Procédé de production d'une molécule d'ADN recombinante destinée à cloner une séquence d'ADN dans des bactéries, des levures ou des cellules animales, la molécule d'ADN recombinante étant caractérisée en ce qu'elle comprend le stade dans lequel on introduit dans un véhicule de clonage, une séquence d'ADN sélectionnée parmi les :

(a) les inserts d'ADN de Z-pBR322(Pst)/HcIF-4c, Z-pBR322(Pst)/HcIF-2h, Z-pBR322(Pst)/HcIF-SN35, Z-pBR322(Pst)/HcIF-SN42 et Z-pKT287 (Pst)/HcIF- 2h-AH6, ces inserts d'ADN étant les inserts d'ADN de molécules d'ADN recombinantes portés par les microorganismes identifiés par les N° DMS 1699-1703, respectivement,

(b) des séquences d'ADN qui produisent par hybridation l'un quelconque des inserts d'ADN précédents, et qui codent pour un polypeptide du type IFN-α, et

(c) des séquences d'ADN qui sont des éléments dégénérés, résultant du code génétique pour les séquences et inserts d'ADN définis en (a) et (b), et qui codent pour un polypeptide du type IFN-α.

**2.** Procédé selon la revendication 1, caractérisé en ce que la séquence d'ADN (b) qui donne par hybridation l'insert d'ADN (a), est sélectionnée parmi :

(d) les inserts d'ADN de Z-pBR322(Pst)/HcIF-II-206 et Z-pBr322(Pst)/HcIF-SN35-AHL6, ces inserts d'ADN étant des inserts d'ADN de molécules d'ADN recombinantes portés par les microorganismes identifiés par les numéros ATCC 31633-31634, respectivement,

(e) des séquences d'ADN qui produisent par hybridation l'un quelconque des inserts d'ADN précédents et qui codent un polypeptide du type IFN-α, et

(f) des séquences d'ADN qui sont les éléments dégénérés résultant du code génétique sur les séquences et inserts d'ADN définis en (d) et (e) et qui codent pour un polypeptide du type IFN-α.

3. Procédé selon les revendications 1 ou 2, caractérisé en ce que la séquence d'ADN (b) ou (e) qui produit par hybridation l'insert d'ADN (a) ou (d), est sélectionnée parmi les :

(g) les portions d'hybridation de chacun des segments de Hchr IF-A, le fragment sous-cloné Hind III de chr 3 dans E. Coli HB 101 (DSM 1914) ; Hchr IF-B, le fragment sous-cloné Eco RI de chr 12 dans E. Coli HB 101 (DSM 1915) ; Hchr IF-C, le fragment sous-cloné Hind III de Chr 12 dans E. Coli HB 101 (DSM 1916) ; Hchr IF-D, le fragment sous-cloné Ecori de Chr 13 dans E. Coli HB 101 (DSM 1917) ; Hchr IF.E, le fragment sous-cloné Eco RI de chr 23 dans E. Coli HB 101 (DSM 1918) ; Hchr IF-F, le fragment sous-cloné Hind III de Chr 23 dans E. Coli HB 101 (DSM 1919) ; Hchr IF-G le fragment sous cloné Eco RI de Chr 26 dans E. Coli HB 101 (DSM 1920) ;

et Hchr IF-H le fragment sous-cloné Hind III de chr 26 dans E. Coli HB 101 (DSM 1921).

4. Procédé selon la revendication 1, caractérisé en ce que cette séquence d'ADN est choisie parmi les séquences d'ADN ayant la formule :

```
                                    ATGGCCTCGCCCTTTG
CTTTACTGATGGTCCTGGTGGTGCTCAGCTGCAAGTCAAGCTGCTCTCTGGG
CTGTGATCTCCCTGAGACCCACAGCCTGGATAACAGGAGGACCTTGATGCTC
CTGGCACAAATGAGCAGAATCTCTCCTTCCTCCTGTCTGATGGACAGACATG
ACTTTGGATTTCCCCAGGAGGAGTTTGATGGCAACCAGTTCCAGAAGGCTCC
AGCCATCTCTGTCCTCCATGAGCTGATCCAGCAGATCTTCAACCTCTTTACC
```

```
ACAAAAGATTCATCTGCTGCTTGGGATGAGGACCTCCTAGACAAATTCTGCA
CCGAACTCTACCAGCAGCTGAATGACTTGGAAGCCTGTGTGATGCAGGAGGA
GAGGGTGGGAGAAACTCCCCTGATGAATGCGGACTCCATCTTGGCTGTGAAG
AAATACTTCCGAAGAATCACTCTCTATCTGACAGAGAAGAAATACAGCCCTT
GTGCCTGGGAGGTTGTCAGAGCAGAAATCATGAGATCCCTCTCTTTATCAAC
AAACTTGCAAGAAAGATTAAGGAGGAAGGAA   et  TGTGATCTCCCTGAGA
CCCACAGCCTGGATAACAGGAGGACCTTGATGCTCCTGGCACAAATGAGCAG
AATCTCTCCTTCCTCCTGTCTGATGGACAGACATGACTTTGGATTTCCCCAG
GAGGAGTTTGATGGCAACCAGTTCCAGAAGGCTCCAGCCATCTCTGTCCTCC
ATGAGCTGATCCAGCAGATCTTCAACCTCTTTACCACAAAAGATTCATCTGC
TGCTTGGGATGAGGACCTCCTAGACAAATTCTGCACCGAACTCTACCAGCAG
CTGAATGACTTGGAAGCCTGTGTGATGCAGGAGGAGAGGGTGGGAGAAACTC
CCCTGATGAATGCGGACTCCATCTTGGCTGTGAAGAAATACTTCCGAAGAAT
CACTCTCTATCTGACAGAGAAGAAATACAGCCCTTGTGCCTGGGAGGTTGTC
AGAGCAGAAATCATGAGATCCCTCTCTTTATCAACAAACTTGCAAGAAAGAT
TAAGGAGGAAGGAA.
```

5. Procédé selon la revendication 3, caractérisé en ce que cette séquence d'ADN est choisie parmi les séquences d'ADN de formule

```
                                        ATGGCCCTGTCCTTT
TCTTTACTGATGGCCGTGCTGGTGCTCAGCTACAAATCCATCTGTTCTCTGGG
CTGTGATCTGCCTCAGACCCACAGCCTGGGTAATAGGAGGACCTTGATACTC
CTGCAACAAATGGGAAGAATCTCTCATTTCTCCTGCCTGAAGGACAGACATG
ATTTCGGATTCCCCGAGGAGGAGTTTGATGGCCACCAGTTCCAGAAGACTCA
AGCCATCTCTGTCCTCCATGAGATGATCCAGCAGACCTTCAATCTCTTCAGCA
CAGAGGACTCATCTGCTGCTTGGGAACAGAGCCTCCTAGAAAAATTTTCCACT
GAACTTTACCAGCAACTGAATGACCTGGAAGCATGTGTGATACAGGAGGTTGG
GGTGGAAGAGACTCCCCTGATGAATGTGGACTCCATCCTGGCTGTGAGGAAAT
ACTTCCAAAGAATCACTCTTTATCTAACAGAGAAGAAATACAGCCCTTGTGCC
TGGGAGGTTGTCAGAGCAGAAATCATGAGATCCCTCTCGTTTTCAACAAACTT
GCAAAAAAGATTAAGGAGGAAGGAT    et TGTGATCTGCCTCAGACCCACAG
CCTGGGTAATAGGAGGACCTTGATACTCCTGCAACAAATGGGAAGAATCTCTC
ATTTCTCCTGCCTGAAGGACAGACATGATTTCGGATTCCCCGAGGAGGAGTTT
GATGGCCACCAGTTCCAGAAGACTCAAGCCATCTCTGTCCTCCATGAGATGAT
CCAGCAGACCTTCAATCTCTTCAGCACAGAGGACTCATCTGCTGCTTGGGAAC
AGAGCCTCCTAGAAAAATTTTCCACTGAACTTTACCAGCAACTGAATGACCTG
```

```
GAAGCATGTGTGATACAGGAGGTTGGGGTGGAAGAGACTCCCCTGATGAATGT
GGACTCCATCCTGGCTGTGAGGAAATACTTCCAAAGAATCACTCTTTATCTAA
CAGAGAAGAAATACAGCCCTTGTGCCTGGGAGGTTGTCAGAGCAGAAATCATG
AGATCCCTCTCGTTTTCAACAAACTTGCAAAAAAGATTAAGGAGGAAGGAT .
```

**6.** Procédé selon l'une quelconque des revendications 1 à 5, caractérisé en ce que la séquence d'ADN est fonctionnellement reliée à une séquence de contrôle de l'expression dans la molécule recombinante d'ADN.

**7.** Procédé selon la revendication 6, caractérisé en ce que la séquence de contrôle de l'expression est sélectionnée parmi un système lac, un système β-lac, et un système trp, les principales régions d'opération et de promotion de phage λ, la région de contrôle de la protéine de revêtement fd, et d'autres séquences qui commandent l'expression des gènes dans les bactéries, levures et cellules animales.

**8.** Procédé selon les revendications 6 ou 7, caractérisé en ce que la molécule d'ADN recombinante produite est sélectionnée parmi :
C8-IFN-α2, LAC-AUG(α2) et β-lac-AUG(α2), ces molécules recombinantes d'ADN étant des molécules recombinantes d'ADN portées par les microorganismes identifiés par les numéros ATCC 31767-31769.

**9.** Procédé pour transformer un hôte sélectionné parmi les bactéries, les levures et les cellules animales, caractérisé par le fait qu'on introduit dans cet hôte une molécule d'ADN recombinante produite par le procédé de l'une quelconque des revendications 1 à 8.

**10.** Procédé de production d'un polypeptide du type IFN-α, caractérisé par les étapes de transformation d'un hôte choisi parmi les bactéries, les levures et les cellules animales, avec une molécule d'ADN recombinante produite par le procédé selon l'une quel conque des revendications 6 à 8, de mise en culture de cet hôte et de recueil de ce polypeptide.

**11.** Procédé selon la revendication 9 ou 10, caractérisé en ce que l'hôte non transformé est choisi parmi les souches de E. Coli, Pseudomonas, Bacillus subtilis, Bacillus stearothermophilus, d'autres bacilles et des cellules de souris.

12. Procédé selon la revendication 10 ou 11, caractérisé en ce que l'hôte transformé est sélectionné parmi les :

E. Coli HB101 (Z-pBR322(Pst)/HcIF-4c) [DSM 1699],
E. Coli HB101 (Z-pBR322(Pst)/HcIF-2h) [DSM 1700],
E. Coli HB101 (Z-pBR322(Pst)/HcIF-SN35) [DSM 1701],
E. Coli HB101 (Z-pBR322(Pst)/HcIF-SN42) [DSM 1702], et
E. Coli HB101 (Z-pKT287(Pst)/HcIF-2h-AH6) [DSM 1703].

13. Procédé selon la revendication 10 ou 11, caractérisé en ce que l'hôte transformé est sélectionné parmi les :

E. Coli HB101 (Z-pBR322(Pst)/HcIF-II-206)[ATCC 31633] et E. Coli HB101 (Z-pBR322(Pst)/HcIF-SN35-AHL6)[ATCC 31634].

14. Procédé selon la revendication 10 ou 11, caractérisé par le fait que l'hôte transformé est sélectionné parmi les HChrIF-A [DSM 1914], HChrIF-B [DSM 1915], HChrIF-C [DSM 1916], HChrIF-D [DSM 1917], HChrIF-E [DSM 1918], HChrIF-F [DSM 1919], HChrIF-G [DSM 1920] et HChrIF-H [DSM 1921].

15. Procédé selon la revendication 10 ou 11, caractérisé en ce que l'hôte transformé est sélectionné parmi les :

E. Coli DS410 (C8-IFN-$\alpha$2) [ATCC 31767].
E. Coli DS410 (LAC-AUG($\alpha$2)) [ATCC 31768], et
E. Coli DS410 ($\beta$-lac-AUG($\alpha$2)) [ATCC 31769].

16. Procédé de production d'un polypeptide du type IFN-$\alpha$ qui comprend la mise en culture d'un hôte choisi parmi les bactéries, les levures et les cellules animales et transformé par une molécule d'ADN recombinante produite par le procédé selon l'une quelconque des revendications 6 à 8 et le recueil de ce polypeptide.

17. Procédé selon l'une quelconque des revendications 10, 11 et 16, caractérisé en ce que le polypeptide est choisi parmi les polypeptides ayant la formule

```
METALASER
PROPHEALALEULEUMETVALLEUVALVALLEUSERCYSLYSSERSERCYSSER
LEUGLYCYSASPLEUPROGLUTHRHISSERLEUASPASNARGARGTHRLEUMET
LEULEUALAGLNMETSERARGILESERPROSERSERCYSLEUMETASPARGHIS
ASPPHEGLYPHEPROGLNGLUGLUPHEASPGLYASNGLNPHEGLNLYSALAPRO
ALAILESERVALLEUHISGLULEUILEGLNGLNILEPHEASNLEUPHETHRTHR
LYSASPSERSERALAALATRPASPGLUASPLEULEUASPLYSPHECYSTHRGLU
LEUTYRGLNGLNLEUASNASPLEUGLUALACYSVALMETGLNGLUGLUARGVAL
GLYGLUTHRPROLEUMETASNALAASPSERILELEUALAVALLYSLYSTYRPHE
ARGARGILETHRLEUTYRLEUTHRGLULYSLYSTYRSERPROCYSALATRPGLU
VALVALARGALAGLUILEMETARGSERLEUSERLEUSERTHRASNLEUGLNGLU
ARGLEUARGARGLYSGLU  et   CYSASPLEUPROGLUTHRHISSERLEUASPA
SNARGARGTHRLEUMETLEULEUALAGLNMETSERARGILESERPROSERSERC
YSLEUMETASPARGHISASPPHEGLYPHEPROGLNGLUGLUPHEASPGLYASNG
LNPHEGLNLYSALAPROALAILESERVALLEUHISGLULEUILEGLNGLNILEP
HEASNLEUPHETHRTHRLYSASPSERSERALAALATRPASPGLUASPLEULEUA
SPLYSPHECYSTHRGLULEUTYRGLNGLNLEUASNASPLEUGLUALACYSVALM
ETGLNGLUGLUARGVALGLYGLUTHRPROLEUMETASNALAASPSERILELEUA
LAVALLYSLYSTYRPHEARGARGILETHRLEUTYRLEUTHRGLULYSLYSTYRS
ERPROCYSALATRPGLUVALVALARGALAGLUILEMETARGSERLEUSERLEUS
ERTHRASNLEUGLNGLUARGLEUARGARGLYSGLU .
```

18. Procédé selon l'une quelconque des revendications 10, 11 et 16, caractérisé en ce que le polypeptide est choisi parmi les polypeptides de formule

```
METALALEUSERPHESERLEULEUMETALAVALLEUVALLEUSERTYRLYS
SERILECYSSERLEUGLYCYSASPLEUPROGLNTHRHISSERLEUGLYASN
```

```
ARGARGTHRLEUILELEULEUGLNGLNMETGLYARGILESERHISPHESER
CYSLEULYSASPARGHISASPPHEGLYPHEPROGLUGLUGLUPHEASPGLY
HISGLNPHEGLNLYSTHRGLNALAILESERVALLEUHISGLUMETILEGLN
GLNTHRPHEASNLEUPHESERTHRGLUASPSERSERALAALATRPGLUGLN
SERLEULEUGLULYSPHESERTHRGLULEUTYRGLNGLNLEUASNASPLEU
GLUALACYSVALILEGLNGLUVALGLYVALGLUGLUTHRPROLEUMETASN
VALASPSERILELEUALAVALARGLYSTYRPHEGLNARGILETHRLEUTYR
LEUTHRGLULULYSLYSTYRSERPROCYSALAATRPGLUVALVALARGALAGLU
ILEMETARGSERLEUSERPHESERTHRASNLEUGLNLYSARGLEUARGARG
LYSASP et   CYSASPLEUPROGLNTHRHISSERLEUGLYASNARGARGT
HRLEUILELEULEUGLNGLNMETGLYARGILESERHISPHESERCYSLEUL
YSASPARGHISASPPHEGLYPHEPROGLUGLUGLUPHEASPGLYHISGLNP
HEGLNLYSTHRGLNALAILESERVALLEUHISGLUMETILEGLNGLNTHRP
HEASNLEUPHESERTHRGLUASPSERSERALAALATRPGLUGLNSERLEUL
EUGLULYSPHESERTHRGLULEUTYRGLNGLNLEUASNASPLEUGLUALAC
YSVALILEGLNGLUVALGLYVALGLUGLUTHRPROLEUMETASNVALASPS
ERILELEUALAVALARGLYSTYRPHEGLNARGILETHRLEUTYRLEUTHRG
LULYSLYSTYRSERPROCYSALATRPGLUVALVALARGALAGLUILEMETA
RGSERLEUSERPHESERTHRASNLEUGLNLYSARGLEUARGARGLYSASP.
```

**19.** Procédé de sélection d'une séquence d'ADN codant pour un polypeptide du type IFN-α à partir d'un groupe de séquences d'ADN, caractérisé par l'étape qui consiste à déterminer la séquence d'ADN qui subit l'hybridation en une séquence d'ADN qui caractérise les molécules recombinantes d'ADN selon l'une quelconque des revendications 1 à 8.

**20.** Procédé selon la revendication 19, caractérisé en ce que la séquence d'ADN essayés est sélectionnée parmi les séquences d'ADN de sources naturelles, de séquences d'ADN synthétiques, de séquences d'ADN de molécules recombinantes d'ADN et de séquences d'ADN qui sont une combinaison quelconque des séquences d'ADN précédentes.

FIG.1

## FIG. 2

NEGATIVE ASSAY          POSITIVE ASSAY

# FIG.3

pBR322-Xc DNA

↓ $^{32}$P-Hif-4c Pst FRAGMENT

X-RAY
EXPOSURE

NEGATIVE RESPONSE

pBR322-IFc DNA

↓ $^{32}$P-Hif-4c Pst FRAGMENT

$^{32}$P-Hif-4c Pst
FRAGMENT

X-RAY
EXPOSURE

POSITIVE RESPONSE

# FIG.4

Eco RI (0)

Mbo II (4845)

Bsp I (4641)

Pst I (4496)

Bsp I (4410)

HcIF-2h

5'

Bgl II (4156)

Z-pBR 322 (Pst)/HcIF-2h

5246 bp

Mbo II (3961)

ECO RI (3820)

3'

Pst I (3611)

Bsp I (3489)

Mbo II (2345)

# FIG. 5

NOT FOUND

FOUND

```
                                  10                              20
      MetSerIleGlnHisPheArgValAlaLeuIleProPhePheAlaAlaPheCysLeuProValPheAlaHisProGluThr
pBR322 ATGAGTATTCAACATTTCCGTGTCGCCCTTATTCCCTTTTTTGCGGCATTTTGCCTTCCTGTTTTTGCTCACCCAGAAACG
      29    101
      LeuVal   ProAlaAlaMet
      CTGGTG···CCTGCAGCAATG···
                ―――
                Pst


                                                                      24
      MetSerIleGlnHisPheArgValAlaLeuIleProPhePheAlaAlaPheCysLeuProValPheAlaHisArgCysSerAsn
pKT279 ATGAGTATTCAACATTTCCGTGTCGCCCTTATTCCCTTTTTTGCGGCATTTTGCCTTCCTGTTTTTGCTCACCGCTGCAGCAATG···
                                                                          ―――
                                                                          Pst


                                                                      25
      MetSerIleGlnHisPheArgValAlaLeuIleProPhePheAlaAlaPheCysLeuProValPheAlaHisProLeuGlnGln
pKT280 ATGAGTATTCAACATTTCCGTGTCGCCCTTATTCCCTTTTTTGCGGCATTTTGCCTTCCTGTTTTTGCTCACCCGCTGCAGCAATG···
                                                                          ―――
                                                                          Pst


                                                                      27
      MetSerIleGlnHisPheArgValAlaLeuIleProPhePheAlaAlaPheCysLeuProValPheAlaHisProGluThr
pKT287 ATGAGTATTCAACATTTCCGTGTCGCCCTTATTCCCTTTTTTGCGGCATTTTGCCTTCCTGTTTTTGCTCACCCAGAAACG··

      AlaAlaAlaMet
      GCTGCAGCAATG···
      ―――
      Pst
```

## FIG 6

FIG.7

FRACTIONATION OF S-100 EXTRACT OF
E. coli HB101 (Z-pBR 322 (Pst)/HcIF-35)
ON SEPHADEX G-100

INTERFERON ACTIVITY ( i.u. per ml)

OD 405 (CYTOCHROME C)

INTERFERON ACTIVITY

OD 280

OD 405

OD 280

FRACTION NUMBER

S1   S10

1   10   20   30   40   50   60   70   80

METALASERPROPHEALALEULEUMETVALLEU

$G_{23}$CTCTAGGTTCAGAGTCACCCATCTCAGCAAGCCCAGAAGTATCTGCAATATCTACGATGGCCTCGCCCTTTGCTTTACTGATGGTCCTG

HINF                                    BSPI                          EcoRII

                                                                      AvaII

S20   1   10

90   100   110   120   130   140   150   160   170

VALVALLEUSERCYSLYSSERSERCYSSERLEUGLYCYSASPLEUPROGLUTHRHISSERLEUASPASNARGARGTHR'EUMETLEULEU

GTGGTGCTCAGCTGCAAGTCAAGCTGCTCTCTGGGCTGTGATCTCCCTGAGACCCACAGCCTGGATAACAGGAGGACCTTGATGCTCCTG

DDEI   PvuII   ALUI         MBOI DDEI EcoPI   EcoRII         AvaII SFANI EcoRII

        ALUI

20   30   40

180   190   200   210   220   230   240   250   260

ALAGLNMETSERARGILESERPROSERSERCYSLEUMETASPARGHISASPPHEGLYPHEPROGLNGLUGLUPHEASPGLYASNGLNPHE

GCACAAATGAGCAGAATCTCTCCTTCCTCCTGTCTGATGGACAGACATGACTTTGGATTTCCCCAGGAGGAGTTTGATGGCAACCAGTTC

HINF                                        EcoRII

FIG. 8

50               60              70

270   280   290   300   310   320   330   340   350

GLNLYSALAPROALAILESERVALLEUHISGLULEUILEGLNGLNILEPHEASNLEUPHETHRTHRLYSASPSERSERALAALATRPASP

CAGAAGGCTCCAGCCATCTCTGTCCTCCATGAGCTGATCCAGCAGATCTTCAACCTCTTTACCACAAAAGATTCATCTGCTGCTTGGGAT

ALUMBOI / BGLII          HINF
EcoP15
MBOI MBOII

80            90           100

360   370   380   390   400   410   420   430   440

GLUASPLEULEUASPLYSPHECYSTHRGLULEUTYRGLNGLNLEUASNASPLEUGLUGLUALACYSVALMETGLNGLUGLUARGVALGLYGLU

GAGGACCTCCTAGACAAATTCTGCACCGAACTCTACCAGCAGCTGAATGACTTGGAAGCCTGTGTGATGCAGGAGGAGAGGGTGGGAGAA

AVAII         EcoP15 PVUII       SFANI
ALUI

110          120          130

450   460   470   480   490   500   510   520   530

THRPROLEUMETASNALAASPSERILELEUALAVALLYSLYSTYRPHEARGARGILETHRLEUTYRLEUTHRGLULYSLYSTYRSERPRO

ACTCCCCTGATGAATGCGGACTCCATCTTGGCTGTGAAGAAATACTTCCGAAGAATCACTCTCTATCTGACAGAGAAGAAATACAGCCCT

HINF       MBOII      MBOII       MBOII
HINF

# FIG.9

EP 0 032 134 B2

140 150 160 166
540    550    560    570    580    590    600    610    620

CYSALATRPGLUVALVALARGALAGLUILEMETARGSERLEUSERLEUSERTHRASNLEUGLNGLUARGLEUARGARGLYSGLU

TGTGCCTGGGAGGTTGTCAGAGCAGAAATCATGAGATCCCTCTCTTTATCAACAAACTTGCAAGAAAGATTAAGGAGGAAGGAATAACAT

EcoRII                                    MBoI

630    640    650    660    670    680    690    700    710

CTGGTCCAACATGAAAACAATTCTTATTGACTCATACACCAGGTCACGCTTTCATGAATTCTGTCATTTCAAAGACTCTCACCCCTGCTA

AvaII                          HINF    EcoRII              EcoRI              HINF  HPH

720    730    740    750    760    770    780    790    800

TAACTATGACCATGCTGATAAACTGATTTATCTATTTAAATATTTATTTAACTATTCATAAGATTTAAATTATTTTTGTTCATATAACGT

810    820    830    840    850    860  865

CATGTGCACCTTTACACTGTGGTTAGTGTAATAAAACATGTTCCTTATATTTACTCAAAAAAAC$_{15}$

AccI

**FIG. 10**

84

FIG. 11

EP 0 032 134 B2

EP 0 032 134 B2

206(II)

-50   -40

G$_{13}$TTACTGGTGGCCCTC

leu leu val ala leu

-20

met ala ser pro phe ala leu leu met val leu

2H(I) G$_{23}$CTCTAGGTTCAGAGTCACCCATCTCAGCAAGCCCAGAAGTATCTGCAATATCTACGATGGCCTCGCCCTTTGCTTTACTGATGGTCCTG

-120 -110 -100 -90 -80 -70 -60 -50 -40

-30 -20 -10 1 10 20 30 40 50

206(II) CTGGTGCTCAGCTGCAAGTCAAGCTGCTCTGTGGGCTGTGATCTGCCTCAAACCCACAGCCTGGGTAGCAGGAGGACCTTGATGCTCCTG

leu val leu ser cys lys ser ser cys ser val gly CYS ASP LEU PRO GLN THR HIS SER LEU GLY SER ARG ARG THR LEU MET LEU LEU

-10  -1 1  10

val val leu ser cys lys ser ser cys ser leu gly CYS ASP LEU PRO GLU THR HIS SER LEU ASP ASN ARG ARG THR LEU MET LEU LEU

2H(I) GTGGTGCTCAGCTGCAAGTCAAGCTGCTCTCTGGGCTGTGATCTCCCTGAGACCCACAGCCTGGATAACAGGAGGACCTTGATGCTCCTG

-30 -20 -10 1 10 20 30 40 50

# FIG. 12

206(II)

```
       60        70        80        90       100       110       120       130       140
GCACAGATGAGGAGAATCTCTCTTTTCTCCTGCTTGAAGGACAGACATGACTTTGGATTTCCCCAGGAGGAGTTT---GGCAACCAGTTC
ALA GLN MET ARG ARG ILE SER LEU PHE SER CYS LEU LYS ASP ARG HIS ASP PHE GLY PHE PRO GLN GLU GLU PHE  -  GLY ASN GLN PHE
```

```
       20                          30                                    40
ALA GLN MET SER ARG ILE SER PRO SER SER CYS LEU MET ASP ARG HIS ASP PHE GLY PHE PRO GLN GLU GLU PHE ASP GLY ASN GLN PHE
```

2H(I)

```
GCACAAATGAGCAGAATCTCTCCTTCCTCCTGTCTGATGGACAGACATGACTTTGGATTTCCCCAGGAGGAGTTTGATGGCAACCAGTTC
       60        70        80        90       100       110       120       130       140
```

206(II)

```
      150       160       170       180       190       200       210       220       230
CAAAAGGCTGAAACCATCCCTGTCCTCCATGAGATGATCCAGCAGATCTTCAATCTCTTCAGCACAAAGGACTCATCTGCTGCTTGGGAT
GLN LYS ALA GLU THR ILE PRO VAL LEU HIS GLU MET ILE GLN GLN ILE PHE ASN LEU PHE SER THR LYS ASP SER SER ALA ALA TRP ASP
```

```
        50                          60                                   70
        50                          60                                   70
GLN LYS ALA PRO ALA ILE SER VAL LEU HIS GLU LEU ILE GLN GLN ILE PHE ASN LEU PHE THR THR LYS ASP SER SER ALA ALA TRP ASP
```

2H(I)

```
CAGAAGGCTCCAGCCATCTCTGTCCTCCATGAGCTGATCCAGCAGATCTTCAACCTCTTTACCACAAAAGATTCATCTGCTGCTTGGGAT
       150       160       170       180       190       200       210       220       230
```

## FIG.13

EP 0 032 134 B2

206(II)

240    250    260    270    280    290    300    310    320

GAGACCCTCCTAGACAAATTCTACACTGAACTCTACCAGCAGCTGAATGACCTGGAAGCCTGTGTGATACAGGGGGTGGGGGTGACAGAG

GLU THR LEU LEU ASP LYS PHE TYR THR GLU LEU TYR GLN GLN LEU ASN ASP LEU GLU ALA CYS VAL ILE GLN GLY VAL GLY VAL THR GLU

80                      90                  100

80                      90                  100

GLU ASP LEU LEU ASP LYS PHE CYS THR GLU LEU TYR GLN GLN LEU ASN ASP LEU GLU ALA CYS VAL MET GLN GLU GLU ARG VAL GLY GLU

GAGGACCTCCTAGACAAATTCTGCACCGAACTCTACCAGCAGCTGAATGACTTGGAAGCCTGTGTGATGCAGGAGGAGAGGGTGGGAGAA

2H(I)

240    250    260    270    280    290    300    310    320

206(II)

330    340    350    360    370    380    390    400    410

ACTCCCCTGATGAAGGAGGACTCCATTCTGGCTGTGAGGAAATACTTCCAAAGAATCACTCTCTATCTGAAAGAGAAGAAATACAGCCCT

THR PRO LEU MET LYS GLU ASP SER ILE LEU ALA VAL ARG LYS TYR PHE GLN ARG ILE THR LEU TYR LEU LYS GLU LYS LYS TYR SER PRO

110                      120                  130

110                      120                  130

THR PRO LEU MET ASN ALA ASP SER ILE LEU ALA VAL LYS LYS TYR PHE ARG ARG ILE THR LEU TYR LEU THR GLU LYS LYS TYR SER PRO

2H(I)

ACTCCCCTGATGAATGCGGACTCCATCTTGGCTGTGAAGAAATACTTCCGAAGAATCACTCTCTATCTGACAGAGAAGAAATACAGCCCT

330    340    350    360    370    380    390    400    410

FIG.14

EP 0 032 134 B2

206(II)

420 · 430 · 440 · 450 · 460 · 470 · 480 · 490 · 500 ·
JGTGCCTGGGAGGTTGTCAGAGCAGAAATCATGAGATCTTTTTCTTTGTCAACAAACTTGCAAGAAAGTTTAAGAAGTAAGGAATGAAAA.
CYS ALA TRP GLU VAL VAL ARG ALA GLU ILE MET ARG SER PHE SER LEU SER THR ASN LEU GLN GLU SER LEU ARG SER LYS GLU
140                                    150                                  160              165

140                                    150                                  160              166
CYS ALA TRP GLU VAL VAL ARG ALA GLU ILE MET ARG SER LEU SER LEU SER THR ASN LEU GLN GLU ARG LEU ARG ARG LYS GLU

2H(I)

TGTGCCTGGGAGGTTGTCAGAGCAGAAATCATGAGATCCCTCTCTTTATCAACAAACTTGCAAGAAAGATTAAGGAGGAAGGAATAACAT
420 · 430 · 440 · 450 · 460 · 470 · 480 · 490 · 500 ·

206(II)

510 · 520 · 530 · 540 · 550 · 560 · 570 · 580 ·
CTGGTTCAACATGGAAATGATTTTCATTGATTCGTATGCCAGCTCACCTTTTTATGA--TCTGCCATTTCAAAGACTCATGTTTCTGCTA

2H(I)

CTGGTCCAACATGAAAACAATTCTTATTGACTCATACACCAGGTCACGCTTTCATGAATTCTGTCATTTCAAAGACTCTCACCCCTGCTA
510 · 520 · 530 · 540 · 550 · 560 · 570 · 580 · 590 ·

# FIG.15

EP 0 032 134 B2

```
          590       600       610       620       630       640       650       660       670       680
206(II)   TGACCATGACACGATTTAAATCTTTTCAAATGTTTTTAGGAGTATTAATCAACATTGTATTCAGCTCTTAAGGCACTAGTCCCTTACAGAG


2H(I)     TAACTATGACCATGCTGATAAACTGATTTATCTATTTAAATATTTATTTAACTATTCATAAGATTTAAATTATTTTTGTTCATATAACGTC
              600       610       620       630       640       650       660       670       680


                690
206(II)   GACCATGCTGAC₂₉


2H(I)     ATGTGCACCTTTACACTGTGGTTAGTGTAATAAAACATGTTCCTTATATTTACTCAAAAAAAC₁₅
              690       700       710       720       730       740
```

**FIG.16**

FIG. 17

# *FIG.*18

PARTIAL RESTRICTION MAPS OF CLONED, IFN·α RELATED
CHROMOSOMAL DNA SEGMENTS

E: EcoRI, Ba: BamHI, Bg: BglII, H: HincII, T: TacI

FIG. 19

AAAACAAAACATTTGAGAAACACGGCTCTAAACTCATGTAAAGAGTGCATGAAGGAAAGCAAAAACAGAAATG

-140              -120            -100            -80           -60

GAAAGTGGCCCAGAAGCATTAAGAAAGTGGAAATCAGTATGTTCCCTATTTAAGGCATTTGCAGGAAGCAAGGCCTTCAGAGAACCTAGA

BspI                                              BspI

S1                   S10

-40                -20          +1            20

*metalaserprophealalauleumetvalleu*

GCCCAAGGTTCAGAGTCACCCATCTCAGCAAGCCCAGAAGTATCTGCAATATCTACGATGGCCTCGCCCTTTGCTTTACTGATGGTCCTG

HINF                                     BspI             EcoRII

AvaII

S20           1                         10

40            60            80          100          120

*valvalleuseroyslyssersaroyssarleuglyCYSASPLEUPROGLUTHRHISSERLEUASPASNARGARGTHRLEUMETLEULEU*

GTGGTGCTCAGCTGCAAGTCAAGCTGCTCTCTGGGCTGTGATCTCCCTGAGACCCACAGCCTGGATAACAGGAGGACCTTGATGCTCCTG

DDEI   PvuII     AluI         MboI  DDEI EcoPI    EcoRII       AvaII  SFANI EcoRII

AluI

**FIG. 20**

20                                      30                                      40

EP 0 032 134 B2

                                140                                     160                                     180                                     200
*ALAGLNMETSERARGILESERPROSERSERCYSLEUMETASPARGHISASPPHEGLYPHEPROGLNGLUGLUPHEASPGLYASNGLNPHE*
GCACAAATGAGCAGAATCTCTCCTTCCTCCTGTCTGATGGACAGACATGACTTTGGATTTCCCCAGGAGGAGTTTGATGGCAACCAGTTC
                    HINF                                                              ECORII

50                                      60                                      70

                220                                     240                                     260                                     280                                     300
*GLNLYSALAPROALAILESERVALLEUHISGLULEUILEGLNGLNILEPHEASNLEUPHETHRTHRLYSASPSERSERALAALATRPASP*
CAGAAGGCTCCAGCCATCTCTGTCCTCCATGAGCTGATCCAGCAGATCTTCAACCTCTTTACCACAAAAGATTCATCTGCTGCTTGGGAT
                                        ALU MBOI/   BGLII                              HINF
                                          ECOP15
                                                   MBOI MBOII

80                                      90                                      100

                320                                     340                                     360                                     380
*GLUASPLEULEUASPLYSPHECYSTHRGLULEULEUTYRGLNGLNLEUASNASPLEUGLUGLUALACYSVALMETGLNGLUGLUARGVALGLYGLU*
GAGGACCTCCTAGACAAATTCTGCACCGAACTCTACCAGCAGCTGAATGACTTGGAAGCCTGTGTGATGCAGGAGGAGAGGGTGGGAGAA
AVAII                                   ECOP15 PVUII                              SFANI
                                               ALUI

# FIG. 21

EP 0 032 134 B2

110      120      130

400    420    440    460    480

*THRPROLEUMETASNALAASPSERILELEUALAVALLYSLYSTYRPHEARGARGILETHRLEUTYRLEUTHRGLULYSLYSTYRSERPRO*

ACTCCCCTGATGAATGCGGACTCCATCTTGGCTGTGAAGAAATACTTCCGAAGAATCACTCTCTATCTGACAGAGAAGAAATACAGCCCT

    HINF     MBoII   MBoII     MBoII

                  HINF

140      150      160   166

500    520    540    560

*CYSALATRPGLUVALVALARGALAGLUILEMETARGSERLEUSERLEUSERTHRASNLEUGLNGLUARGLEUARGARGLYSGLU*

TGTGCCTGGGAGGTTGTCAGAGCAGAAATCATGAGATCCCTCTCTTTATCAACAAACTTGCAAGAAAGATTAAGGAGGAAGGAATAACAT

EcoRII       MBoI

580    600    620    640    660

CTGGTCCAACATGAAAACAATTCTTATTGACTCATACACCAGGTCACGCTTTCATGAATTCTGTCATTTCAAAGACTCTCACCCCTGCTA

AvaII      HINF  EcoRII    EcoRI    HINF HPH

680    700    720    740

TAACTATGACCATGCTGATAAACTGATTTATCTATTTAAATATTTATTTAACTATTCATAAGATTTAAATTATTTTTGTTCATATAACGT

760    780    800    820    840

CATGTGCACCTTTACACTGTGGTTAGTGTAATAAAACATGTTCCTTATATTTACTCAATCCATTATTTTGTGTTGTTCATTAAACTTTTA

HGIA

**FIG. 22**

```
                860                          880                          900                          920
CTATAGGAACTTCCTGTATGTGTTCATTCTTTAATATGAAATTCCTAGCCTGACTGTGCAACCTGATTAGAGAATAAAGGGTATATTTTA

     940              959
TTTGCTTATCATTATTATATGTAAGA
```

**FIG. 23**

EP 0 032 134 B2

# FIG. 24

LINKAGE OF IFN-α RELATED GENES

# FIG. 25

# FIG.26

LAC-AUG (α2)

# FIG. 27

## CONSTRUCTION OF PLASMID LAC-AUG (α-2)

LAC PROMOTER             IFN-α CDNA

      MET                   LEU GLY CYS ASP

, , ACAG CTATG · · ·          CTGGGCTGTGAT · · ·

, , , TGTC GATAC · · '          GACCCGACA CTA · · ·

ALU I                              SAU 3A

             ECO LINKER

, , , AAACAG   CTATGAATTCATAG       ACAAGCTTGT : GAT · · ·

, , , TTTCTC   GATACTTAAGTATC       TGTTCGAACA : CTA · · ·

            LIGASE                            LIGASE

                                            CYS ASP

, , , ACAGCTATG AATT CATAG       ACA AGCTTGTGAT , , ,

, , , TGTCGATAC TTAA GTATC       TGTTCGA ACACTA , , ,

ECO RI                          HIND III

$S_1$ NUCLEASE                    $S_1$ NUCLEASE

, , , ACAGCTATG   TGTGAT · · ·

, , , TCTCGATAC : ACACTA · · ·

                     LIGASE

              Met CYS ASP

, , , ACAGCTATGTGTGAT · · ·

, , , TGTCGATACACACTA · · ·

101

# FIG.28

160        180        200

C24AATGCAAATAGCTTATATTATATGATTTATTTCTAGTAAA
        AluI

220      240      260      280      300

GTTATTCAACACATCAGTACTTATGTCAACTGCTGAAAAGAAAAAAGTGTTGGCAACATCTGGATGAATACTGCAGCTGATGAAGTTTACAAATTATTTT
         RsaI    ChuII                       PstI
                                                  AluI
                                               PvuII
                                            bbRsI

320      340      360      380      400

GTCATATAAAGCAAAATTCAAAGCTTCATACACTAAGAGAAAAATTTTAAAAAATTATTCATTCATATTTTTAGGAGTTTTGAATGATTGGATATGTAAT
        EcoRI  BbRI     DdeI  EcoRI
                AluI

420      440      460      480      500

TATATTCATATTATTAATGTGTATCTATATAGATTTTTATTTTGCATATGTACTTTGATACAAAATTTACATGAACAAATTACACTAAAGTTATTCCACA
                    EcoRI         RsaI       EcoRI

520      540      560      580      600

ATATACTTATCAAATTAAGTTAATGTCAATAGCTTTTAAACTTAAATTTTAGTTTAGTTAGCTTTTCTGTCATTCTTACTTACTTTGAATAAAAAGAGCA
                    AluI       EcoRI      AluI

# FIG. 29

EP 0 032 134 B2

```
          620              640              660              680              700
AACTTTGTAGTTTTTATCTCTGTGAAGTAGAGGTATACGTAATATACATAAATAGATATGCCAAATCTGTGTTATTAAAATTTCATGAAGATTTCAATTA
                     MALI                                    EcoRI         EcoRI    MBoII


          720              740              760              780              800
GAAAAAAATACCATAAAAGGCTTTGAGTGCAGGTGAAAAATAGGCAATGATGAAAAAAAAATGAAAAACTTTTTAAACACATGTGAGAGTGCGTAAGAAGC
                      HPHI


          820              840              860              880              900
AAACAGAGATAGAAAGTACAACTAGGGAATTTAGAAAATGGAAATTAGTATGTTCACTATTTAAGACCTATGCACAGAGCAAAGTCTTCAGAAAACCTAG
                RSAI       EcoRI                                EcoRI           MBoII


          920              940              960              980              1000
                                                  metalaleuserpheserleuleumetalavalleuvalleuser
AGGCCGAAGTTCAAGGTTATCCATCTCAAGTAGCCCAGCAATATTTGCAACATCCAATGGCCCTGTCCTTTTCTTTACTGATGGCCGTGCTGGTGCTCAG
MNLI                                              AsuI                      BspI          DdeI
  BSPI                                             BSPI                                   ALuI


          1020             1040             1060             1080             1100
   tyrlyeserileuyeserleuglyCYSASPLEUPROGLNTHRHISSERLEUGLYASNARGARGTHRLEUILELEULEUGLNGLNMETGLYARGILESER
CTACAAATCCATCTGTTCTCTGGGCTGTGATCTGCCTCAGACCCACAGCCTGGGTAATAGGAGGACCTTGATACTCCTGCAACAAATGGGAAGAATCTCT
ALuI EcoRI                   DPN  MNLI         BSTNI      MNLI                         MBoII
                             MBoI DDEI                    AsuI                         EcoRI
                                                                                      HHAII
```

**FIG. 30**

FIG. 31

EP 0 032 134 B2

AAGACTCACTTCATAACCACCACGAGTTGAATCAAAATTTTCAAATGTTTCAGCAGTGTGAAGAAGCTTGGTGTATACCGTGCAGGTACTAGTTCTTTA
HhaII        HhaII EcoRI                        MboII          RsaI
                                                BbrI
                                                AluI

CAGATGACAATGCTGATGTCTCTGTTCATCTATTTATTGAATATATTATTTGTTTTAAAATTATTAAATTATTTAATGTGATATCATGAGTACCTTTAC
                                             EcoRI                                    RsaI

ATTGTGGTGGATGTAACGATATATGTTCTTCATATTTTAGCCAATA
                        MboIII

FIG 32